# EUROPEAN PATENT SPECIFICATION

(11) **EP 2 188 282 B1**
(45) Date of publication and mention of the grant of the patent: **23.01.2013**
(21) Application number: 08807317.6
(22) Date of filing: 14.08.2008
(51) Int. Cl.: C07D 417/12, C07D 231/56, C07D 307/79, C07D 319/18, C07D 513/04, A61P 25/00, A61K 31/427, A61K 31/416, A61K 31/4188, A61K 31/353, A61K 31/343

(54) **1,2-DIAMIDO-ETHYLENE DERIVATIVES AS OREXIN ANTAGONISTS**
1,2-DIAMIDETHYLENDERIVATE ALS OREXIN-ANTAGONISTEN
DÉRIVÉS DU 1,2-DIAMIDO-ÉTHYLÈNE COMME ANTAGONISTES D'OREXINE

(30) Priority: 15.08.2007 WO PCT/IB2007/053249
(43) Date of publication of application: 26.05.2010
(73) Proprietor: Actelion Pharmaceuticals Ltd., 4123 Allschwil (CH)
(72) Inventor: AISSAOUI, Hamed, F-68840 Pulversheim (FR); BOSS, Christoph, CH-4123 Allschwil (CH); GUDE, Markus, CH-4123 Allschwil (CH); KOBERSTEIN, Ralf, 79539 Lörrach (DE); SIFFERLEN, Thierry, F-68220 Wentzwiller (FR)
(74) Representative: Velker, Jörg
(86) International application number: PCT/IB2008/053264
(87) International publication number: WO 2009/022311

(56) References cited:
- WO-A-01/96302
- WO-A-03/037547
- CAI J ET AL: "Antagonists of the orexin receptors" EXPERT OPINION ON THERAPEUTIC PATENTS, INFORMA HEALTHCARE, GB, vol. 16, no. 5, 1 May 2006 (2006-05-01), pages 631-646, XP002458093 ISSN: 1354-3776

## Description

The present invention relates to novel 1,2-diamido-ethylene derivatives of formula (I) and their use as pharmaceuticals. The invention also concerns related aspects including processes for the preparation of the compounds, pharmaceutical compositions containing one or more compounds of formula (I), and especially their use as orexin receptor antagonists.

Orexins (orexin A or OX-A and orexin B or OX-B) are novel neuropeptides found in 1998 by two research groups, orexin A is a 33 amino acid peptide and orexin B is a 28 amino acid peptide (Sakurai T. et al., Cell, 1998, 92, 573-585). Orexins are produced in discrete neurons of the lateral hypothalamus and bind to G-protein-coupled receptors (OX₁ and OX₂ receptors). The orexin-1 receptor (OX₁) is selective for OX-A, and the orexin-2 receptor (OX₂) is capable to bind OX-A as well as OX-B. Orexins are found to stimulate food consumption in rats suggesting a physiological role for these peptides as mediators in the central feedback mechanism that regulates feeding behaviour (Sakurai T. et al., Cell, 1998, 92, 573-585). On the other hand, it was also observed that orexins regulate states of sleep and wakefulness opening potentially novel therapeutic approaches to narcolepsy as well as insomnia and other sleep disorders (Chemelli R.M. et al., Cell, 1999, 98, 437-451).

Orexin receptors are found in the mammalian brain and may have numerous implications in pathologies as known from the literature.

The present invention provides 1,2-diamido-ethylene derivatives, which are non-peptide antagonists of human orexin receptors. These compounds are in particular of potential use in the treatment of e.g. eating disorders, drinking disorders, sleep disorders, or cognitive dysfunctions in psychiatric and neurologic disorders.

Up to now, several low molecular weight compounds are known having a potential to antagonise either specifically OX₁ or OX₂, or both receptors at the same time (for review see for example Cai et al., Expert Opinion on Therapeutic Patents 2006, 16(5), 631-646). Ethylene diamine derivatives useful as orexin receptor antagonists are disclosed in WO03/051872. WO2001/09630 discloses orexin receptor antagonists which comprise a diamido-ethylene backbone where one of the N atoms together with the neighboring C atom is part of a cyclic piperidine structure.

The present invention describes for the first time 1,2-diamido-ethylene derivatives as orexin receptor antagonists.
i) A first aspect of the invention consists of 1,2-diamido-ethylene derivatives of the formula (I) 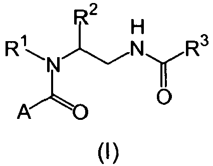 wherein
   R¹ represents cyclopropyl or cyclopropyl-methyl;
   R² represents hydrogen, (C₁₋₄)alkyl, cyclopropyl, or phenyl-(C₁₋₄)alkyl, wherein the phenyl is unsubstituted, or mono-, di-, or tri-substituted, wherein the substituents are independently selected from the group consisting of (C₁₋₄)alkyl, (C₁₋₄)alkoxy, trifluoromethyl, and halogen;
   wherein R¹ and R² do not represent hydrogen at the same time;
   R³ represents heterocyclyl, wherein said heterocyclyl is an 8- to 10-membered bicyclic ring containing 1, 2 or 3 heteroatoms, each independently selected from oxygen, nitrogen and sulfur, wherein at least one ring of said bicyclic ring system is aromatic and the bond with which the heterocyclyl is attached to the rest of the molecule is positioned on an aromatic carbon atom of said bicyclic ring system in alpha position to a bridgehead atom; wherein said heterocyclyl is unsubstituted, mono-, di-, or tri-substituted, wherein the substituents are independently selected from the group consisting of (C₁₋₄)alkyl, (C₁₋₄)alkoxy, halogen, and trifluoromethyl;
   A represents 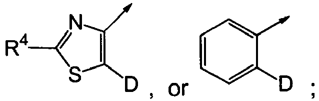 R⁴ represents (C₁₋₄)alkyl, bromo, cyclopropyl, or -NR⁵R⁶;
   R⁵ represents hydrogen, or (C₁₋₄)alkyl;
   R⁶ represents hydrogen, or (C₁₋₄)alkyl; and
   D represents phenyl, which is unsubstituted, mono-, di-, or tri-substituted, wherein the substituents are independently selected from the group consisting of (C₁₋₄)alkyl, (C₁₋₄)alkoxy, trifluoromethyl, trifluoromethoxy, cyano, and halogen.

The compounds of formula (I) may contain one or more stereogenic or asymmetric centers, such as one or more asymmetric carbon atoms. Substituents at a double bond may be present in the *Z*- or *E*-configuration unless indicated otherwise. The compounds of formula (I) may thus be present as mixtures of stereoisomers or preferably as pure stereoisomers. Mixtures of stereoisomers may be separated in a manner known to a person skilled in the art.

Where the plural form is used for compounds, salts, pharmaceutical compositions, diseases and the like, this is intended to mean also a single compound, salt, or the like.

Any reference to a compound of formula (I) is to be understood as referring also to the salts (and especially the pharmaceutically acceptable salts) of such compounds, as appropriate and expedient.

The term "pharmaceutically acceptable salts" refers to non-toxic, inorganic or organic acid and/or base addition salts. Reference can be made to "Salt selection for basic drugs", Int. J. Pharm. (1986), 33, 201-217.

The following paragraphs provide definitions of the various chemical moieties for the compounds according to the invention and are intended to apply uniformly throughout the specification and claims, unless an otherwise expressly set out definition provides a broader or narrower definition.

The term "halogen" means fluorine, chlorine, or bromine, preferably fluorine or chlorine.

The term "alkyl", alone or in any combination, means a straight-chain or branched-chain alkyl group with the indicated number of carbon atoms. For example, the term "(C₁₋₆)alkyl" means an alkyl group with 1 to 6 carbon atoms. Examples of (C₁₋₆)alkyl groups are methyl, ethyl, propyl, isopropyl, n-butyl, isobutyl, sec.-butyl, tert.-butyl, n-pentyl, isopentyl, n-hexyl, and 3,3-dimethyl-butyl. The term "(C₁₋₄)alkyl" means an alkyl group with 1 to 4 carbon atoms. Examples of (C₁₋₄)alkyl groups are methyl, ethyl, n-propyl, isopropyl, n-butyl, isobutyl, sec.-butyl and tert.-butyl. Preferred are methyl, ethyl, and n-propyl. Most preferred is methyl. Examples of "R¹" representing (C₁₋₆)alkyl are methyl, isobutyl, and 3,3-dimethyl-butyl, preferred is methyl. Examples of "R²" representing (C₁₋₄)alkyl are methyl, isopropyl, and isobutyl.

The term "(C₃₋₆)cycloalkyl" means a saturated cyclic hydrocarbon moiety containing 3-6 carbon atoms. Examples are cyclopropyl, cyclopentyl and cyclohexyl, preferred is cyclopropyl.

The term "(C₃₋₆)cycloalkyl-(C₁₋₄)alkyl" means a (C₃₋₆)cycloalkyl group as previously defined that is attached to the parent molecular moiety through a (C₁₋₄)alkyl group as previously defined. Examples are cyclopropyl-methyl, cyclopentyl-methyl and cyclohexyl-methyl, preferred are cyclopropyl-methyl, and cyclohexyl-methyl; most preferred is cyclopropyl-methyl.

The term "(C₁₋₄)alkoxy" means a group of the formula (C₁₋₄)alkyl-O- in which the term "(C₁₋₄)alkyl" has the previously given significance. Examples of (C₁₋₄)alkoxy groups are methoxy, ethoxy, n-propoxy, isopropoxy, n-butoxy, isobutoxy, sec.-butoxy and tert.-butoxy. Preferred are methoxy and ethoxy. Most preferred is methoxy.

The term "heterocyclyl" means a 8- to 10-membered bicyclic ring containing 1, 2 or 3 heteroatoms, each independently selected from oxygen, nitrogen and sulfur, wherein at least one ring of said bicyclic ring system is aromatic and the bond with which the heterocyclyl is attached to the rest of the molecule is positioned on an aromatic carbon atom of said bicyclic ring system in alpha position to a bridgehead atom as further illustrated in the following examples: 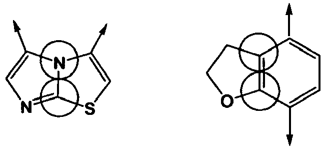 ○ = bridgehead atom of the bicyclic ring system
↑ = bond with which in these examples the heterocyclyl group may be attached to the rest of the molecule

Examples of such heterocyclyl groups are quinoline-8-yl, isoquinoline-1-yl, indol-3-yl, indol-4-yl, indol-7-yl, indazol-3-yl, indazol-4-yl, indazol-7-yl, benzofuran-4-yl, benzofuran-7-yl, benzisoxazol-3-yl, benzisoxazol-4-yl, benzisoxazol-7-yl, benzoxazol-4-yl, benzoxazol-7-yl, benzoxadiazol-4-yl, benzoxadiazol-7-yl, benzothiophene-3-yl, benzothiophene-4-yl, benzothiophene-7-yl, benzthiazol-4-yl, benzthiazol-7-yl, benzoisothiazol-3-yl, benzoisothiazol-4-yl, benzoisothiazol-7-yl, benzothiadiazol-4-yl, benzothiadiazol-7-yl, benzimidazol-4-yl, benzimidazol-7-yl, imidazo[2,1-b]thiazol-3-yl, imidazo[2,1-b]thiazol-5-yl, pyrazolo[1,5-a]pyridine-3-yl, imidazo[1,2-a]pyridine-3-yl, 2,3-dihydro-thieno[3,4-b][1,4]dioxine-5-yl, benzo[1,3]dioxol-4-yl, 2,3-dihydrobenzofuran-4-yl, 2,3-dihydro-benzofuran-7-yl, 4H-benzo[1,3]dioxin-8-yl, 4H-benzo[1,3]dioxin-5-yl, and 2,3-dihydro-benzo[1,4]dioxin-5-yl. The above-mentioned heterocyclyl groups are unsubstituted, mono-, di-, or tri-substituted, wherein the substituents are independently selected from the group consisting of (C₁₋₄)alkyl, (C₁₋₄)alkoxy, halogen, and trifluoromethyl.

In "R³" representing "heterocyclyl" said heterocyclyl group is preferably unsubstituted, mono-, or di-substituted, wherein the substituents preferably are independently selected from the group consisting of (C₁₋₄)alkyl. If substituted, indolyl, indazolyl, and imidazo[2,1-b]thiazolyl groups are preferably mono- or di-substituted (preferred mono-substituted) with methyl. 2,3-Dihydro-benzofuranyl, benzo[1,3]dioxol-4-yl, 4H-benzo[1,3]dioxinyl, and 2,3-dihydro-benzo[1,4]dioxin-5-yl groups are preferably unsubstituted. Particular examples of R³ representing "heterocyclyl" are quinoline-8-yl, isoquinoline-1-yl, 1,2-dimethyl-indol-3-yl, 1-methyl-indol-3-yl, indazol-3-yl, 1-methyl-indazol-3-yl, 2-methyl-benzofuran-4-yl, benzofuran-4-yl, benzisoxazole-3-yl, 2-methyl-benzoxazole-4-yl, benzoisothiazole-3-yl, imidazo[2,1-b]-thiazole-5-yl, 2-methyl-imidazo[2,1-b]thiazol-5-yl, 3-methyl-imidazo[2,1-b]thiazol-5-yl, 6-methyl-imidazo[2,1-b]-thiazole-5-yl, pyrazolo[1,5-a]pyridine-3-yl, imidazo[1,2-a]pyridine-3-yl, 2,3-dihydro-thieno[3,4-b][1,4]dioxine-5-yl, 2,3-dihydro-benzofuran-4-yl, 2,3-dihydrobenzofuran-7-yl, and 2,3-dihydro-benzo[1,4]dioxine-5-yl. Preferred examples are 1-methyl-indazol-3-yl, benzofuran-4-yl, imidazo[2,1-b]-thiazole-5-yl, 6-methyl-imidazo[2,1-b]-thiazole-5-yl, 3-methyl-imidazo[2,1-b]thiazol-5-yl, and 2,3-dihydro-benzo[1,4]dioxine-5-yl; more preferred examples are 1-methyl-indazol-3-yl, benzofuran-4-yl, and 6-methyl-imidazo[2,1-b]-thiazole-5-yl.

The term "aryl", alone or in any combination, means phenyl, wherein the aryl group is unsubstituted, mono-, di-, or tri-substituted, wherein the substituents are independently selected from the group consisting of (C₁-4)alkyl, (C,₄)alkoxy, trifluoromethyl, trifluoromethoxy, cyano, and halogen.

Examples of "D" representing "aryl" are phenyl, 3-methylphenyl, 4-methylphenyl, 3,4-dimethylphenyl, 3-cyanophenyl, 3-methoxyphenyl, 2-fluorophenyl, 3-fluorophenyl, 4-fluorophenyl, 3-chlorophenyl, 4-chlorophenyl, 3-trifluoromethylphenyl and 4-trifluoromethylphenyl.

The term "aryl-(C₁₋₄)alkyl" means an aryl group as previously defined that is attached to the parent molecular moiety through a (C₁₋₄)alkyl group as previously defined.

In "R¹" representing "aryl-(C₁₋₄)alkyl" the term aryl preferably means phenyl, which is unsubstituted, or mono-substituted, wherein the substituent is selected from the group consisting of (C₁₋₄)alkyl, (C₁₋₄)alkoxy, trifluoromethyl, and halogen (preferred trifluoromethyl, and halogen). Examples of "R¹" representing aryl-(C₁₋₄)alkyl are benzyl, 4-fluoro-benzyl, 4-trifluoromethyl-benzyl, phenyl-ethyl, and phenyl-propyl.

In "R²" representing "aryl-(C₁₋₄)alkyl" the term aryl preferably means phenyl, which is unsubstituted. In another embodiment the phenyl group may be mono-, di-, or tri-substituted (preferably mono-substituted), wherein the substituents preferably are independently selected from the group consisting of (C₁₋₄)alkyl, (C₁₋₄)alkoxy, trifluoromethyl, and halogen. Preferred examples of "R²" representing aryl-(C₁₋₄)alkyl are aryl-(C₁₋₂)alkyl groups such as benzyl, and phenyl-ethyl.

Examples of "-NR⁵R⁶" groups are -NH₂ (preferred) and -N(CH₃)₂.

The term "acyl" as used in the specification means an aryl-CO-, an alkyl-CO-, or a heteroaryl-CO- group, such as for example A-CO-, or R³-CO-, wherein A and R³ have the meaning given for formula (I).
ii) A further embodiment of the invention relates to 1,2-diamido-ethylene derivatives according to embodiment i), wherein
   A represents 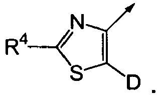
iii) A further embodiment of the invention relates to 1,2-diamido-ethylene derivatives according to embodiments i) and ii), wherein
   R⁴ represents methyl, -NH₂, bromo, or cyclopropyl (especially methyl, or -NH₂).
iv) A further embodiment of the invention relates to 1,2-diamido-ethylene derivatives according to embodiments i) and ii), wherein
   R⁴ represents cyclopropyl.
v) A further embodiment of the invention relates to 1,2-diamido-ethylene derivatives according to embodiment i), wherein
   A represents 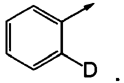
vi) A further embodiment of the invention relates to 1,2-diamido-ethylene derivatives according to any one of embodiments i) to v), wherein D represents phenyl, which is unsubstituted, mono- or di-substituted, wherein the substituents are independently selected from the group consisting of (C₁₋₄)alkyl, (C₁₋₄)alkoxy, trifluoromethyl, cyano, and halogen.
x) A further embodiment of the invention relates to 1,2-diamido-ethylene derivatives according to any one of embodiments i) to vi), wherein R² represents hydrogen, methyl, cyclopropyl, or phenyl-(C₁₋₂)alkyl, wherein the phenyl is unsubstituted.
xi) A further embodiment of the invention relates to 1,2-diamido-ethylene derivatives according to any one of embodiments i) to vi), wherein R² represents methyl, cyclopropyl, or phenyl-(C₁₋₂)alkyl, wherein the phenyl is unsubstituted.
xii) A further embodiment of the invention relates to 1,2-diamido-ethylene derivatives according to any one of embodiments i) to xi), wherein, in case R² represents other than hydrogen, the carbon center to which R² is attached is in (*R*)-configuration.
xiii) A further embodiment of the invention relates to 1,2-diamido-ethylene derivatives according to any one of embodiments i) to xi), wherein, in case R² represents other than hydrogen, the carbon center to which R² is attached is in (*S*)-configuration.
xiv) A further embodiment of the invention relates to 1,2-diamido-ethylene derivatives according to any one of embodiments i) to viii), wherein R² represents hydrogen.
xv) A further embodiment of the invention relates to 1,2-diamido-ethylene derivatives according to any one of embodiments i) to xiv), wherein R³ represents a group selected from quinoline-8-yl, isoquinoline-1-yl, indol-3-yl, indol-4-yl, indol-7-yl, indazol-3-yl, indazol-4-yl, indazol-7-yl, benzofuran-4-yl, benzofuran-7-yl, benzisoxazol-3-yl, benzisoxazol-4-yl, benzisoxazol-7-yl, benzoxazol-4-yl, benzoxazol-7-yl, benzoxadiazol-4-yl, benzoxadiazol-7-yl, benzothiophene-3-yl, benzothiophene-4-yl, benzothiophene-7-yl, benzthiazol-4-yl, benzthiazol-7-yl, benzoisothiazol-3-yl, benzoisothiazol-4-yl, benzoisothiazol-7-yl, benzothiadiazol-4-yl, benzothiadiazol-7-yl, benzimidazol-4-yl, benzimidazol-7-yl, imidazo[2,1-b]thiazol-3-yl, imidazo[2,1-b]thiazol-5-yl, pyrazolo[1,5-a]pyridine-3-yl, imidazo[1,2-a]pyridine-3-yl, 2,3-dihydro-thieno[3,4-b][1,4]dioxine-5-yl, benzo[1,3]dioxol-4-yl, 2,3-dihydrobenzofuran-4-yl, 2,3-dihydro-benzofuran-7-yl, 4H-benzo[1,3]dioxin-8-yl, 4H-benzo[1,3]dioxin-5-yl, and 2,3-dihydro-benzo[1,4]dioxin-5-yl; wherein said group is unsubstituted, mono-, or di-substituted (especially unsubstituted or mono-substituted), wherein the substituents are independently selected from the group consisting of methyl, methoxy, halogen, and trifluoromethyl (especially methyl).
xvi) A further embodiment of the invention relates to 1,2-diamido-ethylene derivatives according to any one of embodiments i) to xiv), wherein R³ represents quinoline-8-yl, isoquinoline-1-yl, 1,2-dimethyl-indol-3-yl, 1-methyl-indol-3-yl, indazol-3-yl, 1-methyl-indazol-3-yl, 2-methyl-benzofuran-4-yl, benzofuran-4-yl, benzisoxazole-3-yl, 2-methyl-benzoxazole-4-yl, benzoisothiazole-3-yl, imidazo[2,1-b]-thiazole-5-yl, 2-methyl-imidazo[2,1-b]thiazol-5-yl, 3-methyl-imidazo[2,1-b]thiazol-5-yl, 6-methyl-imidazo[2,1-b]-thiazole-5-yl, pyrazolo[1,5-a]pyridine-3-yl, imidazo[1,2-a]pyridine-3-yl, 2,3-dihydro-thieno[3,4-b][1,4]dioxine-5-yl, 2,3-dihydro-benzofuran-4-yl, 2,3-dihydrobenzofuran-7-yl, and 2,3-dihydro-benzo[1,4]dioxine-5-yl (especially 1-methyl-indazol-3-yl, benzofuran-4-yl, 6-methyl-imidazo[2,1-b]-thiazole-5-yl, or 2,3-dihydro-benzo[1,4]dioxine-5-yl).
xvii) A further embodiment of the invention relates to 1,2-diamido-ethylene derivatives according to any one of embodiments i) to xiv), wherein R³ represents 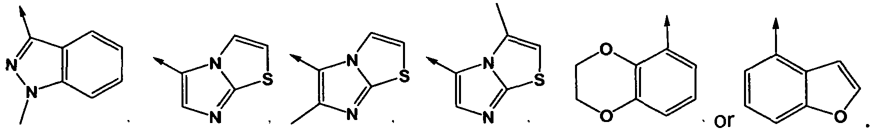
xviii) A further embodiment of the invention relates to 1,2-diamido-ethylene derivatives according to any one of embodiments i) to xiv), wherein R³ represents 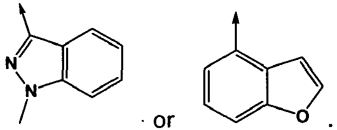
xix) A further embodiment of the invention relates to 1,2-diamido-ethylene derivatives according to any one of embodiments i) to xiv), wherein R³ represents 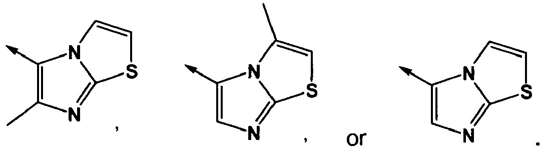
xx) A further embodiment of the invention relates to 1,2-diamido-ethylene derivatives according to any one of embodiments i) to xiv), wherein R³ represents 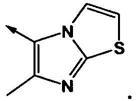
xxi) Preferred examples of 1,2-diamido-ethylene derivatives of formula (I) according to embodiment i) are selected from the group consisting of:
   2-Methyl-5-phenyl-thiazole-4-carboxylic acid {2-[(benzofuran-4-carbonylramino]-ethyl}-cyclopropylmethyl-amide;
   6-Methyl-imidazo[2,1-b]thiazole-5-carboxylic acid {2-[cyclopropylmethyl-(2-methyl-5-phenyl-thiazole-4-carbonylhamino]-ethylfamide;
   2-Methyl-5-phenyl-thiazole-4-carboxylic acid cyclopropylmethyl-{2-[(2,3-dihydro-benzo[1,4]dioxine-5-carbonyl)-amino]-ethyl}-amide;
   2-Methyl-5-phenyl-thiazole-4-carboxylic acid {2-[(benzofuran-4-carbonyl)-amino]-ethyl]-cyclopropyl-amide;
   6-Methyl-imidazo[2,1-b]thiazole-5-carboxylic acid {2-[cyclopropyl-(2-methyl-5-phenyl-thiazole-4-carbonylhamino]-ethylfamide;
   2-Methyl-5-phenyl-thiazole-4-carboxylic acid cyclopropyl-{2-[(2,3-dihydro-benzo[1,4]dioxine-5-carbonyl)-amino]-ethyl}-amide;
   6-Methyl-imidazo[2,1-b]thiazole-5-carboxylic acid {2-[cyclopropyl-(2-cyclopropyl-5-phenyl-thiazole-4-carbonylhamino]-ethyl}-amide;
   6-Methyl-imidazo[2,1-b]thiazole-5-carboxylic acid {2-[cyclopropyl-(2-cyclopropyl-5-*m-*tolyl-thiazole-4-carbonyl)-amino]-ethyl}-amide;
   6-Methyl-imidazo[2,1-b]thiazole-5-carboxylic acid (2-{cyclopropyl-[2-cyclopropyl-5-(3-methoxy-phenyl)-thiazole-4-carbonyl]-amino}ethyl)-amide;
   6-Methyl-imidazo[2,1-b]thiazole-5-carboxylic acid {2-[(2-amino-5-*m*-tolyl-thiazole-4-carbonyl)-cyclopropyl-amino]-ethyl}-amide;
   6-Methyl-imidazo[2,1-b]thiazole-5-carboxylic acid (2-{[2-amino-5-(3-fluoro-phenyl)-thiazole-4-carbonyl]-cyclopropyl-amino}-ethyl)-amide;
   6-Methyl-imidazo[2,1-b]thiazole-5-carboxylic acid (2-{[2-amino-5-(3-methoxy-phenyl}-thiazole-4-carbonyl]-cyclopropyl-amino}-ethyl)-amide;
   6-Methyl-imidazo[2,1-b]thiazole-5-carboxylic acid (2-{[2-amino-5-(4-fluoro-phenyl)-thiazole-4-carbonyl]-cyclopropyl-amino}-ethyl)-amide;
   6-Methyl-imidazo[2,1-b]thiazole-5-carboxylic acid (2-{[2-amino-5-(3-chloro-phenyl}-thiazole-4-carbonyl]-cyclopropyl-amino}-ethyl)-amide;
   6-Methyl-imidazo[2,1-b]thiazole-5-carboxylic acid {2-[(2-amino-5-phenyl-thiazole-4-carbonyl)-cyclopropyl-amino]-ethyl}-amide;
   6-Methyl-imidazo[2,1-b]thiazole-5-carboxylic acid (2-{[2-bromo-5-(3-methoxy-phenyl)-thiazole-4-carbonyl]-cyclopropyl-amino}-ethyl)-amide;
   6-Methyl-imidazo[2,1-b]thiazole-5-carboxylic acid {2-[(2-bromo-5-*p*-tolyl-thiazole-4-carbonyl)-cyclopropyl-amino]-ethyl}-amide;
   6-Methyl-imidazo[2,1-b]thiazole-5-carboxylic acid (2-{cyclopropyl-[5-(2-fluoro-phenyl)-2-methyl-thiazole-4-carbonyl]-amino}-ethyl)-amide;
   6-Methyl-imidazo[2,1-b]thiazole-5-carboxylic acid (2-{cyclopropyl-[2-methyl-5-(3-trifluoromethyl-phenyl)-thiazole-4-carbonyl]-amino}-ethyl)-amide;
   6-Methyl-imidazo[2,1-b]thiazole-5-carboxylic acid (2-{cyclopropyl-[5-(3-fluoro-phenyl)-2-methyl-thiazole-4-carbonyl]-amino}-ethyl)-amide;
   6-Methyl-imidazo[2,1-b]thiazole-5-carboxylic acid (2-{cyclopropyl-[2-methyl-5-(4-trifluoromethyl-phenyl)-thiazole-4-carbonyl]-amino}-ethyl)-amide;
   6-Methyl-imidazo[2,1-b]thiazole-5-carboxylic acid (2-{[5-(4-chloro-phenyl)-2-methylthiazole-4-carbonyl]-cyclopropyl-amino}-ethyl)-amide;
   6-Methyl-imidazo[2,1-b]thiazole-5-carboxylic acid (2-{cyclopropyl-[5-(3-methoxyphenyl)-2-methyl-thiazole-4-carbonyl]-amino}-ethyl)-amide;
   6-Methyl-imidazo[2,1-b]thiazole-5-carboxylic acid (2-{[5-(3-cyano-phenyl)-2-methylthiazole-4-carbonyl]-cyclopropyl-amino}-ethyl)-amide;
   6-Methyl-imidazo[2,1-b]thiazole-5-carboxylic acid (2-{cyclopropyl-[5-(3,4-dimethylphenyl)-2-methyl-thiazole-4-carbonyl]-amino}-ethyl)-amide;
   6-Methyl-imidazo[2,1-b]thiazole-5-carboxylic acid {2-[cyclopropyl-(2-methyl-5-*p*-tolyl-thiazole-4-carbonyl)-amino]-ethyl}-amide;
   6-Methyl-imidazo[2,1-b]thiazole-5-carboxylic acid {2-[cyclopropyl-(3'-methyl-biphenyl-2-carbonyl)-amino]-ethyl}-amide;
   6-Methyl-imidazo[2,1-b]thiazole-5-carboxylic acid {2-[cyclopropyl-(4'-methyl-biphenyl-2-carbonyl)-amino]-ethyl}-amide;
   6-Methyl-imidazo[2,1-b]thiazole-5-carboxylic acid {2-[cyclopropyl-(4'-fluoro-biphenyl-2-carbonyl)-amino]-ethyl}-amide;
   1-Methyl-1*H*-indazole-3-carboxylic acid {2-[cyclopropyl-(2-cyclopropyl-5-phenyl-thiazole-4-carbonyl)-amino]-ethyl}-amide;
   1-Methyl-1*H*-indazole-3-carboxylic acid {2-[cyclopropyl-(2-cyclopropyl-5-*m*-tolyl-thiazole-4-carbonyl)-amino]-ethyl}-amide;
   1-Methyl-1*H*-indazole-3-carboxylic acid (2-{cyclopropyl-[2-cyclopropyl-5-(3-methoxyphenyl)-thiazole-4-carbonyl]-amino}-ethyl)-amide;
   1-Methyl-1*H*-indazole-3-carboxylic acid {2-[(2-amino-5-*m*-tolyl-thiazole-4-carbonyl)-cyclopropyl-amino]-ethyl}-amide;
   1-Methyl-1*H*-indazole-3-carboxylic acid (2-{[2-amino-5-(3-fluoro-phenyl)-thiazole-4-carbonyl]-cyclopropyl-amino}ethyl)-amide;
   1-Methyl-1*H*-indazole-3-carboxylic acid (2-{[2-amino-5-(3-methoxy-phenyl)-thiazole-4-carbonyl]-cyclopropyl-amino}-ethyl)-amide;
   1-Methyl-1*H*-indazole-3-carboxylic acid (2-{[2-amino-5-(4-fluoro-phenyl)-thiazole-4-carbonyl]-cyclopropyl-amino}-ethyl)-amide;
   1-Methyl-1*H*-indazole-3-carboxylic acid (2-{[2-amino-5-(3-chloro-phenyl)-thiazole-4-carbonyl]-cyclopropyl-amino}-ethyl)-amide;
   1-Methyl-1*H*-indazole-3-carboxylic acid {2-[(2-amino-5-phenyl-thiazole-4-carbonyl)-cyclopropyl-amino]-ethyl}-amide;
   1-Methyl-1*H*-indazole-3-carboxylic acid (2-{[2-bromo-5-(3-methoxy-phenyl)-thiazole-4-carbonyl]-cyclopropyl-amino}-ethyl)-amide;
   1-Methyl-1*H*-indazole-3-carboxylic acid {2-[(2-bromo-5-*p*-tolyl-thiazole-4-carbonyl)-cyclopropyl-amino]-ethyl}-amide;
   1-Methyl-1*H*-indazole-3-carboxylic acid (2-{cyclopropyl-[5-(2-fluoro-phenyl)-2-methylthiazole-4-carbonyl]-amino}-ethyl)-amide;
   1-Methyl-1*H*-indazole-3-carboxylic acid (2-{cyclopropyl-[2-methyl-5-(3-trifluoromethylphenyl)-thiazole-4-carbonyl]-amino}-ethyl)-amide;
   1-Methyl-1*H*-indazole-3-carboxylic acid (2-{cyclopropyl-[5-(3-fluoro-phenyl)-2-methylthiazole-4-carbonyl]-amino}-ethyl)-amide;
   1-Methyl-1*H*-indazole-3-carboxylic acid (2-{cyclopropyl-[2-methyl-5-(4-trifluoromethylphenyl)-thiazole-4-carbonyl]-amino}-ethyl)-amide;
   1-Methyl-1*H*-indazole-3-carboxylic acid (2-{[5-(4-chloro-phenyl)-2-methyl-thiazole-4-carbonyl]-cyclopropyl-amino}-ethyl)-amide;
   1-Methyl-1*H*-indazole-3-carboxylic acid (2-{cyclopropyl-[5-(3-methoxy-phenyl)-2-methyl-thiazole-4-carbonyl]-amino}-ethyl)-amide;
   1-Methyl-1*H*-indazole-3-carboxylic acid (2-{[5-(3-cyano-phenyl)-2-methyl-thiazole-4-carbonyl]-cyclopropyl-amino}-ethyl)-amide;
   1-Methyl-1*H*-indazole-3-carboxylic acid (2-{cyclopropyl-[5-(3,4-dimethyl-phenyl)-2-methyl-thiazole-4-carbonyl]-amino}-ethyl)-amide;
   1-Methyl-1*H*-indazole-3-carboxylic acid {2-[cyclopropyl-(2-methyl-5-*p*-tolyl-thiazole-4-carbonyl)-amino]-ethyl}-amide;
   1-Methyl-1*H*-indazole-3-carboxylic acid {2-[cyclopropyl-(3'-methyl-biphenyl-2-carbonyl)-amino]-ethyl}-amide;
   1-Methyl-1*H*-indazole-3-carboxylic acid {2-[cyclopropyl-(4'-methyl-biphenyl-2-carbonyl)-amino]-ethyl}-amide;
   1-Methyl-1*H*-indazole-3-carboxylic acid {2-[cyclopropyl-(4'-fluoro-biphenyl-2-carbonyl)-amino]-ethyl}-amide;
   2-Cyclopropyl-5-phenyl-thiazole-4-carboxylic acid {2-[(benzofuran-4-carbonyl)-amino]-ethyl}-cyclopropyl-amide;
   2-Cyclopropyl-5-*m*-tolyl-thiazole-4-carboxylic acid {2-[(benzofuran-4-carbonyl)-amino]-ethyl}-cyclopropyl-amide;
   2-Cyclopropyl-5-(3-methoxy-phenylythiazole-4-carboxylic acid {2-[(benzofuran-4-carbonyl)-amino]-ethyl}-cyclopropyl-amide;
   2-Amino-5-*m*-tolyl-thiazole-4-carboxylic acid {2-[(benzofuran-4-carbonyl)-amino]-ethyl}-cyclopropyl-amide;
   2-Amino-5-(3-fluoro-phenyl)-thiazole-4-carboxylic acid {2-[(benzofuran-4-carbonyl)-amino]-ethyl}-cyclopropyl-amide;
   2-Amino-5-(3-methoxy-phenyl)-thiazole-4-carboxylic acid {2-[(benzofuran-4-carbonyl)-amino]-ethyl}-cyclopropyl-amide;
   2-Amino-5-(4-fluoro-phenyl)-thiazole-4-carboxylic acid {2-[(benzofuran-4-carbonyl)-amino]-ethyl}-cyclopropyl-amide;
   2-Amino-5-(3-chloro-phenyl)-thiazole-4-carboxylic acid {2-[(benzofuran-4-carbonyl)-amino]-ethyl}-cyclopropyl-amide;
   2-Amino-5-phenyl-thiazole-4-carboxylic acid {2-[(benzofuran-4-carbonyl)-amino]-ethyl}-cyclopropyl-amide;
   2-Bromo-5-(3-methoxy-phenylrthiazole-4-carboxylic acid {2-[(benzofuran-4-carbonyl)-amino]-ethyl}-cyclopropyl-amide;
   2-Bromo-5-*p*-tolyl-thiazole-4-carboxylic acid {2-[(benzofuran-4-carbonyl)-amino]-ethyl}-cyclopropyl-amide;
   5-(2-Fluoro-phenyl)-2-methyl-thiazole-4-carboxylic acid {2-[(benzofuran-4-carbonyl)-amino]-ethyl}-cyclopropyl-amide;
   2-Methyl-5-(3-trifluoromethyl-phenyl)-thiazole-4-carboxylic acid {2-[(benzofuran-4-carbonyl)-amino]-ethyl}cyclopropyl-amide;
   5-(3-Fluoro-phenyl)-2-methyl-thiazole-4-carboxylic acid {2-[(benzofuran-4-carbonyl)-amino]-ethyl}-cyclopropyl-amide;
   2-Methyl-5-(4-trifluoromethyl-phenyl)-thiazole-4-carboxylic acid {2-[(benzofuran-4-carbonylramino]-ethyl}-cyclopropyl-amide;
   5-(4-Chloro-phenyl)-2-methyl-thiazole-4-carboxylic acid {2-[(benzofuran-4-carbonyl)-amino]-ethyl}-cyclopropyl-amide;
   5-(3-Methoxy-phenyl)-2-methyl-thiazole-4-carboxylic acid {2-[(benzofuran-4-carbonyl)-amino]-ethyl}-cyclopropyl-amide;
   5-(3-Cyano-phenyl)-2-methyl-thiazole-4-carboxylic acid {2-[(benzofuran-4-carbonyl)-amino]-ethyl}-cyclopropyl-amide;
   5-(3,4-Dimethyl-phenyl)-2-methyl-thiazole-4-carboxylic acid {2-[(benzofuran-4-carbonyl)-amino]-ethyl}-cyclopropyl-amide;
   2-Methyl-5-*p*-tolyl-thiazole-4-carboxylic acid {2-[(benzofuran-4-carbonyl)-amino]-ethyl}-cyclopropyl-amide;
   Benzofuran-4-carboxylic acid {2-[cyclopropyl-(3'-methyl-biphenyl-2-carbonyl)-amino]-ethyl}-amide;
   Benzofuran-4-carboxylic acid {2-[cyclopropyl-(4'-methyl-biphenyl-2-carbonyl)-amino]-ethyl}-amide;
   Benzofuran-4-carboxylic acid (2-[cyclopropyl-(4'-fluoro-biphenyl-2-carbonyl)-amino]-ethyl}-amide;
   6-Methyl-imidazo[2,1-b]thiazole-5-carboxylic acid {2-[cyclopropylmethyl-(2-cyclopropyl-5-phenyl-thiazole-4-carbonyl)-amino]-ethyl}-amide;
   6-Methyl-imidazo[2,1-b]thiazole-5-carboxylic acid {2-[cyclopropylmethyl-(2-cyclopropyl-5-*m*-tolyl-thiazole-4-carbonyl)-amino]-ethyl}-amide;
   6-Methyl-imidazo[2,1-b]thiazole-5-carboxylic acid (2-{[2-cyclopropyl-5-(3-methoxyphenyl)-thiazole-4-carbonyl]-cyclopropylmethyl-amino}-ethyl)-amide;
   6-Methyl-imidazo[2,1-b]thiazole-5-carboxylic acid {2-[(2-amino-5-*m*-tolyl-thiazole-4-carbonyl)-cyclopropylmethyl-amino]-ethyl}-amide;
   6-Methyl-imidazo[2,1-b]thiazole-5-carboxylic acid (2-{[2-amino-5-(3-fluoro-phenyl)-thiazole-4-carbonyl]-cyclopropylmethyl-amino}-ethyl)amide;
   6-Methyl-imidazo[2,1-b]thiazole-5-carboxylic acid (2-{[2-amino-5-(3-methoxy-phenyl)-thiazole-4-carbonyl]-cyclopropylmethyl-amino}-ethyl)-amide;
   6-Methyl-imidazo[2,1-b]thiazole-5-carboxylic acid (2-{[2-amino-5-(4-fluoro-phenyl)-thiazole-4-carbonyl]-cyclopropylmethyl-amino}-ethyl}-amide;
   6-Methyl-imidazo[2,1-b]thiazole-5-carboxylic acid (2-{[2-amino-5-(3-chloro-phenyl)-thiazole-4-carbonyl]-cyclopropylmethyl-amino}-ethyl)-amide;
   6-Methyl-imidazo[2,1-b]thiazole-5-carboxylic acid {2-[(2-amino-5-phenyl-thiazole-4-carbonyl)-cyclopropylmethyl-amino]-ethyl}-amide;
   6-Methyl-imidazo[2,1-b]thiazole-5-carboxylic acid (2-{[2-bromo-5-(3-methoxy-phenyl)-thiazole-4-carbonyl]-cyclopropylmethyl-amino}-ethyl)-amide;
   6-Methyl-imidazo[2,1-b]thiazole-5-carboxylic acid {2-[(2-bromo-5-*p*-tolyl-thiazole-4-carbonyl)-cyclopropylmethyl-amino]-ethyl}-amide;
   6-Methyl-imidazo[2,1-b]thiazole-5-carboxylic acid (2-{cyclopropylmethyl-[5-(2-fluorophenyl)-2-methyl-thiazole-4-carbonyl]-amino}-ethyl)-amide;
   6-Methyl-imidazo[2,1-b]thiazole-5-carboxylic acid (2-{cyclopropylmethyl-[2-methyl-5-(3-trifluoromethyl-phenyl)-thiazole-4-carbonyl]-amino}-ethyl)-amide;
   6-Methyl-imidazo[2,1-b]thiazole-5-carboxylic acid (2-{cyclopropylmethyl-[5-(3-fluorophenyl)-2-methyl-thiazole-4-carbonyl]-amino}-ethyl)-amide;
   6-Methyl-imidazo[2,1-b]thiazole-5-carboxylic acid (2-{cyclopropylmethyl-[2-methyl-5-(4-trifluoromethyl-phenyl)-thiazole-4-carbonyl]-amino}-ethyl)-amide;
   6-Methyl-imidazo[2,1-b]thiazole-5-carboxylic acid (2-{[5-(4-chloro-phenyl)-2-methylthiazole-4-carbonyl]-cyclopropylmethyl-amino}-ethyl)-amide;
   6-Methyl-imidazo[2,1-b]thiazole-5-carboxylic acid (2-{cyclopropylmethyl-[5-(3-methoxy-phenyl)-2-methyl-thiazole-4-carbonyl]-amino}-ethyl)-amide;
   6-Methyl-imidazo[2,1-b]thiazole-5-carboxylic acid (2-{[5-(3-cyano-phenyl)-2-methylthiazole-4-carbonyl]-cyclopropylmethyl-amino}-ethyl)-amide;
   6-Methyl-imidazo[2,1-b]thiazole-5-carboxylic acid (2-{cyclopropylmethyl-[5-(3,4-dimethyl-phenyl)-2-methyl-thiazole-4-carbonyl]-amino}-ethyl)-amide;
   6-Methyl-imidazo[2,1-b]thiazole-5-carboxylic acid {2-[cyclopropylmethyl-(2-methyl-5-*p*-tolyl-thiazole-4-carbonylhamino]-ethylfamide;
   6-Methyl-imidazo[2,1-b]thiazole-5-carboxylic acid {2-[cyclopropylmethyl-(3'-methylbiphenyl-2-carbonyl)-amino]-ethylfamide;
   6-Methyl-imidazo[2,1-b]thiazole-5-carboxylic acid {2-[cyclopropylmethyl-(4'-methylbiphenyl-2-carbonyl)-amino]-ethyl}-amide;
   1-Methyl-1*H*-indazole-3-carboxylic acid {2-[cyclopropylmethyl-(2-cyclopropyl-5-phenyl-thiazole-4-carbonyl)-amino]-ethyl}-amide;
   1-Methyl-1*H*-indazole-3-carboxylic acid {2-[cyclopropylmethyl-(2-cyclopropyl-5-*m-*tolyl-thiazole-4-carbonylhamino]-ethylfamide;
   1-Methyl-1*H*-indazole-3-carboxylic acid (2-{[2-cyclopropyl-5-(3-methoxy-phenyl)-thiazole-4-carbonyl]-cyclopropylmethyl-amino}-ethyl)-amide;
   1-Methyl-1*H*-indazole-3-carboxylic acid {2-[(2-amino-5-*m*-tolyl-thiazole-4-carbonyl)-cyclopropylmethyl-amino]-ethyl}-amide;
   1-Methyl-1*H*-indazole-3-carboxylic acid (2-{[2-amino-5-(3-fluoro-phenyl)-thiazole-4-carbonyl]-cyclopropylmethyl-amino}ethyl)-amide;
   1-Methyl-1*H*-indazole-3-carboxylic acid (2-{[2-amino-5-(3-methoxy-phenyl)-thiazole-4-carbonyl]-cyclopropylmethyl-amino}-ethyl)-amide;
   1-Methyl-1*H*-indazole-3-carboxylic acid (2-{[2-amino-5-(4-fluoro-phenyl)-thiazole-4-carbonyl]-cyclopropylmethyl-amino}-ethyl)-amide;
   1-Methyl-1*H*-indazole-3-carboxylic acid (2-{[2-amino-5-(3-chloro-phenyl)-thiazole-4-carbonyl]-cyclopropylmethyl-amino}-ethyl)-amide;
   1-Methyl-1*H*-indazole-3-carboxylic acid {2-[(2-amino-5-phenyl-thiazole-4-carbonyl)-cyclopropylmethyl-amino]-ethyl}-amide;
   1-Methyl-1*H*-indazole-3-carboxylic acid (2-{[2-bromo-5-(3-methoxy-phenyl)-thiazole-4-carbonyl]-cyclopropylmethyl-amino}-ethyl)-amide;
   1-Methyl-1*H*-indazole-3-carboxylic acid {2-[(2-bromo-5-*p*-tolyl-thiazole-4-carbonyl)-cyclopropylmethyl-amino]-ethyl}-amide;
   1-Methyl-1*H*-indazole-3-carboxylic acid (2-{cyclopropylmethyl-[5-(2-fluoro-phenyl)-2-methyl-thiazole-4-carbonyl]-amino}-ethyl)-amide;
   1-Methyl-1*H*-indazole-3-carboxylic acid (2-{cyclopropylmethyl-[2-methyl-5-(3-trifluoromethyl-phenyl)-thiazole-4-carbonyl]-amino}-ethyl)-amide;
   1-Methyl-1*H*-indazole-3-carboxylic acid (2-{cyclopropylmethyl-[5-(3-fluoro-phenyl)-2-methyl-thiazole-4-carbonyl]-amino}-ethyl)-amide;
   1-Methyl-1*H*-indazole-3-carboxylic acid (2-{cyclopropylmethyl-[2-methyl-5-(4-trifluoromethyl-phenyl)-thiazole-4-carbonyl]-amino}-ethyl)-amide;
   1-Methyl-1*H*-indazole-3-carboxylic acid (2-{[5-(4-chloro-phenyl)-2-methyl-thiazole-4-carbonyl]-cyclopropylmethyl-amino}-ethyl)-amide;
   1-Methyl-1*H*-indazole-3-carboxylic acid (2-{cyclopropylmethyl-[5-(3-methoxy-phenyl)-2-methyl-thiazole-4-carbonyl]-amino}-ethyl)-amide;
   1-Methyl-1*H*-indazole-3-carboxylic acid (2-{[5-(3-cyano-phenyl)-2-methyl-thiazole-4-carbonyl]-cyclopropylmethyl-amino}-ethyl)-amide;
   1-Methyl-1*H*-indazole-3-carboxylic acid (2-{cyclopropylmethyl-[5-(3,4-dimethylphenyl)-2-methyl-thiazole-4-carbonyl]-amino}-ethyl)-amide;
   1-Methyl-1*H*-indazole-3-carboxylic acid {2-[cyclopropylmethyl-(2-methyl-5-p-tolyl-thiazole-4-carbonyl)-amino]-ethyl}-amide;
   1-Methyl-1*H*-indazole-3-carboxylic acid {2-[cyclopropylmethyl-(3'-methyl-biphenyl-2-carbonyl}-amino]-ethyl}-amide;
   1-Methyl-1*H*-indazole-3-carboxylic acid {2-[cyclopropylmethyl-(4'-methyl-biphenyl-2-carbonyl)-amino]-ethyl}-amide;
   1-Methyl-1*H*-indazole-3-carboxylic acid {2-[cyclopropylmethyl-(4'-fluoro-biphenyl-2-carbonylhamino]-ethylfamide;
   2-Cyclopropyl-5-phenyl-thiazole-4-carboxylic acid cyclopropyl-{2-[(2,3-dihydro-benzo[1,4]dioxine-5-carbonyl)-amino]-ethyl}-amide;
   2-Cyclopropyl-5-*m*-tolyl-thiazole-4-carboxylic acid cyclopropyl-{2-[(2,3-dihydro-benzo[1,4]dioxine-5-carbonyl)-amino]-ethyl}-amide;
   2-Cyclopropyl-5-(3-methoxy-phenyl)-thiazole-4-carboxylic acid cyclopropyl-{2-[(2,3-dihydro-benzo[1,4]dioxine-5-carbonyl)-amino]-ethyl}-amide;
   2-Amino-5-*m*-tolyl-thiazole-4-carboxylic acid cyclopropyl-{2-[(2,3-dihydro-benzo[1,4]dioxine-5-carbonyl)-amino]-ethyl}-amide;
   2-Amino-5-(3-fluoro-phenyl)-thiazole-4-carboxylic acid cyclopropyl-{2-[(2,3-dihydro-benzo[1,4]dioxine-5-carbonyl)-amino]-ethyl}-amide;
   2-Amino-5-(3-methoxy-phenyl)-thiazole-4-carboxylic acid cyclopropyl-{2-[(2,3-dihydro-benzo[1,4]dioxine-5-carbonyl)-amino]-ethyl}-amide;
   2-Amino-5-(4-fluoro-phenyl)-thiazole-4-carboxylic acid cyclopropyl-{2-[(2,3-dihydro-benzo[1,4]dioxine-5-carbonyl)-amino]-ethyl}-amide;
   2-Amino-5-(3-chloro-phenyl)-thiazole-4-carboxylic acid cyclopropyl-{2-[(2,3-dihydro-benzo[1,4]dioxine-5-carbonyl)-amino]-ethyl}-amide;
   2-Amino-5-phenyl-thiazole-4-carboxylic acid cyclopropyl-{2-[(2,3-dihydro-benzo[1,4]dioxine-5-carbonyl)-amino]-ethyl}-amide;
   2-Bromo-5-(3-methoxy-phenyl)-thiazole-4-carboxylic acid cyclopropyl-{2-[(2,3-dihydro-benzo[1,4]dioxine-5-carbonyl)-amino]-ethyl}-amide;
   2-Bromo-5-p-tolyl-thiazole-4-carboxylic acid cyclopropyl-{2-[(2,3-dihydro-benzo[1,4]dioxine-5-carbonyl)-amino]-ethyl}-amide;
   5-(2-Fluoro-phenyl)-2-methyl-thiazole-4-carboxylic acid cyclopropyl-{2-[(2,3-dihydro-benzo[1,4]dioxine-5-carbonyl)-amino]-ethyl}-amide;
   2-Methyl-5-(3-trifluoromethyl-phenyl)-thiazole-4-carboxylic acid cyclopropyl-{2-[(2,3-dihydro-benzo[1,4]dioxine-5-carbonyl)-amino]-ethyl}-amide;
   5-(3-Fluoro-phenyl)-2-methyl-thiazole-4-carboxylic acid cyclopropyl-{2-[(2,3-dihydro-benzo[1,4]dioxine-5-carbonyl)-amino]-ethyl}-amide;
   5-(4-Chloro-phenyl)-2-methyl-thiazole-4-carboxylic acid cyclopropyl-{2-[(2,3-dihydro-benzo[1,4]dioxine-5-carbonyl)-amino]-ethyl}-amide;
   5-(3-Methoxy-phenyl)-2-methyl-thiazole-4-carboxylic acid cyclopropyl-{2-[(2,3-dihydro-benzo[1,4]dioxine-5-carbonyl)-amino]-ethyl}-amide;
   5-(3,4-Dimethyl-phenyl)-2-methyl-thiazole-4-carboxylic acid cyclopropyl-{2-[(2,3-dihydro-benzo[1,4]dioxine-5-carbonyl)-amino]-ethyl}-amide;
   2-Methyl-5-p-tolyl-thiazole-4-carboxylic acid cyclopropyl-{2-[(2,3-dihydro-benzo[1,4]dioxine-5-carbonyl)-amino]-ethyl}-amide;
   2,3-Dihydro-benzo[1,4]dioxine-5-carboxylic acid {2-[cyclopropyl-(3'-methyl-biphenyl-2-carbonyl)-amino]-ethyl}-amide;
   2,3-Dihydro-benzo[1,4]dioxine-5-carboxylic acid {2-[cyclopropyl-(4'-methyl-biphenyl-2-carbonyl)-amino]-ethyl}-amide;
   2,3-Dihydro-benzo[1,4]dioxine-5-carboxylic acid {2-[cyclopropyl-(4'-fluoro-biphenyl-2-carbonyl)-amino]-ethyl}-amide;
   2-Cyclopropyl-5-phenyl-thiazole-4-carboxylic acid cyclopropylmethyl-{2-[(2,3-dihydro-benzo[1,4]dioxine-5-carbonyl)-amino]-ethyl}-amide;
   2-Cyclopropyl-5-*m*-tolyl-thiazole-4-carboxylic acid cyclopropylmethyl-{2-[(2,3-dihydro-benzo[1,4]dioxine-5-carbonyl)-amino]-ethyl}-amide;
   2-Cyclopropyl-5-(3-methoxy-phenyl)-thiazole-4-carboxylic acid cyclopropylmethyl-{2-[(2,3-dihydro-benzo[1,4]dioxine-5-carbonyl)-amino]-ethyl}-amide;
   2-Amino-5-*m*-tolyl-thiazole-4-carboxylic acid cyclopropylmethyl-{2-[(2,3-dihydro-benzo[1,4]dioxine-5-carbonyl)-amino]-ethyl}-amide;
   2-Amino-5-(3-fluoro-phenyl)-thiazole-4-carboxylic acid cyclopropylmethyl-{2-((2,3-dihydro-benzo[1,4]dioxine-5-carbonyl)-amino]-ethyl}-amide;
   2-Amino-5-(3-methoxy-phenyl)-thiazole-4-carboxylic acid cyclopropylmethyl-{2-[(2,3-dihydro-benzo[1,4]dioxine-5-carbonyl)-amino]-ethyl}-amide;
   2-Amino-5-phenyl-thiazole-4-carboxylic acid cyclopropylmethyl-{2-[(2,3-dihydro-benzo[1,4]dioxine-5-carbonyl)-amino]-ethyl}-amide;
   2-Bromo-5-(3-methoxy-phenyl)-thiazole-4-carboxylic acid cyclopropylmethyl-{2-[(2,3-dihydro-benzo[1,4]dioxine-5-carbonyl)-amino]-ethyl}-amide;
   2-Bromo-5-*p*-tolyl-thiazole-4-carboxylic acid cyclopropylmethyl-{2-[(2,3-dihydro-benzo[1,4]dioxine-5-carbonyl)-amino]-ethyl}-amide;
   5-(2-Fluoro-phenyl)-2-methyl-thiazole-4-carboxylic acid cyclopropylmethyl-{2-[(2,3-dihydro-benzo[1,4]dioxine-5-carbonyl)-amino]-ethyl}-amide;
   2-Methyl-5-(3-trifluoromethyl-phenyl)-thiazole-4-carboxylic acid cyclopropylmethyl-{2-[(2,3-dihydro-benzo[1,4]dioxine-5-carbonyl)-amino]-ethyl}-amide;
   5-(3-Fluoro-phenyl)-2-methyl-thiazole-4-carboxylic acid cyclopropylmethyl-{2-[(2,3-dihydro-benzo[1,4]dioxine-5-carbonyl)-amino]-ethyl}-amide;
   5-(3-Methoxy-phenyl)-2-methyl-thiazole-4-carboxylic acid cyclopropylmethyl-{2-[(2,3-dihydro-benzo[1,4]dioxine-5-carbonyl)-amino]-ethyl}-amide;
   5-(3-Cyano-phenyl)-2-methyl-thiazole-4-carboxylic acid cyclopropylmethyl-{2-[(2,3-dihydro-benzo[1,4]dioxine-5-carbonyl)-amino]-ethyl}-amide;
   5-(3,4-Dimethyl-phenylh2-methyl-thiazole-4-carboxylic acid cyclopropylmethyl-{2-[(2,3-dihydro-benzo[1,4]dioxine-5-carbonyl)-amino]-ethyl}-amide;
   2-Methyl-5-p-tolyl-thiazole-4-carboxylic acid cyclopropylmethyl-{2-[(2,3-dihydro-benzo[1,4]dioxine-5-carbonyl)-amino]-ethyl}-amide;
   2,3-Dihydro-benzo[1,4]dioxine-5-carboxylic acid {2-[cyclopropylmethyl-(3'-methylbiphenyl-2-carbonyl)-amino]-ethyl}-amide;
   2,3-Dihydro-benzo[1,4]dioxine-5-carboxylic acid {2-[cyclopropylmethyl-(4'-methylbiphenyl-2-carbonyl)-amino]-ethyl}-amide;
   2,3-Dihydro-benzo[1,4]dioxine-5-carboxylic acid {2-[cyclopropylmethyl-(4'-fluoro-biphenyl-2-carbonyl)-amino]-ethyl}-amide;
   2-Cyclopropyl-5-phenyl-thiazole-4-carboxylic acid {2-[(benzofuran-4-carbonyl)-amino]-ethyl}-cyclopropylmethyl-amide;
   2-Cyclopropyl-5-*m*-tolyl-thiazole-4-carboxylic acid {2-[(benzofuran-4-carbonyl)-amino]-ethyl}-cyclopropylmethyl-amide;
   2-Cyclopropyl-5-(3-methoxy-phenyl)-thiazole-4-carboxylic acid {2-[(benzofuran-4-carbonyl)-amino]-ethyl}-cyclopropylmethyl-amide;
   2-Amino-5-*m*-tolyl-thiazole-4-carboxylic acid {2-[(benzofuran-4-carbonyl)-amino]-ethyl}-cyclopropylmethyl-amide;
   2-Amino-5-(3-fluoro-phenyl)-thiazole-4-carboxylic acid {2-[(benzofuran-4-carbonyl)-amino]-ethyl}-cyclopropylmethyl-amide;
   2-Amino-5-(3-methoxy-phenyl)-thiazole-4-carboxylic acid {2-[(benzofuran-4-carbonyl)-amino]-ethyl}-cyclopropylmethyl-amide;
   2-Amino-5-(4-fluoro-phenyl)-thiazole-4-carboxylic acid {2-[(benzofuran-4-carbonyl)-amino]-ethyl}-cyclopropylmethyl-amide;
   2-Amino-5-(3-chloro-phenyl)-thiazole-4-carboxylic acid {2-[(benzofuran-4-carbonyl)-amino]-ethyl}-cyclopropylmethyl-amide;
   2-Amino-5-phenyl-thiazole-4-carboxylic acid {2-[(benzofuran-4-carbonyl)-amino]-ethyl}-cyclopropylmethyl-amide;
   2-Bromo-5-(3-methoxy-phenyl)-thiazole-4-carboxylic acid {2-[(benzofuran-4-carbonyl)-amino]-ethyl}-cyclopropylmethyl-amide;
   2-Bromo-5-*p*-tolyl-thiazole-4-carboxylic acid {2-[(benzofuran-4-carbonyl)-amino]-ethyl}-cyclopropylmethyl-amide;
   5-(2-Fluoro-phenyl)-2-methyl-thiazole-4-carboxylic acid {2-[(benzofuran-4-carbonyl)-amino]-ethyl}-cyclopropylmethyl-amide;
   2-Methyl-5-(3-trifluoromethyl-phenyl)-thiazole-4-carboxylic acid {2-[(benzofuran-4-carbonyl)-amino]-ethyl}-cyclopropylmethyl-amide;
   5-(3-Fluoro-phenyl)-2-methyl-thiazole-4-carboxylic acid {2-[(benzofuran-4-carbonyl)-amino]-ethyl}-cyclopropylmethyl-amide;
   2-Methyl-5-(4-trifluoromethyl-phenyl)-thiazole-4-carboxylic acid {2-[(benzofuran-4-carbonyl)-amino]-ethyl}-cyclopropylmethyl-amide;
   5-(4-Chloro-phenyl)-2-methyl-thiazole-4-carboxylic acid {2-[(benzofuran-4-carbonyl)-amino]-ethyl}-cyclopropylmethyl-amide;
   5-(3-Methoxy-phenyl)-2-methyl-thiazole-4-carboxylic acid {2-[(benzofuran-4-carbonyl)-amino]-ethyl}-cyclopropylmethyl-amide;
   5-(3-Cyano-phenyl)-2-methyl-thiazole-4-carboxylic acid {2-[(benzofuran-4-carbonyl)-amino]-ethyl}-cyclopropylmethyl-amide;
   5-(3,4-Dimethyl-phenyl)-2-methyl-thiazole-4-carboxylic acid {2-[(benzofuran-4-carbonyl)-amino]-ethyl}-cyclopropylmethyl-amide;
   2-Methyl-5-*p*-tolyl-thiazole-4-carboxylic acid {2-[(benzofuran-4-carbonyl)-amino]-ethyl}-cyclopropylmethyl-amide;
   Benzofuran-4-carboxylic acid {2-[cyclopropylmethyl-(3'-methyl-biphenyl-2-carbonyl)-amino]-ethyl}-amide;
   Benzofuran-4-carboxylic acid {2-[cyclopropylmethyl-(4'-methyl-biphenyl-2-carbonyl)-amino]-ethyl}-amide; and
   Benzofuran-4-carboxylic acid {2-[cyclopropylmethyl-(4'-fluoro-biphenyl-2-carbonyl)-amino]-ethyl}-amide.

The compounds of formula (I) and their pharmaceutically acceptable salts can be used as medicaments, e.g. in the form of pharmaceutical compositions for enteral or parenteral administration.

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds of formula (I) or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

The present invention also relates to a method for the prevention or treatment of a disease or disorder mentioned herein comprising administering to a subject a pharmaceutically active amount of a compound of formula (I).

The compounds according to formula (I) may be used for the preparation of a medicament and are suitable for the prevention or treatment of diseases selected from the group consisting of dysthymic disorders including major depression and cyclothymia, affective neurosis, all types of manic depressive disorders, delirium, psychotic disorders, schizophrenia, catatonic schizophrenia, delusional paranoia, adjustment disorders and all clusters of personality disorders; schizoaffective disorders; anxiety disorders including generalized anxiety, obsessive compulsive disorder, posttraumatic stress disorder, panic attacks, all types of phobic anxiety and avoidance; separation anxiety; all psychoactive substance use, abuse, seeking and reinstatement; all types of psychological or physical addictions, dissociative disorders including multiple personality syndromes and psychogenic amnesias; sexual and reproductive dysfunction; psychosexual dysfunction and addiction; tolerance to narcotics or withdrawal from narcotics; increased anaesthetic risk, anaesthetic responsiveness; hypothalamic-adrenal dysfunctions; disturbed biological and circadian rhythms; sleep disturbances associated with diseases such as neurological disorders including neuropathic pain and restless leg syndrome; sleep apnea; narcolepsy; chronic fatigue syndrome; insomnias related to psychiatric disorders; all types of idiopathic insomnias and parasomnias; sleep-wake schedule disorders including jet-lag; all dementias and cognitive dysfunctions in the healthy population and in psychiatric and neurological disorders; mental dysfunctions of aging; all types of amnesia; severe mental retardation; dyskinesias and muscular diseases; muscle spasticity, tremors, movement disorders; spontaneous and medication-induced dyskinesias; neurodegenerative disorders including Huntington's, Creutzfeld-Jacob's, Alzheimer's diseases and Tourette syndrome; Amyotrophic lateral sclerosis; Parkinson's disease; Cushing's syndrome; traumatic lesions; spinal cord trauma; head trauma; perinatal hypoxia; hearing loss; tinnitus; demyelinating diseases; spinal and cranial nerve diseases; ocular damage; retinopathy; epilepsy; seizure disorders; absence seizures, complex partial and generalized seizures; Lennox-Gastaut syndrome; migraine and headache; pain disorders; anaesthesia and analgesia; enhanced or exaggerated sensitivity to pain such as hyperalgesia, causalgia, and allodynia; acute pain; burn pain; atypical facial pain; neuropathic pain; back pain; complex regional pain syndrome I and II; arthritic pain; sports injury pain; dental pain; pain related to infection e.g. by HIV; post-chemotherapy pain; post-stroke pain; postoperative pain; neuralgia; osteoarthritis; conditions associated with visceral pain such as irritable bowel syndrome; eating disorders; diabetes; toxic and dysmetabolic disorders including cerebral anoxia, diabetic neuropathies and alcoholism; appetite, taste, eating, or drinking disorders; somatoform disorders including hypochondriasis; vomiting/nausea; emesis; gastric dyskinesia; gastric ulcers; Kallman's syndrome (anosmia); impaired glucose tolerance; intestinal motility dyskinesias; hypothalamic diseases; hypophysis diseases; hyperthermia syndromes, pyrexia, febrile seizures, idiopathic growth deficiency; dwarfism; gigantism; acromegaly; basophil adenoma; prolactinoma; hyperprolactinemia; brain tumors, adenomas; benign prostatic hypertrophy, prostate cancer; endometrial, breast, colon cancer; all types of testicular dysfunctions, fertility control; reproductive hormone abnormalities; hot flashes; hypothalamic hypogonadism, functional or psychogenic amenorrhea; urinary bladder incontinence; asthma; allergies; all types of dermatitis, acne and cysts, sebaceous gland dysfunctions; cardiovascular disorders; heart and lung diseases, acute and congestive heart failure; hypotension; hypertension; dyslipidemias, hyperlipidemias, insulin resistance; urinary retention; osteoporosis; angina pectoris; myocardial infarction; arrhythmias, coronary diseases, left ventricular hypertrophy; ischemic or haemorrhagic stroke; all types of cerebrovascular disorders including subarachnoid haemorrhage, ischemic and hemorrhagic stroke and vascular dementia; chronic renal failure and other renal diseases; gout; kidney cancer; urinary incontinence; and other diseases related to general orexin system dysfunctions.

Compounds of formula (I) may be used for the preparation of a medicament and are particularly suitable for the treatment of diseases or disorders selected from the group consisting of all types of sleep disorders, of stress-related syndromes, of psychoactive substance use, abuse, seeking and reinstatement, of cognitive dysfunctions in the healthy population and in psychiatric and neurologic disorders, of eating or drinking disorders.

Eating disorders may be defined as comprising metabolic dysfunction; dysregulated appetite control; compulsive obesities; emeto-bulimia or anorexia nervosa. Pathologically modified food intake may result from disturbed appetite (attraction or aversion for food); altered energy balance (intake vs. expenditure); disturbed perception of food quality (high fat or carbohydrates, high palatability); disturbed food availability (unrestricted diet or deprivation) or disrupted water balance. Drinking disorders include polydipsias in psychiatric disorders and all other types of excessive fluid intake. Sleep disorders include all types of parasomnias, insomnias, narcolepsy and other disorders of excessive sleepiness, sleep-related dystonias; restless leg syndrome; sleep apneas; jet-lag syndrome; shift-work syndrome, delayed or advanced sleep phase syndrome or insomnias related to psychiatric disorders. Insomnias are defined as comprising sleep disorders associated with aging; intermittent treatment of chronic insomnia; situational transient insomnia (new environment, noise) or short-term insomnia due to stress; grief; pain or illness. Insomnia also include stress-related syndromes including post-traumatic stress disorders as well as other types and subtypes of anxiety disorders such as generalized anxiety, obsessive compulsive disorder, panic attacks and all types of phobic anxiety and avoidance. Psychoactive substance use, abuse, seeking and reinstatement are defined as all types of psychological or physical addictions and their related tolerance and dependence components. Cognitive dysfunctions include deficits in all types of attention, learning and memory functions occurring transiently or chronically in the normal, healthy, young, adult or aging population, and also occurring transiently or chronically in psychiatric, neurologic, cardiovascular and immune disorders.

In a further preferred embodiment of the invention compounds of formula (I) may be used for the preparation of a medicament and are particularly suitable for the treatment of diseases or disorders selected from the group consisting of sleep disorders that comprises all types of insomnias, narcolepsy and other disorders of excessive sleepiness, sleep-related dystonias, restless leg syndrome, sleep apneas, jet-lag syndrome, shift-work syndrome, delayed or advanced sleep phase syndrome or insomnias related to psychiatric disorders.

In another preferred embodiment of the invention compounds of formula (I) may be used for the preparation of a medicament and are particularly suitable for the treatment of diseases or disorders selected from the group consisting of cognitive dysfunctions that comprise deficits in all types of attention, learning and memory functions occurring transiently or chronically in the normal, healthy, young, adult or aging population, and also occurring transiently or chronically in psychiatric, neurologic, cardiovascular and immune disorders.

In another preferred embodiment of the invention compounds of formula (I) may be used for the preparation of a medicament and are particularly suitable for the preparation of a medicament for the treatment of diseases or disorders selected from the group consisting of eating disorders that comprise metabolic dysfunction; dysregulated appetite control; compulsive obesities; emeto-bulimia or anorexia nervosa.

In another preferred embodiment of the invention compounds of formula (I) may be used for the preparation of a medicament and are particularly suitable for the treatment of diseases or disorders selected from the group consisting of psychoactive substance use, abuse, seeking and reinstatement that comprise all types of psychological or physical addictions and their related tolerance and dependence components.

### Preparation of compounds of formula (I):

A further aspect of the invention is a process for the preparation of compounds of formula (I). Compounds according to formula (I) of the present invention can be prepared according to the sequence of reactions outlined in the schemes below wherein A, D, R¹, R², R³, R⁴, R⁵ and R⁶ are as defined for formula (I). Other abbreviations used are defined in the experimental section. In some instances the generic groups A, D, R¹, R², R³, R⁴, R⁵ and R⁶ might be incompatible with the assembly illustrated in the schemes below and so will require the use of protecting groups (PG). The use of protecting groups is well known in the art (see for example "Protective Groups in Organic Synthesis", T.W. Greene, P.G.M. Wuts, Wiley-Interscience, 1999). For the purposes of this discussion, it will be assumed that such protecting groups as are necessary are in place.

In general, all chemical transformations can be performed according to well-known standard methodologies as described in the literature or as described in the procedures below. The compounds obtained may also be converted into pharmaceutically acceptable salts thereof in a manner known *per se.*

1,2-Diamido-ethylene derivatives of formula (I) can be prepared from (2-amino-ethyl)-carbamic acid tert-butyl ester derivatives of structure (2) as shown in scheme 1. For the introduction of substituents R¹, wherein R¹ is other than hydrogen, compounds of structure (2) are reacted under reductive amination conditions with an appropriate, commercially available or well-known aldehyde in presence of a reducing agent such as NaBH₄ to provide compounds of structure (3). Acylation of amines of structure (3) with carboxylic acid derivatives A-COOH in the presence of a coupling reagent such as EDC with additives like HOAt results in the formation of amides of structure (4) which, after removal of the BOC-group under acidic conditions leading to compounds of structure (5), are acylated with carboxylic acid derivatives R³-COOH using amide coupling reagents such as HATU to give compounds of formula (I) according to scheme 1.

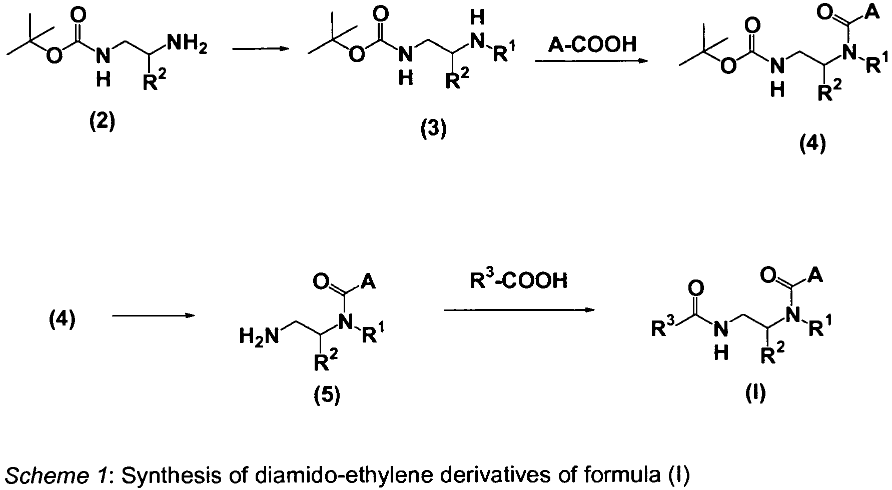

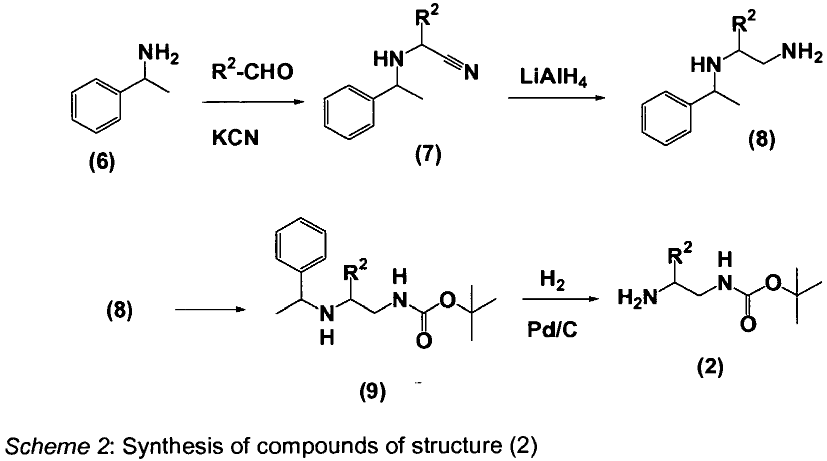

Compounds of structure (2) wherein R² represents hydrogen are commercially available. Alternatively, compounds of structure (2) can be prepared from commercially available, racemic or enantiomerically pure 1-phenyl-ethylamine (6). This amine is reacted with commercially available aldehydes of formula R²-CHO and KCN under conditions of a Strecker synthesis such as aq. HCl in a mixture of MeOH/water to yield α-aminonitriles of structure (7). Reduction of nitrile (7) can be performed in the presence of a reducing agent like LiAlH₄ in solvents such as ether leading to compounds of structure (8). Introduction of a Boc protecting group with agents such as Boc₂O in presence of a base such as NEt₃ lead to compounds of structure (9). Removal of the 1-phenyl-ethyl group under conditions of hydrogenation such as hydrogen in the presence of Pd/C under pressure leads to compounds of structure (2) as shown in scheme 2.

### Preparation of carboxylic acids A-COOH

Acids of the formula A-COOH are commercially available or synthesized according to methods described below.

Carboxylic acid derivatives A-COOH wherein A represents a thiazole-4-yl derivative are commercially available or can be synthesised according to scheme 3.

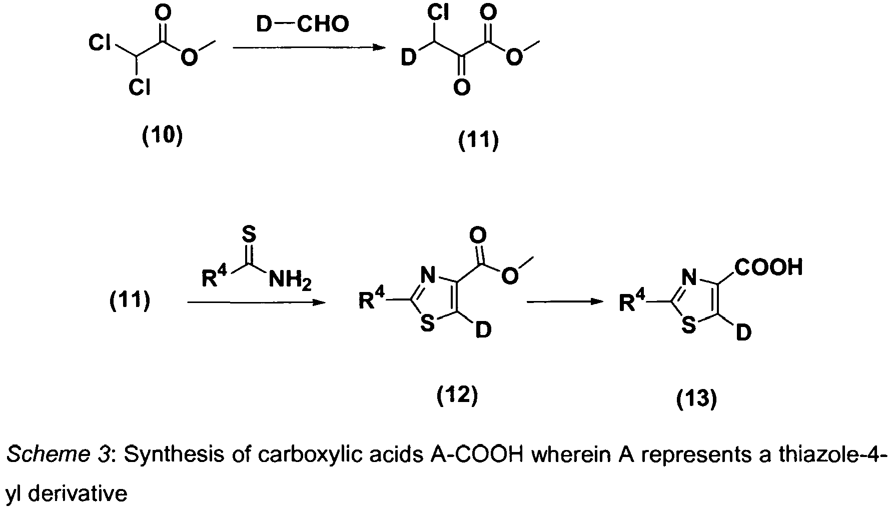

By reaction of methyl dichloroacetate (10) with an aldehyde of the formula D-CHO in the presence of a base such as KOtBu in an aprotic polar solvent such as THF at RT 3-lchloro-2-oxo-propionic acid ester derivatives (11) are obtained. Compounds of structure (15) can be transformed to thiazol-4-carboxylic acid ester derivatives (12) by reaction with thioamides or thioureas of formula R⁴-C(S)-NH₂ at RT in solvents such as MeCN. Thiazol-4-carboxylic acid ester derivatives (12), R⁴ being -NH₂, can be transformed into compounds (12), R⁴ being bromo, via Sandmeyer reaction. Thiazol-4-carboxylic acid ester derivatives (12), wherein R⁴ represents cyclopropyl, can alternatively be synthesized from compounds (12), R⁴ being bromo, via Pd-catalyzed Stille-coupling with tributyl-cyclopropyl-stannane (prepared from cyclopropyl magnesium bromide and tributyl tin chloride according to well established procedures). Saponification of the ester function using methods known in the art such as treatment with a base such as NaOH in a solvent such as MeOH provides the corresponding thiazol-4-carboxylic acid derivatives (13). Aldehydes of formula D-CHO are commercially available or well known in the art. Thioamides of formula R⁴-C(S)-NH₂, wherein R⁴ represents (C₁₋₄)alkyl or cyclopropyl are commercially available or, alternatively, can be synthesized from commercially available carboxamides with *Lawesson's* reagent.

Carboxylic acid derivatives A-COOH wherein A represents a phenyl-2-yl derivative are commercially available or can be synthesised according to scheme 4.

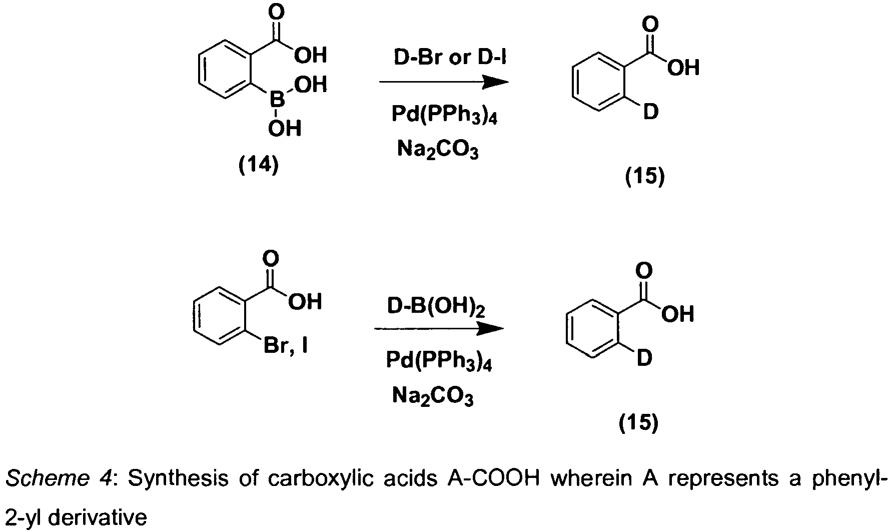

Reaction of commercially available (2-carboxyphenyl)-boronic acid derivatives (14) or esters thereof with commercially available aryl-bromides or aryl-iodides of formula D-Br or D-I in presence of a catalyst such as Pd(PPh₃)₄ and a base such as Na₂CO₃ under heating in a solvent such as toluene, dioxane, THF provides, after saponification, if needed, of the ester using well known methods, the corresponding phenyl-2-carboxylic acid derivatives (15). Alternatively, reaction of commercially available 2-bromo-, or 2-iodo-benzoic acid, or esters thereof, with commercially available boronic acid derivatives of formula D-B(OH)₂ using the conditions described before provides the corresponding phenyl-2-carboxylic acid derivatives (15).

### Synthesis of Carboxylic Acids R³-COOH

Carboxylic acids of formula R³-COOH are commercially available or well known in the art (Lit. e.g. WO2001/96302; T. Eicher, S. Hauptmann "The chemistry of Heterocycles: Structure, Reactions, Syntheses, and Applications", 2nd Edition 2003, Wiley, ISBN 978-3-527-30720-3).

Carboxylic acid derivatives R³-COOH which represent an imidazo[2,1-b]thiazole-5-carboxylic acid derivative are commercially available or can be synthesised according to the literature (see for example WO1995/029922 or according to scheme 5.

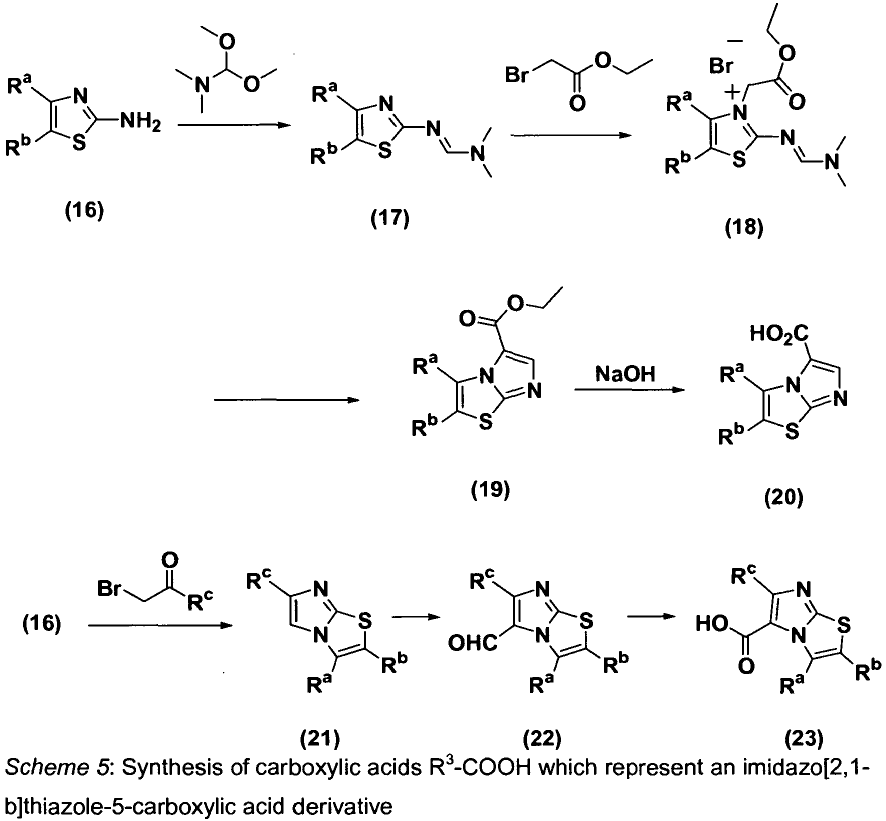

By heating a compound of structure (16) with *N,N*-dimethylformamide dimethylacetal in a solvent such as toluene formamidine derivatives (17) can be obtained. They can be alkylated with ethyl bromoacetate yielding the respective thiazolium bromide (18), which can be cyclised with strong bases such as DBU to the ester (19). Saponification of the ester function using methods known in the art such as treatment with a base such as NaOH in a solvent such as EtOH/water provides the corresponding imidazo[2,1-b]thiazole-5-carboxylic acid derivatives (20).

Alternatively, acids of structure (23) wherein R^{c} represents methyl, chloro, or trifluoromethyl can be synthesized by alkylating and cyclizing compounds of structure (16) with bromoacetone, chloroacetaldehyde, or 3-bromo-1,1,1-trifluoro-acetone, respectively, followed by formylation of the obtained imidazo[2,1-b]thiazole (21) in position 5 with POCl₃/DMF and oxidation of the obtained aldehyde (22) to the corresponding carboxylic acid according to well known methods. In scheme 5 preferably R^{a} and R^{b} independently represent hydrogen or methyl and R^{c} preferably represents hydrogen, methyl, chloro, or trifluoromethyl.

Whenever the compounds of formula (I) are obtained in the form of mixtures of enantiomers, the enantiomers can be separated using methods known to the one skilled in the art: e.g. by formation and separation of diastereomeric salts or by HPLC over a chiral stationary phase such as a Regis Whelk-O1(R,R) (10 µm) column, a Daicel ChiralCel OD-H (5-10 µm) column, or a Daicel ChiralPak IA (10 µm) or AD-H (5 µm) column. Typical conditions of chiral HPLC are an isocratic mixture of eluent A (EtOH, in presence or absence of an amine such as NEt₃, diethylamine) and eluent B (hexane), at a flow rate of 0.8 to 150 mL/min.

### Experimental Section

### Abbrevations (as used herein):

- aq.: aqueous
- anh.: anhydrous
- Boc: *tert*.-butoxycarbonyl
- Boc₂O: di-*tert*.-butyl dicarbonate
- BSA: bovine serum albumine
- CHO: chinese hamster ovary
- DBU: 1,8-diazabicyclo[5.4.0]undec-7-ene
- DCM: dichloromethane
- DIPEA: diisopropylethylamine
- DMF: *N,N*-dimethylformamide
- DMSO: dimethylsulfoxide
- EA: ethyl acetate
- eq: equivalent(s)
- ES: electron spray
- ether: diethylether
- EtOH: ethanol
- FC: flash chromatography
- FCS: foatal calf serum
- FLIPR: fluorescent imaging plate reader
- h: hour(s)
- HATU: (O-(7-azabenzotriazol-1-yl)-1,1,3,3-tetramethyl-uronium hexafluorphoshate
- HOAt: [1,2,3]Triazolo[4,5-b]pyridin-3-ol
- HBSS: Hank's balanced salt solution
- HEPES: 4-(2-hydroxyethyl)-piperazine-1-ethanesulfonic acid
- HPLC: high performance liquid chromatography
- IsOH: propan-2-ol
- KOtBu: potassium tert. butoxide
- LC: liquid chromatography
- M: molar(ity)
- MeCN: acetonitrile
- MeOH: methanol
- min: minute(s)
- MS: mass spectroscopy
- NaBH₄: sodium borohydride
- NEt₃: triethylamine
- org.: organic
- prep.: preparative
- RT: room temperature
- sat.: saturated
- sec.: secundary
- t_{R}: retention time
- tert.: tertiary
- TFA: trifluoroacetic acid
- THF: tetrahydrofuran

### I-Chemistry

All temperatures are stated in °C. Compounds are characterized by ¹H-NMR (300 MHz: Varian Oxford or 400 MHz: Bruker Avance); chemical shifts are given in ppm relative to the solvent used; multiplicities: s = singlet, d = doublet, t = triplet; q = quartett, p = pentuplet, hex = hexet, hept = heptet, m = multiplet, br = broad, coupling constants are given in Hz); by LC-MS (Finnigan Navigator with HP 1100 Binary Pump and DAD, column: 4.6x50 mm, Zorbax SB-AQ, 5 µm, 120 A, using the following conditions: acidic: eluent A: MeCN, eluent B: TFA in water (0.4 mL/L), 5% to 95% CH₃CN), t_{R} is given in min; by TLC (TLC-plates from Merck, Silica gel 60 F₂₅₄); or by melting point. Compounds are purified by column chromatography (FC) on silica gel or by preparative HPLC (column: X-terra RP18, 50x19 mm, 5 µm, gradient: 10-95% MeCN in water containing 0.5 % of formic acid). Racemates can be separated into their enantiomers by chiral preparative HPLC.

The following examples illustrate the preparation of pharmacologically active compounds of the invention but do not at all limit the scope thereof.

### Preparation of precursors and intermediates:

### A.1 Synthesis of thiazole-carboxylic acid derivatives

### A.1.1 Synthesis of 3-chloro-2-oxo-propionic ester derivatives (general procedure)

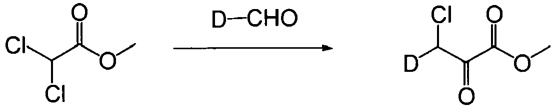

A solution of the respective aldehyde D-CHO (338 mmol, 1.0 eq) and methyl dichloroacetate (338 mmol, 1.0 eq) in THF (100 mL) is added dropwise to a cold (-60°C) suspension of KOtBu (335 mmol, 1.0 eq) in THF (420 mL). After 4 h the mixture is allowed to reach RT, stirred over night and concentrated in vacuo. DCM and ice-cold water are added, the layers are separated and the aq. layer is extracted twice with DCM. The combined org. layers are washed with ice-cold water and brine, dried over MgSO₄ and concentrated in vacuo to give the corresponding 3-chloro-2-oxo-propionic acid methyl ester derivative which is used without further purification.
**3-Chloro-2-oxo-3-phenyl-propionic acid methyl ester**
   prepared by reaction of benzaldehyde with methyl dichloroacetate.
**3-Chloro-2-oxo-3-(cyano-phenyl)-propionic acid methyl ester**
   prepared by reaction of 3-cyano-benzaldehyde with methyl dichloroacetate
**3-Chloro-2-oxo-3-*m*-tolyl-propionic acid methyl ester**
   prepared by reaction of 3-methyl-benzaldehyde with methyl dichloroacetate.
**3-Chloro-2-oxo-3-*p*-tolyl-propionic acid methyl ester**
   prepared by reaction of 4-methyl-benzaldehyde with methyl dichloroacetate.
**3-Chloro-3-(2-fluoro-phenyl)-2-oxo-propionic acid methyl ester**
   prepared by reaction of 3-fluoro-benzaldehyde with methyl dichloroacetate.
**3-Chloro-3-(3-fluoro-phenyl)-2-oxo-propionic acid methyl ester**
   prepared by reaction of 3-fluoro-benzaldehyde with methyl dichloroacetate.
**3-Chloro-3-(4-fluoro-phenyl)-2-oxo-propionic acid methyl ester**
   prepared by reaction of 4-fluoro-benzaldehyde with methyl dichloroacetate.
**3-Chloro-2-oxo-3-(3-trifluoromethyl-phenyl)-propionic acid methyl ester**
   prepared by reaction of 3-trifluoromethyl-benzaldehyde with methyl dichloro-acetate.
**3-Chloro-2-oxo-3-(4-trifluoromethyl-phenyl)-propionic acid methyl ester**
   prepared by reaction of 4-trifluoromethyl-benzaldehyde with methyl dichloro-acetate.
**3-Chloro-3-(3-chloro-phenyl)-2-oxo-propionic acid methyl ester**
   prepared by reaction of 3-chloro-benzaldehyde with methyl dichloro-acetate.
**3-Chloro-3-(4-chloro-phenyl)-2-oxo-propionic acid methyl ester**
   prepared by reaction of 4-chloro-benzaldehyde with methyl dichloro-acetate.
**3-Chloro-3-(3-methoxy-phenyl)-2-oxo-propionic acid methyl ester**
   prepared by reaction of 3-methoxy-benzaldehyde with methyl dichloro-acetate.
**3-Chloro-3-(3,4-dimethyl-phenyl)-2-oxo-propionic acid methyl ester**
   prepared by reaction of 3,4-dimethyl-benzaldehyde with methyl dichloro-acetate.

### A.1.2 Synthesis of 2-methyl-thiazole-4-carboxylic acid methyl ester derivatives (general procedure)

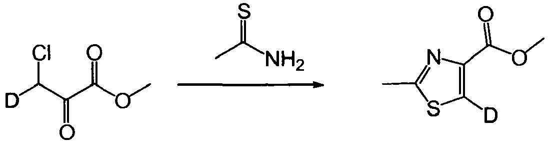

A solution of thioacetamide (132 mmol, 1.0 eq) in MeCN (250 mL) is added to a mixture of the respective 3-chloro-2-oxo-propionic acid methyl ester derivative (132 mmol, 1.0 eq) and molecular sieves (4A, 12 g) in MeCN (60 mL). After stirring for 5 h the mixture is cooled in an ice-bath and the obtained precipitate is filtered off. The residue is washed with cold MeCN, dried, dissolved in MeOH (280 mL) and stirred at 50°C for 6 h. The solvents are removed in vacuo to give the corresponding 2-methyl-thiazole-4-carboxylic acid methyl ester derivatives.
**5-Phenyl-2-methyl-thiazole-4-carboxylic acid methyl ester**
   prepared by reaction of 3-chloro-3-phenyl-2-oxo-propionic acid methyl ester with thioacetamide. LC-MS: t_{R} = 0.88 min; [M+H]⁺ = 234.23.
**2-Methyl-5-*p*-tolyl-thiazole-4-carboxylic acid methyl ester**
   prepared by reaction of 3-chloro-2-oxo-3-*p*-tolyl-propionic acid methyl ester with thioacetamide. LC-MS: t_{R} = 0.93 min; [M+H]⁺ = 248.02.
**5-(2-Fluoro-phenyl)-2-methyl-thiazole-4-carboxylic acid methyl ester**
   prepared by reaction of 3-chloro-3-(2-fluoro-phenyl)-2-oxo-propionic acid methyl ester with thioacetamide. LC-MS: t_{R} = 0.89 min; [M+H]⁺ = 251.99.
**5-(3-Fluoro-phenyl)-2-methyl-thiazole-4-carboxylic acid methyl ester**
   prepared by reaction of 3-chloro-3-(3-fluoro-phenyl)-2-oxo-propionic acid methyl ester with thioacetamide. LC-MS: t_{R} = 0.91 min; [M+H]⁺ = 252.1.
**5-(4-Fluoro-phenyl)-2-methyl-thiazole-4-carboxylic acid methyl ester**
   prepared by reaction of 3-chloro-3-(4-fluoro-phenyl)-2-oxo-propionic acid methyl ester with thioacetamide. ¹H-NMR (CDCl₃): δ = 2.75 (s, 3H); 3.84 (s, 3H); 7.10 (m, 2H); 7.47 (m, 2H).
**2-Methyl-5-(3-trifluoromethyl-phenyl)-thiazole-4-carboxylic acid methyl ester**
   prepared by reaction of 3-chloro-3-(3-trifluoromethyl-phenyl)-2-oxo-propionic acid methyl ester with thioacetamide. LC-MS: t_{R} = 0.99 min; [M+H]⁺ = 301.99.
**2-Methyl-5-(4-trifluoromethyl-phenyl)-thiazole-4-carboxylic acid methyl ester**
   prepared by reaction of 3-chloro-3-(4-trifluoromethyl-phenyl)-2-oxo-propionic acid methyl ester with thioacetamide. LC-MS: t_{R} = 0.99 min; [M+H]⁺ = 301.99.
**5-(4-Chloro-phenyl)-2-methyl-thiazole-4-carboxylic acid methyl ester**
   prepared by reaction of 3-chloro-3-(4-chloro-phenyl)-2-oxo-propionic acid methyl ester with thioacetamide. LC-MS: t_{R} = 0.94 min; [M+H]⁺ = 267
**5-(3-Methoxy-phenyl)-2-methyl-thiazole-4-carboxylic acid methyl ester**
   prepared by reaction of 3-chloro-3-(3-methoxy-phenyl)-2-oxo-propionic acid methyl ester with thioacetamide. LC-MS: t_{R} = 0.90 min; [M+H]⁺ = 263.87.
**2-Methyl-5-(3,4-dimethyl-phenyl)-thiazole-4-carboxylic acid methyl ester**
   prepared by reaction of 3-chloro-3-(3,4-dimethyl-phenyl)-2-oxo-propionic acid methyl ester with thioacetamide. LC-MS: t_{R} = 0.96 min; [M+H]⁺ = 262.34.
**2-Methyl-5-(3-cyano-phenyl)-thiazole-4-carboxylic acid methyl ester**
   prepared by reaction of 3-chloro-3-(3-cyano-phenyl)-2-oxo-propionic acid methyl ester with thioacetamide. LC-MS: t_{R} = 0.86 min; [M+H]⁺ = 259.31.

### A.1.3 Synthesis of 2-amino-thiazole-4-carboxylic acid methyl ester derivatives (general procedure)

A solution of the respective 3-chloro-2-oxo-propionic acid methyl ester derivative (22.1 mmol, 1.0 eq) in acetone (25 mL) is added to a suspension of thiourea (22.1 mmol, 1.0 eq) in acetone (45 mL). The mixture is heated to 57°C (bath temperature), stirred for 24 h and concentrated to half of the volume. The obtained suspension is filtered and the residue is washed with acetone. After drying the desired amino-thiazole derivative is obtained as a solid.
**2-Amino-5-*p*-tolyl-thiazole-4-carboxylic acid methyl ester**
   prepared by reaction of 3-chloro-2-oxo-3-*p*-tolyl-propionic acid methyl ester with thiourea. LC-MS: t_{R} = 0.77 min; [M+H]⁺ = 249.3.
**2-Amino-5-*m*-tolyl-thiazole-4-carboxylic acid methyl ester**
   prepared by reaction of 3-chloro-2-oxo-3-*m*-tolyl-propionic acid methyl ester with thiourea. LC-MS: t_{R} = 0.78 min; [M+H]⁺ = 249.0.
**2-Amino-5-(3-fluoro-phenyl)-thiazole-4-carboxylic acid methyl ester**
   prepared by reaction of 3-chloro-3-(3-fluoro-phenyl)-2-oxo-propionic acid methyl ester with thiourea. LC-MS: t_{R} = 0.78 min; [M+H]⁺ = 252.9.
**2-Amino-5-(4-fluoro-phenyl)-thiazole-4-carboxylic acid methyl ester**
   prepared by reaction of 3-chloro-3-(4-fluoro-phenyl)-2-oxo-propionic acid methyl ester with thiourea. LC-MS: t_{R} = 0.75 min; [M+H]⁺ = 253.
**2-Amino-5-(3-methoxy-phenyl)-thiazole-4-carboxylic acid methyl ester**
   prepared by reaction of 3-chloro-3-(3-methoxy-phenyl)-2-oxo-propionic acid methyl ester with thiourea. LC-MS: t_{R} = 0.75 min; [M+H]⁺ = 265.25
**2-Amino-5-(3-chloro-phenyl)-thiazole-4-carboxylic acid methyl ester**
   prepared by reaction of 3-chloro-3-(3-chloro-phenyl)-2-oxo-propionic acid methyl ester with thiourea. LC-MS: t_{R} = 0.82 min; [M+H]⁺ = 310.18.
**2-Amino-5-phenyl-thiazole-4-carboxylic acid methyl ester**
   prepared by reaction of 3-chloro-3-phenyl-2-oxo-propionic acid methyl ester with thiourea. LC-MS: t_{R} = 0.77 min; [M+H]⁺ = 235.

### A.1.4. Synthesis of thiazole-4-carboxylic acid derivatives (general procedure)

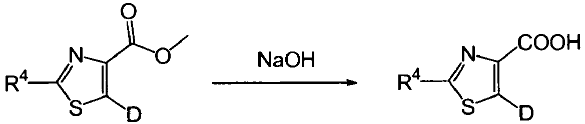

A solution of the respective thiazole-4-carboxylic acid methyl ester (96.2 mmol) in a mixture of THF (150 mL) and MeOH (50 mL) is treated with 1 M aq. NaOH (192 mL). After stirring for 3 h a white suspension is formed and the org. volatiles are removed in vacuo. The remaining mixture is diluted with water (100 mL), cooled in an ice-bath and acidified (pH = 3-4) by addition of 1 M aq. HCl. The suspension is filtered and the residue is washed with cold water. After drying the corresponding thiazole-4-carboxylic acid derivative is obtained.
**2-Methyl-5-phenyl-thiazole-4-carboxylic acid**
   prepared by saponification of 2-methyl-5-phenyl-thiazole-4-carboxylic acid methyl ester. LC-MS: t_{R} = 0.78 min; [M+H]⁺ = 220.01.
**2-Methyl-5-*p*-tolyl-thiazole-4-carboxylic acid**
   prepared by saponification of 2-methyl-5-*p*-tolyl-thiazole-4-carboxylic acid methyl ester. LC-MS: t_{R} = 0.83 min; [M+H]⁺ = 234.0.
**5-(2-Fluoro-phenyl)-2-methyl-thiazole-4-carboxylic acid**
   prepared by saponification of 5-(3-fluoro-phenylr2-methyl-thiazole-4-carboxylic acid methyl ester. LC-MS: t_{R} = 0.82 min; [M+H]⁺ = 238.1.
**5-(3-Fluoro-phenyl)-2-methyl-thiazole-4-carboxylic acid**
   prepared by saponification of 5-(3-fluoro-phenyl)-2-methyl-thiazole-4-carboxylic acid methyl ester. LC-MS: t_{R} = 0.82 min; [M+H]⁺ = 238.1.
**5-(4-Fluoro-phenyl)-2-methyl-thiazole-4-carboxylic acid**
   prepared by saponification of 5-(4-fluoro-phenyl)-2-methyl-thiazole-4-carboxylic acid methyl ester. ¹H-NMR (DMSO-d₆): δ = 2.67 (s, 3H); 7.27 (m, 2H); 7.53 (m, 2H); 12.89 (br.s, 1H).
**5-(4-Chloro-phenyl)-2-methyl-thiazole-4-carboxylic acid**
   prepared by saponification of 5-(4-chloro-phenyl)-2-methyl-thiazole-4-carboxylic acid methyl ester. LC-MS: t_{R} = 0.85 min; [M+H]⁺ = 253.
**2-Methyl-5-(3-trifluoromethyl-phenyl)-thiazole-4-carboxylic acid**
   prepared by saponification of 2-methyl-5-(3-trifluoromethyl-phenyl)-thiazole-4-carboxylic acid methyl ester. LC-MS: t_{R} = 0.88 min; [M+H]⁺ = 287.99.
**2-Methyl-5-(4-trifluoromethyl-phenyl)-thiazole-4-carboxylic acid**
   prepared by saponification of 2-methyl-5-(4-trifluoromethyl-phenyl)-thiazole-4-carboxylic acid methyl ester. LC-MS: t_{R} = 0.90 min; [M+H]⁺ = 287.99.
**2-Methyl-5-(3-methoxy-phenyl)-thiazole-4-carboxylic acid**
   prepared by saponification of 2-methyl-5-(3-methoxy-phenyl)-thiazole-4-carboxylic acid methyl ester. LC-MS: t_{R} = 0.80 min; [M+H]⁺ = 250.04.
**2-Methyl-5-(3,4-dimethyl-phenyl)-thiazole-4-carboxylic acid**
   prepared by saponification of 2-methyl-5-(3,4-dimethyl-phenyl)-thiazole-4-carboxylic acid methyl ester. LC-MS: t_{R} = 0.97 min; [M+H]⁺ = 382.38.
**2-Methyl-5-(3-cyano-phenyl)-thiazole-4-carboxylic acid**
   prepared by saponification of 2-methyl-5-(3-cyano-phenyl)-thiazole-4-carboxylic acid methyl ester. LC-MS: t_{R} = 0.76 min; [M+H]⁺ = 245.25.
**2-Amino-5-*p*-tolyl-thiazole-4-carboxylic acid**
   prepared by saponification of 2-amino-5-*m*-tolyl-thiazole-4-carboxylic acid methyl ester. LC-MS: t_{R} = 0.64 min; [M+H]⁺ = 235.24.
**2-Amino-5-*m*-tolyl-thiazole-4-carboxylic acid**
   prepared by saponification of 2-amino-5-*m*-tolyl-thiazole-4-carboxylic acid methyl ester. LC-MS: t_{R} = 0.65 min; [M+H]⁺ = 235.0.
**2-Amino-5-(3-fluoro-phenyl)-thiazole-4-carboxylic acid**
   prepared by saponification of 2-amino-5-(3-fluoro-phenyl)-thiazole-4-carboxylic acid methyl ester. LC-MS: t_{R} = 0.62 min; [M+H]⁺ = 239.1.
**2-Amino-5-(4-fluoro-phenyl)-thiazole-4-carboxylic acid**
   prepared by saponification of 2-amino-5-(4-fluoro-phenyl)-thiazole-4-carboxylic acid methyl ester. LC-MS: t_{R} = 0.61 min; [M+H]⁺ = 239.
**2-Amino-5-phenyl-thiazole-4-carboxylic acid**
   prepared by saponification of 2-amino-5-phenyl-thiazole-4-carboxylic acid methyl ester. LC-MS: t_{R} = 0.63 min; [M+H]⁺ = 221.
**2-Amino-5-(3-methoxy-phenyl)-thiazole-4-carboxylic acid**
   prepared by saponification of 2-amino-5-(3-methoxy-phenyl)-thiazole-4-carboxylic acid methyl ester. LC-MS: t_{R} = 0.63 min; [M+H]⁺ = 221.
**2-Amino-5-(3-chloro-phenyl)-thiazole-4-carboxylic acid**
   prepared by saponification of 2-amino-5-(3-chloro-phenyl)-thiazole-4-carboxylic acid methyl ester. LC-MS: t_{R} = 0.63 min; [M+H]⁺ = 221.

### A.1.5. Synthesis of 2-bromo-thiazole-4-carboxylic acid methyl ester derivatives

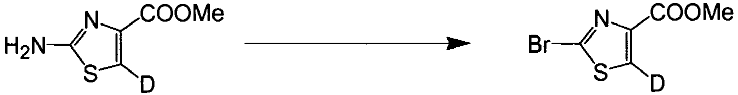

In an inert atmosphere, copper(II)bromide (47.3 mmol, 1.0 eq) was suspended in MeCN (200 ml) and cooled to 5-10°C followed by the addition of 3-methylbutylnitrite (71 mmol, 1.45 eq) over 15 min. To this reaction mixture the respective 2-aminothiazole derivative (47.3 mmol, 1 eq) was added in portions over 35 min. at 5-10°C. The reaction mixture was then carefully heated to 65°C and stirring continued for 2 h. The volatiles were removed under reduced pressure and the black residue was purified by FC (SiO₂; heptan / EA) to give the products as slightly yellow oils or solids.
**2-Bromo-5-*m*-tolyl-thiazole-4-carboxylic acid methyl ester**
   LC-MS: t_{R} = 1.01 min; [M+H]⁺ = 311.79/313.94.
**2-Bromo-5-phenyl-thiazole-4-carboxylic acid methyl ester**
   LC-MS: t_{R} = 1.00 min; [M+H]⁺ = 300.10..
**2-Bromo-5-*p*-tolyl-thiazole-4-carboxylic acid methyl ester**
   LC-MS: t_{R} = 1.01 min; [M+H]⁺ = 311.79/313.94.
**2-Bromo-5-(3-methoxy-phenyl)-thiazole-4-carboxylic acid methyl ester**
   LC-MS: t_{R} = 0.97 min; [M+H]⁺ = 318.17.

### A.1.6 Synthesis of 2-bromo-thiazole-4-carboxylic acid derivatives

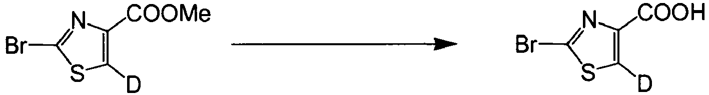

To a solution of the respective 2-bromo-5-arylthiazole-2-carboxylic acid methyl ester derivative (3.2 mmol, 1 eq) in THF (5 ml) and methanol (1 ml) was added sodium hydroxide solution (6 ml, 1 M). Stirring at room temperature was continued for 14 h. The volatiles were removed under reduced pressure followed by the addition of water (10 ml) to the residue. The pH of the solution was adjusted to 3-4 by the addition of aq HCl. The product precipitated and was isolated by filtration as a colorless solid.
**2-Bromo-5-*p*-tolyl-thiazole-4-carboxylic acid**
   LC-MS: t_{R} = 0.89 min; [M+H]⁺ = 300.23.
**2-Bromo-5-(3-methoxy-phenyl)-thiazole-4-carboxylic acid**
   LC-MS: t_{R} = 0.86 min; [M+H]⁺ = 316.16

### A.1.7. Synthesis of 2-cyclopropyl-thiazole-4-carboxylic acid methyl ester derivatives using the Stille reaction

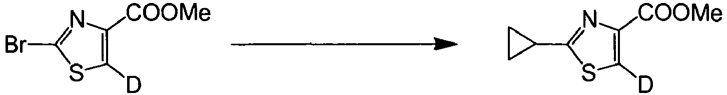

Cyclopropyl-tributyl-stannane (10.4 mmol, 1.1 eq) was dissolved in 1,2-dichloroethane followed by the addition of the respective 2-bromo-thiazole derivative (9.5 mmol, 1 eq). The mixture was degassed with Ar or N₂ for 5 min followed by the addition of bis(triphenylphosphine)palladium(II)dichloride (0.47 mmol, 0.05 eq). The reaction mixture was heated to 80°C and stirring continued for 24 h. The solvent was evaporated under reduced pressure and the residue was purified by FC (SiO₂ / heptane : EA = 2 : 1) to give the 2-cyclopropyl thiazole derivatives.
**2-Cyclopropyl-5-phenyl-thiazole-4-carboxylic acid methyl ester**
   LC-MS: t_{R} = 0.99 min; [M+H]⁺ = 260.45.
**2-Cyclopropyl-5-(3-methyl-phenyl)-thiazole-4-carboxylic acid methyl ester**
   LC-MS: t_{R} = 0.99 min; [M+H]⁺ = 274.26.
**2-Cyclopropyl-5-(3-methoxy-phenyl)-thiazole-4-carboxylic acid methyl ester**
   LC-MS: tR = 0.96 min; [M+H]+ = 290.30.

### A.1.8. Hydrolysis of the methyl esters to the carboxylic acids:

2-Cyclopropyl-5-phenyl-thiazole-4-carboxylic acid methyl ester (47.4 mmol, 1.0 eq.) was dissolved in 185 ml of a mixture of THF/IsOH (125/60, v/v). 1 M aq. NaOH (95 ml, 2.0 eq.) was added and stirring continued overnight. The solvent was stripped off and the residue treated with water (200ml) and adjusted to pH = 3-4 by addition of a solution of 10% citric acid in water. The aq. phase was extracted twice with each 200 ml of EE. The combined organic phases were dried over Na₂SO₄, filtered and evaporated to dryness yielding the desired acids in yields of ca. 85%, which were used for subsequent reactions without further purifications.
**2-Cyclopropyl-5-(3-methyl-phenyl)-thiazole-4-carboxylic acid**
   LC-MS: t_{R} = 0.90 min; [M+H]⁺ = 260.23.
**2-Cyclopropyl-5-(3-methoxy-phenyl)-thiazole-4-carboxylic acid**
   LC-MS: t_{R} = 0.87 min; [M+H]⁺ = 276.27.
**2-Cyclopropyl-5-phenyl-thiazole-4-carboxylic acid**
   LC-MS: t_{R} = 0.90 min; [M+H]⁺ = 260.22.

### A.1.9. Synthesis of compounds of structure (2)

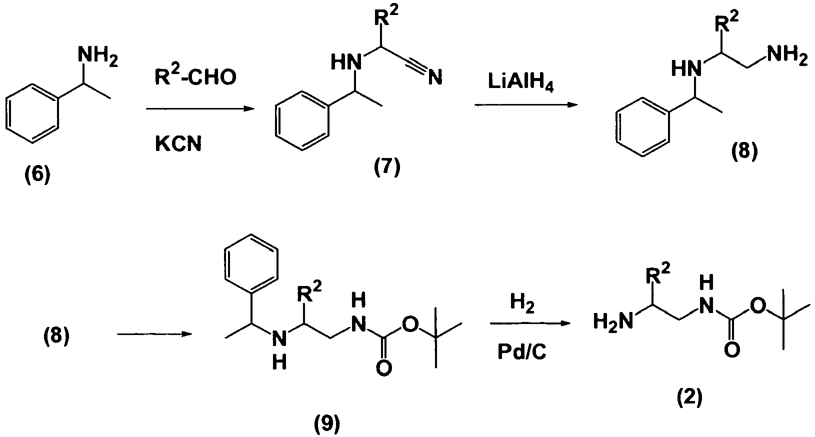

### General Procedure: Synthesis of ((RS)-2-amino-2-cyclopropyl-ethyl)-carbamic acid tert-butyl ester

### Step 1: Cyclopropyl-(1-phenyl-ethylamino)-acetonitrile

Cyclopropanecarboxaldehyde (43 mmol, 1.05 eq.) was dissolved in a mixture of MeOH (59.8 ml), water (53.5 ml) and 25% HCl (41.9 mmol, 1.01 eq.). (+/-)-1-Phenyl-ethylamine (41.5 mmol, 1.0 eq.) and KCN (41.5 mmol, 1.0 eq.) are added and the mixture was allowed to stir for 65 h. The solvent was stripped off and the crude product suspended in DCM. The suspension was treated with aq. sat. NaHCO₃. The organic phase was dried over Na₂SO₄ and evaporated to dryness. The crude product (7.89 g, 95%) was used for the next step without any further purification.
LC-MS: t_{R} = 0.65/0.69 min; [M+H]⁺ = 201.09.

### Step 2: 1-Cyclopropyl-N¹-(1-phenyl-ethyl)-ethane-1,2-diamine

LiAlH₄ (79.6 mmol, 2.0 eq.) was suspended under argon in dry ether (66 ml) and cooled down to 0°C. Then a solution of cyclopropyl-(1-phenyl-ethylamino)-acetonitrile (39.9 mmol, 1.0 eq.) in dry ether (10 ml) was dropped within 10 min to the suspension under virgous stirring. Stirring continued for 2 h at 0°C and further 2 h at ambient temperature. The reaction solution was cooled to 0°C and water (3.0 ml) followed by 15% NaOH (3.0 ml) were added slowly to the reaction solution. Then 60 ml of THF were added and stirring continued overnight. The mixture was diluted with THF, dried over Na₂SO₄ and evaporated to dryness. The crude product (7.85 g, 96%) was used for the next step without any further purification.
LC-MS: t_{R} = na; [M+H]⁺ = 205.10.

### Step 3: [2-Cyclopropyl-2-(1-phenyl-ethylamino)-ethyl]-carbamic acid tert-butyl ester

1-Cyclopropyl-*N¹*-(1-phenyl-ethyl)-ethane-1,2-diamine (38.4 mmol, 1.0 eq.) was dissolved in EE (128 ml) and cooled down to 0°C. To this solution was added NEt₃ (57.6 mmol, 1.5 eq.) followed by a 2.5 M solution of Boc₂O (38.4 mmol, 1.0 eq.) in EE. Stirring continued for 66 h at ambient temperature. The organic phase was washed with aq. sat. NaHCO₃ and dried over Na₂SO₄. After evaporated to dryness the crude product (10.86 g, 93%) was used for the next step without any further purification.
LC-MS: t_{R} = 0.78 min; [M+H]⁺ = 305.14.

### Step 4: ((RS)-2-Amino-2-cyclopropyl-ethyl)-carbamic acid tert-butyl ester

[2-Cyclopropyl-2-(1-phenyl-ethylamino)-ethyl]-carbamic acid tert-butyl ester (35.7 mmol, 1.0 eq.) was dissolved in a mixture of EE/EtOH (142 ml, 1/1, v/v). 10% Pd/C (0.1 eq.) was added and the suspension was treated for 90 h with H₂ for 90 h at 5 bar. The solution filtered over Celite and evaporated to dryness. The crude product was purified by flash chromatography yielding the desired product (5.90 g, 83%) as yellowish oil.
LC-MS: t_{R} = 0.58 min; [M+H]⁺ = 201.11.

The following (2-amino-ethyl)-carbamic acid tert-butyl ester derivatives of structure (2) have been synthesized from **(+/-)-1**-phenyl-ethylamine and the appropriate commercially available aldehyde according to the method given above:
**((*RS*)-2-Amino-4-phenyl-butyl)-carbamic acid tert-butyl ester**
   LC-MS: t_{R} = 0.74 min; [M+H]⁺ = 265.08.
**((*RS*)-2-Amino-propyl)-carbamic acid tert-butyl ester**
   LC-MS: t_{R} = 0.48 min; [M+H]⁺ = 175.08.
**((*RS*)-2-Amino-3-phenyl-propyl)-carbamic acid tert-butyl ester**
   LC-MS: t_{R} = 0.70 min; [M+H]⁺ = 251.08.
**((*RS*)-2-Amino-3-methyl-butyl)-carbamic acid tert-butyl ester**
   LC-MS: t_{R} = 0.60 min; [M+H]⁺ = 203.11.
**((*RS*)-2-Amino-4-methyl-pentyl)-carbamic acid tert-butyl ester**
   LC-MS: t_{R} = 0.67 min; [M+H]⁺ = 217.13.

### B. Preparation of examples

### Reference Example 1: 2-Methyl-5-phenyl-thiazole-4-carboxylic acid {(RS)-2-[(benzofuran-4-carbonyl)-amino]-1-cyclopropyl-ethyl}-methyl-amide

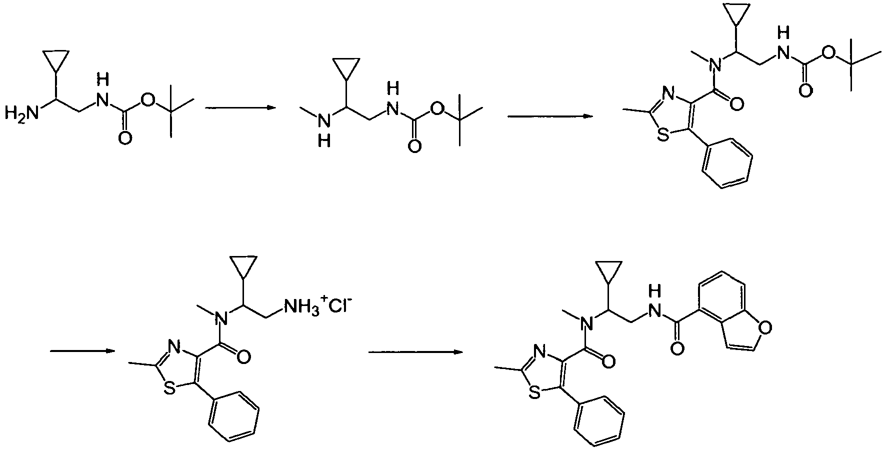

### Step 1: ((RS)-2-Cyclopropyl-2-methylamino-ethyl)-carbamic acid tert-butyl ester

((*RS*)-2-Amino-2-cyclopropyl-ethyl)-carbamic acid tert-butyl ester (0.75 mmol, 1.0 eq.) was dissolved in MeOH (7.5 ml) and a 36.5 % solution of formaldehyde in water (0.825 mmol, 1.1 eq.) was added. Stirring continued for 3 h and then NaBH₄ (0.99 mmol, 1.4 eq.) was added. Stirring continued overnight and the solvent was stripped off. The crude was dissolved in DCM and the organic phase was washed with aq. sat. NaHCO₃ and dried over Na₂SO₄. The crude product was used for the next reaction without any further purification.
LC-MS: t_{R} = 0.59 min; [M+H]⁺ = 215.14.

### Step 2: {(RS)-2-Cyclopropyl-2-[methyl-(2-methyl-5-phenyl-thiazole-4-carbonyl)-amino]-ethyl}-carbamic acid tert-butyl ester

2-Methyl-5-phenyl-thiazole-4-carboxylic acid (0.75 mmol, 1.0 eq.), DIPEA (1.5 mmol, 2.2 eq.) and HATU (0.68 mmol, 0.9 eq.) were dissolved in DMF (3.75 ml) and a solution of ((*RS*)-2-cyclopropyl-2-methylamino-ethyl)-carbamic acid tert-butyl ester (0.75 mmol, 1.0 eq.) in DMF (0.4 ml) was added under stirring. The reaction was allowed to stir overnight and the solvent was evaporated. The crude was dissolved in DCM and the organic phase was washed with aq. sat. NaHCO₃ and dried over Na₂SO₄. The crude product was purified by preparative HPLC yielding the desired product (0.142 g, 50%).
LC-MS: t_{R} = 1.00 min; [M+H]⁺ = 416.18.

### Step 3: (RS)-2-Cyclopropyl-2-[methyl-(2-methyl-5-phenyl-thiazole-4-carbonyl)-amino]-ethyl-ammonium chloride

{(*RS*)-2-Cyclopropyl-2-[methyl-(2-methyl-5-phenyl-thiazole-4-carbonyl)-amino]-ethyl}-carbamic acid tert-butyl ester (0.34 mmol, 1.0 eq.) was dissolved in dry MeOH (0.5 ml) and 4M HCl in dioxane (2.0 ml, 30 eq.) was added and the reaction mixture was allowed to stir for 1.5 h. The solvent was evaporated to dryness and the crude product dissolved in MeOH and the mixture was once again evaporated to dryness yielding the product which was used without further purification for the next step.

### Step 4: 2-Methyl-5-phenyl-thiazole-4-carboxylic acid {(RS)-2-[(benzofuran-4-carbonyl)-amino]-1-cyclopropyl-ethyl}-methyl-amide

(*RS*)-2-Cyclopropyl-2-[methyl-(2-methyl-5-phenyl-thiazole-4-carbonyl)-amino]-ethylammonium chloride (0.17 mmol, 1.0 eq.) was suspended in DMF (0.2 ml) and a solution of benzofuran-4-carboxylic acid (0.135 mmol, 0.79 eq.) and DIPEA (0.57 mmol, 3.4 eq.) in DMF (0.54 ml) was added. Finally a solution of HATU (0.135 ml, 0.79 eq.) was added and the mixture was shaken overnight. The solvent was stripped off and the residue was purified by preparative HPLC yielding the desired product (0.0134 g, 22%)
LC-MS: t_{R} = 0.99; [M+H]⁺ = 460.18

### Examples and reference examples 2 to 277:

The following examples and reference examples were prepared according to the method given above using the appropriate (2-amino-ethyl)-carbamic acid tert-butyl ester derivative, which was reacted with the appropriate aldehyde in step 1 (in case R¹ # H), the appropriate acid A-COOH in step 2, and the appropriate acid R³-COOH in step 4 to provide the desired final compounds.
Reference Example 2: 5-(4-Fluoro-phenyl)-2-methyl-thiazole-4-carboxylic acid {2-[(benzofuran-4-carbonyl)-amino]-ethyl}-benzyl-amide.
   LC-MS: t_{R} = 1.04 min; [M+H]⁺ = 514.06.
Reference Example 3: 5-(4-Fluoro-phenylh2-methyl-thiazole-4-carboxylic acid {2-[(benzofuran-4-carbonyl)amino]-ethyl}methyl-amide.
   LC-MS: t_{R} = 0.92 min; [M+H]⁺ = 438.15.
Reference Example 4: 1-Methyl-1*H*-indazole-3-carboxylic acid (2-{[5-(4-fluorophenyl)-2-methyl-thiazole-4-carbonyl]-methyl-amino}-ethyl)-amide.
   LC-MS: t_{R} = 0.91 min; [M+H]⁺ = 452.18.
Reference Example 5: 5-(4-Fluoro-phenyl)-2-methyl-thiazole-4-carboxylic acid {2-[(2,3-dihydro-benzofuran-7-carbonyl)-amino]-ethyl}-methyl-amide.
   LC-MS: t_{R} = 0.91 min; [M+H]⁺ = 440.15.
Reference Example 6: 6-Methyl-imidazo[2,1-b]thiazole-5-carboxylic acid (2-{[5-(4-fluoro-phenyl)-2-methyl-thiazole-4-carbonyl]-methyl-amino}ethyl)-amide.
   LC-MS: t_{R} = 0.75 min; [M+H]⁺ = 458.08.
Reference Example 7: 5-(4-Fluoro-phenyl)-2-methyl-thiazole-4-carboxylic acid benzyl-{2-[(2,3-dihydro-benzo[1,4]dioxine-5-carbonyl)-amino]-ethyl}amide.
   LC-MS: t_{R} = 1.02 min; [M+H]⁺ = 532.16.
Reference Example 8: 1-Methyl-1*H*-indazote-3-carboxylic acid (2-{benzyl-[5-(4-fluoro-phenyl)-2-methyl-thiazole-4-carbonyl]-amino}-ethyl)-amide.
   LC-MS: t_{R} = 1.04 min; [M+H]⁺ = 528.18.
Reference Example 9: 5-(4-Fluoro-phenyl)-2-methyl-thiazole-4-carboxylic acid benzyl-{2-[(2,3-dihydro-benzofuran-7-carbonyl)-amino]-ethyl}amide.
   LC-MS: t_{R} = 1.03 min; [M+H]⁺ = 516.18.
Reference Example 10: 6-Methyl-imidazo[2,1-b]thiazole-5-carboxylic acid (2-{benzyl-[5-(4-fluoro-phenyl)-2-methyl-thiazole-4-carbonyl]-amino}-ethyl)-amide.
   LC-MS: t_{R} = 0.91 min; [M+H]⁺ = 534.14.
Reference Example 11: 2-Methyl-5-phenyl-thiazole-4-carboxylic acid {2-[(benzofuran-4-carbonyl)-amino]-ethyl}-(3,3-dimethyl-butyl)-amide.
   LC-MS: t_{R} = 1.06 min; [M+H]⁺ = 489.21.
Reference Example 12: 6-Methyl-imidazo[2,1-b]thiazole-5-carboxylic acid {2-[(3,3-dimethyl-butyl)-(2-methyl-5-phenyl-thiazole-4-carbonyl)-amino]-ethyl}-amide.
   LC-MS: t_{R} = 0.94 min; [M+H]⁺ = 509.19.
Reference Example 13: 2-Methyl-5-phenyl-thiazole-4-carboxylic acid {2-[(2,3-dihydro-benzo[1,4]dioxine-5-carbonyl)amino]-ethyl}-(3,3-dimethyl-butyl)-amide.
   LC-MS: t_{R} = 1.05 min; [M+H]⁺ = 507.22.
Reference Example 14: 2-Methyl-5-phenyl-thiazole-4-carboxylic acid {2-[(benzofuran-4-carbonyl)-amino]-ethyl}-(3-phenyl-propyl)-amide.
   LC-MS: t_{R} = 1.05 min; [M+H]⁺ = 523.19.
Reference Example 15: 6-Methyl-imidazo[2,1-b]thiazole-5-carboxylic acid {2-[(2-methyl-5-phenyl-thiazole-4-carbonyl)-(3-phenyl-propyl)-amino]-ethyl}-amide.
   LC-MS: t_{R} = 0.92 min; [M+H]⁺ = 543.18.
Reference Example 16: 2-Methyl-5-phenyl-thiazole-4-carboxylic acid {2-[(2,3-dihydro-benzo[1,4]dioxine-5-carbonyl)-amino]-ethyl}-(3-phenyl-propyl)-amide.
   LC-MS: t_{R} = 1.04 min; [M+H]⁺ = 541.2.
Reference Example 17: 2-Methyl-5-phenyl-thiazole-4-carboxylic acid {2-[(benzofuran-4-carbonyl)-amino]-ethyl}-(4-fluoro-benzyl)-amide.
   LC-MS: t_{R} = 1.04 min; [M+H]⁺ = 513.15.
Reference Example 18: 6-Methyl-imidazo[2,1-b]thiazole-5-carboxylic acid {2-[(4-fluoro-benzyl)-(2-methyl-5-phenyl-thiazole-4-carbonyl)-amino]-ethyl}amide.
   LC-MS: t_{R} = 0.91 min; [M+H]⁺ = 533.14.
Reference Example 19: 2-Methyl-5-phenyl-thiazole-4-carboxylic acid {2-[(2,3-dihydro-benzo[1,4]dioxine-5-carbonyl)-amino]-ethyl}-(4-fluoro-benzyl)-amide.
   LC-MS: t_{R} = 1.02 min; [M+H]⁺ = 531.16.
Reference Example 20: 2-Methyl-5-phenyl-thiazole-4-carboxylic acid {2-[(benzofuran-4-carbonyl)-amino]-ethyl}-phenethyl-amide.
   LC-MS: t_{R} = 1.04 min; [M+H]⁺ = 509.18.
Reference Example 21: 6-Methyl-imidazo[2,1-b]thiazole-5-carboxylic acid {2-[(2-methyl-5-phenyl-thiazole-4-carbonyl)-phenethyl-amino]-ethyl}-amide.
   LC-MS: t_{R} = 0.91 min; [M+H]⁺ = 529.16.
Reference Example 22: 2-Methyl-5-phenyl-thiazole-4-carboxylic acid {2-[(2,3-dihydro-benzo[1,4]dioxine-5-carbonyl)-amino]-ethyl}-phenethyl-amide.
   LC-MS: t_{R} = 1.04 min; [M+H]⁺ = 527.19.
Reference Example 23: 2-Methyl-5-phenyl-thiazole-4-carboxylic acid {2-[(benzofuran-4-carbonyl)-amino]-ethyl}-(4-trifluoromethyl-benzyl)-amide.
   LC-MS: t_{R} = 1.08 min; [M+H]⁺ = 563.15.
Reference Example 24: 6-Methyl-imidazo[2,1-b]thiazole-5-carboxylic acid {2-[(2-methyl-5-phenyl-thiazole-4-carbonyl)-(4-trifluoromethyl-benzyl)-amino]-ethyl}-amide.
   LC-MS: t_{R} = 0.97 min; [M+H]⁺ = 583.13.
Reference Example 25: 2-Methyl-5-phenyl-thiazole-4-carboxylic acid {2-[(2,3-dihydro-benzo[1,4]dioxine-5-carbonyl)-amino]-ethyl}-(4-trifluoromethyl-benzyl)-amide.
   LC-MS: t_{R} = 1.06 min; [M+H]⁺ = 581.16.
Reference Example 26: 2-Methyl-5-phenyl-thiazole-4-carboxylic acid {2-[(benzofuran-4-carbonyl)-amino]-ethyl}-cyclohexylmethyl-amide.
   LC-MS: t_{R} = 1.07 min; [M+H]⁺ = 501.21.
Reference Example 27: 6-Methyl-imidazo[2,1-b]thiazole-5-carboxylic acid {2-[cydohexylmethyl-(2-methyl-5-phenyl-thiazole-4-carbonyl)-amino]-ethyl}amide.
   LC-MS: t_{R} = 0.94 min; [M+H]⁺ = 521.19.
Reference Example 28: 2-Methyl-5-phenyl-thiazole-4-carboxylic acid cyclohexylmethyl-{2-[(2,3-dihydro-benzo[1,4]dioxine-5-carbonyl)-amino]-ethyl}-amide.
   LC-MS: t_{R} = 1.06 min; [M+H]⁺ = 519.22.
Reference Example 29: 2-Methyl-5-phenyl-thiazole-4-carboxylic acid {2-[(benzofuran-4-carbonyl)-amino]-ethyl}-isobutyl-amide.
   LC-MS: t_{R} = 1.01 min; [M+H]⁺ = 461.18.
Reference Example 30: 6-Methyl-imidazo[2,1-b]thiazole-5-carboxylic acid {2-[isobutyl-(2-methyl-5-phenyl-thiazole-4-carbonyl)-amino]-ethyl}-amide.
   LC-MS: t_{R} = 0.86 min; [M+H]⁺ = 481.16.
Reference Example 31: 2-Methyl-5-phenyl-thiazole-4-carboxylic acid {2-[(2,3-dihydro-benzo[1,4]dioxine-5-carbonyl)-amino]-ethyl}-isobutyl-amide.
   LC-MS: t_{R} = 0.98 min; [M+H]⁺ = 479.19.
**Example 32: 2-Methyl-5-phenyl-thiazole-4-carboxylic acid {2-[(benzofuran-4-carbonyl)-amino]-ethyl}-cyclopropylmethyl-amide.**
   LC-MS: t_{R} = 0.99 min; [M+H]⁺ = 459.16.
**Example 33: 6-Methyl-imidazo[2,1-b]thiazole-5-carboxylic acid {2-[cyclopropylmethyl-(2-methyl-5-phenyl-thiazole-4-carbonyl)-amino]-ethyl}-amide.**
   LC-MS: t_{R} = 0.84 min; [M+H]⁺ = 479.14.
**Example 34: 2-Methyl-5-phenyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 0.97 min; [M+H]⁺ = 477.17.
Reference Example 35: 2-Methyl-5-phenyl-thiazole-4-carboxylic acid {2-[(benzofuran-4-carbonyl)-amino]-ethyl}-benzyl-amide.
   LC-MS: t_{R} = 1.03 min; [M+H]⁺ = 495.16.
Reference Example 36: 6-Methyl-imidazo[2,1-b]thiazole-5-carboxylic acid {2-[benzyl-(2-methyl-5-phenyl-thiazole-4-carbonyl)-amino]-ethyl}-amide.
   LC-MS: t_{R} = 0.9 min; [M+H]⁺ = 515.14.
Reference Example 37: 2-Methyl-5-phenyl-thiazole-4-carboxylic acid benzyl-{2-[(2,3-dihydro-benzo[1,4]dioxine-5-carbonyl)-amino]-ethyl}-amide.
   LC-MS: t_{R} = 1.01 min; [M+H]⁺ = 513.17.
Reference Example 38: 2-Methyl-5-phenyl-thiazole-4-carboxylic acid {2-[(benzofuran-4-carbonyl)-amino]-ethyl}-methyl-amide.
   LC-MS: t_{R} = 0.91 min; [M+H]⁺ = 419.13.
Reference Example 39: 6-Methyl-imidazo[2,1-b]thiazole-5-carboxylic acid {2-[methyl-(2-methyl-5-phenyl-thiazole-4-carbonyl)-amino]-ethyl}-amide.
   LC-MS: t_{R} = 0.75 min; [M+H]⁺ = 439.6.
Reference Example 40: 2-Methyl-5-phenyl-thiazole-4-carboxylic acid {2-[(2,3-dihydro-benzo[1,4]dioxine-5-carbonyl)-amino]-ethyl}-methyl-amide.
   LC-MS: t_{R} = 0.89 min; [M+H]⁺ = 437.14.
**Example 41: 2-Methyl-5-phenyl-thiazole-4-carboxylic acid {2-[(benzofuran-4-carbonyl)-amino]-ethyl}-cyclopropyl-amide.**
   LC-MS: t_{R} = 0.96 min; [M+H]⁺ = 445.15.
**Example 42: 6-Methyl-imidazo[2,1-b]thiazole-5-carboxylic acid {2-[cyclopropyl-(2-methyl-5-phenyl-thiazole-4-carbonyl)-amino]-ethyl}-amide.**
   LC-MS: t_{R} = 0.8 min; [M+H]⁺ = 465.13.
**Example 43: 2-Methyl-5-phenyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.93 min; [M+H]⁺ = 463.16.
Reference Example 44: 2-Methyl-5-phenyl-thiazole-4-carboxylic acid {(RS)-2-[(benzofuran-4-carbonyl)-amino]-1-cyclopropyl-ethyl}-benzyl-amide.
   LC-MS: t_{R} = 1.08 min; [M+H]⁺ = 536.19.
Reference Example 45: 2-Methyl-5-phenyl-thiazole-4-carboxylic acid {(RS)-2-[(benzofuran-4-carbonyl)-amino]-1-cyclopropyl-ethyl}-amide.
   LC-MS: t_{R} = 0.99 min; [M+H]⁺ = 446.15.
Reference Example 46: Benzofuran-4-carboxylic acid {(RS)-2-[(biphenyl-2-carbonyl)-amino]-2-cyclopropyl-ethyl}amide.
   LC-MS: t_{R} = 0.98 min; [M+H]⁺ = 425.16.
Reference Example 47: 6-Methyl-imidazo[2,1-b]thiazole-5-carboxylic acid {(RS)-2-cyclopropyl-2-[methyl-(2-methyl-5-phenyl-thiazole-4-carbonyl)-amino]-ethyl}-amide.
   LC-MS: t_{R} = 0.82 min; [M+H]⁺ = 480.18.
Reference Example 48: 6-Methyl-imidazo[2,1-b]thiazole-5-carboxylic acid {(RS)-2-[benzyl-(2-methyl-5-phenyl-thiazole-4-carbonyl)-amino]-2-cydopropyl-ethyl}-amide.
   LC-MS: t_{R} = 0.95 min; [M+H]⁺ = 556.17.
Reference Example 49: 6-Methyl-imidazo[2,1-b]thiazole-5-carboxylic acid {(RS)-2-cyclopropyl-2-[(2-methyl-5-phenyl-thiazole-4-carbonyl)-amino]-ethyl}-amide.
   LC-MS: t_{R} = 0.85 min; [M+H]⁺ = 466.14.
Reference Example 50: 6-Methyl-imidazo[2,1-b]thiazole-5-carboxylic acid {(RS)-2-[(biphenyl-2-carbonyl)-amino]-2-cyclopropyl-ethyl}-amide.
   LC-MS: t_{R} = 0.84 min; [M+H]⁺ = 445.17.
Reference Example 51: 2-Methyl-5-phenyl-thiazole-4-carboxylic acid ((RS)-1-{[(benzofuran-4-carbonyl)-amino]-methyl}-3-phenyl-propyl)-methyl-amide.
   LC-MS: t_{R} = 1.07 min; [M+H]⁺ = 524.19.
Reference Example 52: 2-Methyl-5-phenyl-thiazole-4-carboxylic acid ((RS)-1-{[(benzofuran-4-carbonyl)-amino]-methyl}-3-phenyl-propyl)-benzyl-amide.
   LC-MS: t_{R} = 1.14 min; [M+H]⁺ = 600.23.
Reference Example 53: 2-Methyl-5-phenyl-thiazole-4-carboxylic acid ((RS)-1-{[(benzofuran-4-carbonyl)-amino]-methyl}-3-phenyl-propyl)-amide.
   LC-MS: t_{R} = 1.07 min; [M+H]⁺ = 510.17.
Reference Example 54: Benzofuran-4-carboxylic acid {(RS)-2-[(biphenyl-2-carbonyl)-amino]-4-phenyl-butyl}-amide.
   LC-MS: t_{R} = 1.05 min; [M+H]⁺ = 489.23.
Reference Example 55: 6-Methyl-imidazo[2,1-b]thiazole-5-carboxylic acid {(RS)-2-[methyl-(2-methyl-5-phenyl-thiazole-4-carbonyl)-amino]-4-phenyl-butyl}-amide.
   LC-MS: t_{R} = 0.93 min; [M+H]⁺ = 544.18.
Reference Example 56: 6-Methyl-imidazo[2,1-b]thiazole-5-carboxylic acid {(RS)-2-[benzyl-(2-methyl-5-phenyl-thiazole-4-carbonyl)-amino]-4-phenyl-butyl}amide.
   LC-MS: t_{R} = 1.02 min; [M+H]⁺ = 620.22.
Reference Example 57: 6-Methyl-imidazo[2,1-b]thiazole-5-carboxylic acid {(RS)-2-[(2-methyl-5-phenyl-thiazole-4-carbonyl)-amino]-4-phenyl-butyl}amide.
   LC-MS: t_{R} = 0.95 min; [M+H]⁺ = 530.18.
Reference Example 58: 6-Methyl-imidazo[2,1-b]thiazole-5-carboxylic acid {(RS)-2-[(biphenyl-2-carbonyl)-amino]-4-phenyl-butyl}-amide.
   LC-MS: t_{R} = 0.94 min; [M+H]⁺ = 509.2.
Reference Example 59: 2-Methyl-5-phenyl-thiazole-4-carboxylic acid {(RS)-2-[(benzofuran-4-carbonyl)-amino]-1-methyl-ethyl}-methyl-amide.
   LC-MS: t_{R} = 0.93 min; [M+H]⁺ = 434.14.
Reference Example 60: 2-Methyl-5-phenyl-thiazole-4-carboxylic acid {(RS)-2-[(benzofuran-4-carbonyl)-amino]-1-methyl-ethyl}-benzyl-amide.
   LC-MS: t_{R} = 1.05 min; [M+H]⁺ = 510.13.
Reference Example 61: 2-Methyl-5-phenyl-thiazole-4-carboxylic acid {(RS)-2-[(benzofuran-4-carbonyl)-amino]-1-methyl-ethyl}-amide.
   LC-MS: t_{R} = 0.95 min; [M+H]⁺ = 420.1.
Reference Example 62: Benzofuran-4-carboxylic acid {(RS)-2-[(biphenyl-2-carbonyl)-amino]-propyl}amide.
   LC-MS: t_{R} = 0.94 min; [M+H]⁺ = 399.12.
Reference Example 63: 6-Methyl-imidazo[2,1-b]thiazole-5-carboxylic acid {(RS)-2-[benzyl-(2-methyl-5-phenyl-thiazole-4-carbonyl)-amino]-propyl}amide.
   LC-MS: t_{R} = 0.9 min; [M+H]⁺ = 530.16.
Reference Example 64: 6-Methyl-imidazo[2,1-b]thiazole-5-carboxylic acid {(RS)-2-[(2-methyl-5-phenyl-thiazole-4-carbonyl)-amino]-propyl}-amide.
   LC-MS: t_{R} = 0.8 min; [M+H]⁺ = 440.12.
Reference Example 65: 6-Methyl-imidazo[2,1-b]thiazole-5-carboxylic acid {(RS)-2-[(biphenyl-2-carbonyl)-amino]-propylfamide.
   LC-MS: t_{R} = 0.79 min; [M+H]⁺ = 419.13.
Reference Example 66: 2-Methyl-5-phenyl-thiazole-4-carboxylic acid {(RS)-2-[(benzofuran-4-carbonyl)-amino]-1-benzyl-ethyl}methyl-amide.
   LC-MS: t_{R} = 1.04 min; [M+H]⁺ = 510.15.
Reference Example 67: 2-Methyl-5-phenyl-thiazole-4-carboxylic acid {(RS)-2-[(benzofuran-4-carbonyl)-amino]-1-benzyl-ethyl}-benzyl-amide.
   LC-MS: t_{R} = 1.12 min; [M+H]⁺ = 586.2.
Reference Example 68: 2-Methyl-5-phenyl-thiazole-4-carboxylic acid {(RS)-2-[(benzofuran-4-carbonyl)-amino]-1-benzyl-ethyl}-amide.
   LC-MS: t_{R} = 1.04 min; [M+H]⁺ = 496.19.
Reference Example 69: Benzofuran-4-carboxylic acid {(RS)-2-[(biphenyl-2-carbonyl)-amino]-3-phenyl-propyl}-amide.
   LC-MS: t_{R} = 1.03 min; [M+H]⁺ = 475.23.
Reference Example 70: 6-Methyl-imidazo[2,1-b]thiazole-5-carboxylic acid {(RS)-2-[methyl-(2-methyl-5-phenyl-thiazole-4-carbonyl)-amino]-3-phenyl-propyl}-amide.
   LC-MS: t_{R} = 0.88 min; [M+H]⁺ = 530.15.
Reference Example 71: 6-Methyl-imidazo[2,1-b]thiazole-5-carboxylic acid {(RS)-2-[benzyl-(2-methyl-5-phenyl-thiazole-4-carbonyl)-amino]-3-phenyl-propyl}-amide.
   LC-MS: t_{R} = 1.01 min; [M+H]⁺ = 606.11.
Reference Example 72: 6-Methyl-imidazo[2,1-b]thiazole-5-carboxylic acid {(RS)-2-[(2-methyl-5-phenyl-thiazole-4-carbonyl)-amino]-3-phenyl-propyl}-amide.
   LC-MS: t_{R} = 0.92 min; [M+H]⁺ = 516.16.
Reference Example 73: 6-Methyl-imidazo[2,1-b]thiazole-5-carboxylic acid {(RS)-2-[(biphenyl-2-carbonyl)-amino]-3-phenyl-propyl}-amide.
   LC-MS: t_{R} = 0.91 min; [M+H]⁺ = 495.18.
Reference Example 74: 2-Methyl-5-phenyl-thiazole-4-carboxylic acid ((RS)-1-{[(benzofuran-4-carbonyl)-amino]-methyl}-2-methyl-propyl)-methyl-amide.
   LC-MS: t_{R} = 1 min; [M+H]⁺ = 462.2.
Reference Example 75: 2-Methyl-5-phenyl-thiazole-4-carboxylic acid ((RS)-1-{[(benzofuran-4-carbonyl)-amino]-methyl}-2-methyl-propyl)-benzyl-amide.
   LC-MS: t_{R} = 1.12 min; [M+H]⁺ = 538.23.
Reference Example 76: 2-Methyl-5-phenyl-thiazole-4-carboxylic acid ((RS)-1-{[(benzofuran-4-carbonyl)-amino]-methyl}-2-methyl-propyl)-amide.
   LC-MS: t_{R} = 1.01 min; [M+H]⁺ = 448.18.
Reference Example 77: Benzofuran-4-carboxylic acid {(RS)-2-[(biphenyl-2-carbonyl)-amino]-3-methyl-butyl}-amide.
   LC-MS: t_{R} = 1 min; [M+H]⁺ = 427.18.
Reference Example 78: 6-Methyl-imidazo[2,1-b]thiazole-5-carboxylic acid {(RS)-2-[benzyl-(2-methyl-5-phenyl-thiazole-4-carbonyl)-amino]-3-methyl-butyl}-amide.
   LC-MS: t_{R} = 0.99 min; [M+H]⁺ = 558.1.
Reference Example 79: 6-Methyl-imidazo[2,1-b]thiazole-5-carboxylic acid {(RS)-3-methyl-2-[(2-methyl-5-phenyl-thiazole-4-carbonyl)-amino]-butyl}-amide.
   LC-MS: t_{R} = 0.87 min; [M+H]⁺ = 468.17.
Reference Example 80: 6-Methyl-imidazo[2,1-b]thiazole-5-carboxylic acid {(RS)-2-[(biphenyl-2-carbonyl)-amino]-3-methyl-butyl}-amide.
   LC-MS: t_{R} = 0.86 min; [M+H]⁺ = 447.2.
Reference Example 81: 2-Methyl-5-phenyl-thiazole-4-carboxylic acid ((RS)-1-{[(benzofuran-4-carbonyl)-amino]-methyl}-3-methyl-butyl)-methyl-amide.
   LC-MS: t_{R} = 1.05 min; [M+H]⁺ = 476.22.
Reference Example 82: 2-Methyl-5-phenyl-thiazole-4-carboxylic acid ((RS)-1-{[(benzofuran-4-carbonyl)-amino]-methyl}-3-methyl-butyl)-benzyl-amide.
   LC-MS: t_{R} = 1.13 min; [M+H]⁺ = 552.22.
Reference Example 83: 2-Methyl-5-phenyl-thiazole-4-carboxylic acid ((RS)-1-{[(benzofuran-4-carbonyl)-amino]-methyl}-3-methyl-butyl)-amide.
   LC-MS: t_{R} = 1.04 min; [M+H]⁺ = 462.2.
Reference Example 84: Benzofuran-4-carboxylic acid {(RS)-2-[(biphenyl-2-carbonyl)-amino]-4-methyl-pentyl}-amide.
   LC-MS: t_{R} = 1.03 min; [M+H]⁺ = 441.21.
Reference Example 85: 6-Methyl-imidazo[2,1-b]thiazole-5-carboxylic acid {(RS)-4-methyl-2-[methyl-(2-methyl-5-phenyl-thiazole-4-carbonyl)-amino]-pentyl}-amide.
   LC-MS: t_{R} = 0.87 min; [M+H]⁺ = 496.19.
Reference Example 86: 6-Methyl-imidazo[2,1-b]thiazole-5-carboxylic acid {(RS)-2-[benzyl-(2-methyl-5-phenyl-thiazole-4-carbonyl)-amino]-4-methyl-pentyl}-amide.
   LC-MS: t_{R} = 1 min; [M+H]⁺ = 572.22.
Reference Example 87: 6-Methyl-imidazo[2,1-b]thiazole-5-carboxylic acid {(RS)-4-methyl-2-[(2-methyl-5-phenyl-thiazole-4-carbonyl)-amino]-pentyl}-amide.
   LC-MS: t_{R} = 0.91 min; [M+H]⁺ = 482.19.
Reference Example 88: 6-Methyl-imidazo[2,1-b]thiazole-5-carboxylic acid {(RS)-2-[(biphenyl-2-carbonyl)-amino]-4-methyl-pentyl}-amide.
   LC-MS: t_{R} = 0.9 min; [M+H]⁺ = 461.21.
Reference Example 89: 6-Methyl-imidazo[2,1-b]thiazole-5-carboxylic acid {(RS)-2-[(biphenyl-2-carbonyl)-methyl-amino]-2-cyclopropyl-ethyl}-amide.
   LC-MS: t_{R} = 0.88 min; [M+H]⁺ = 459.2.
Reference Example 90: 6-Methyl-imidazo[2,1-b]thiazole-5-carboxylic acid {(RS)-2-[(biphenyl-2-carbonyl)-methyl-amino]-4-phenyl-butyl}-amide.
   LC-MS: t_{R} = 0.97 min; [M+H]⁺ = 523.2.
Reference Example 91: 6-Methyl-imidazo[2,1-b]thiazole-5-carboxylic acid {(RS)-2-[(biphenyl-2-carbonyl)-methyl-amino]-propyl}-amide.
   LC-MS: t_{R} = 0.83 min; [M+H]⁺ = 433.14.
Reference Example 92: 6-Methyl-imidazo[2,1-b]thiazole-5-carboxylic acid {(RS)-2-[(biphenyl-2-carbonyl)-methyl-amino]-3-phenyl-propyl}-amide.
   LC-MS: t_{R} = 0.92 min; [M+H]⁺ = 509.2.
Reference Example 93: 6-Methyl-imidazo[2,1-b]thiazole-5-carboxylic acid {(RS)-2-[(biphenyl-2-carbonyl)-methyl-amino]-3-methyl-butyl}-amide.
   LC-MS: t_{R} = 0.9 min; [M+H]⁺ = 461.21.
Reference Example 94: 6-Methyl-imidazo[2,1-b]thiazole-5-carboxylic acid {(RS)-2-[(biphenyl-2-carbonyl)-methyl-amino]-4-methyl-pentyl}amide.
   LC-MS: t_{R} = 0.91 min; [M+H]⁺ = 475.22.
Reference Example 95: 6-Methyl-imidazo[2,1-b]thiazole-5-carboxylic acid {(RS)-2-[benzyl-(biphenyl-2-carbonyl)-amino]-2-cyclopropyl-ethyl}-amide.
   LC-MS: t_{R} = 0.99 min; [M+H]⁺ = 535.13.
Reference Example 96: 6-Methyl-imidazo[2,1-b]thiazole-5-carboxylic acid {(RS)-2-[benzyl-(biphenyl-2-carbonyl)-amino]-4-phenyl-butyl}-amide.
   LC-MS: t_{R} = 1.04 min; [M+H]⁺ = 599.17.
Reference Example 97: 6-Methyl-imidazo[2,1-b]thiazole-5-carboxylic acid {(RS)-2-[benzyl-(biphenyl-2-carbonyl)-amino]-propyl}-amide.
   LC-MS: t_{R} = 0.94 min; [M+H]⁺ = 509.2.
Reference Example 98: 6-Methyl-imidazo[2,1-b]thiazole-5-carboxylic acid {(RS)-2-[benzyl-(biphenyl-2-carbonyl)-amino]-3-phenyl-propyl}-amide.
   LC-MS: t_{R} = 1.05 min; [M+H]⁺ = 585.23.
Reference Example 99: 6-Methyl-imidazo[2,1-b]thiazole-5-carboxylic acid {(RS)-2-[benzyl-(biphenyl-2-carbonyl)-amino]-3-methyl-butyl}-amide.
   LC-MS: t_{R} = 1.03 min; [M+H]⁺ = 537.23.
Reference Example 100: 6-Methyl-imidazo[2,1-b]thiazole-5-carboxylic acid {(RS)-2-[benzyl-(biphenyl-2-carbonyl)-amino]-4-methyl-pentyl}amide..
   LC-MS: t_{R} = 1.04 min; [M+H]⁺ = 551.15.
**Example 101: 6-Methyl-imidazo[2,1-b]thiazole-5-carboxylic acid {2-[cyclopropyl-(2-cyclopropyl-5-phenyl-thiazole-4-carbonyl)-amino]-ethyl}-amide.**
   LC-MS: t_{R} = 0.87 min; [M+H]⁺ = 492.18.
**Example 102: 6-Methyl-imidazo[2,1-b]thiazole-5-carboxylic acid {2-[cyclopropyl-(2-cyclopropyl-5-*m*-tolyl-thiazole-4-carbonyl)-amino]-ethyl}-amide.**
   LC-MS: t_{R} = 0.9 min; [M+H]⁺ = 506.19.
**Example 103: 6-Methyl-imidazo[2,1-b]thiazole-5-carboxylic acid (2-{cyclopropyl-[2-cyclopropyl-5-(3-methoxy-phenyl)-thiazole-4-carbonyl]-amino}ethyl)-amide.**
   LC-MS: t_{R} = 0.88 min; [M+H]⁺ = 522.17.
**Example 104:6-Methyl-imidazo[2,1-b]thiazole-5-carboxylic acid {2-[(2-amino-5-*m*-tolyl-thiazole-4-carbonyl)-cyclopropyl-amino]-ethyl}-amide.**
   LC-MS: t_{R} = 0.76 min; [M+H]⁺ = 481.18.
**Example 105: 6-Methyl-imidazo[2,1-b]thiazole-5-carboxylic acid (2-{[2-amino-5-(3-fluoro-phenyl)-thiazole-4-carbonyl]-cyclopropyl-amino}-ethyl)-amide.**
   LC-MS: t_{R} = 0.75 min; [M+H]⁺ = 485.16.
**Example 106:6-Methyl-imidazo[2,1-b]thiazole-5-carboxylic acid (2-{[2-amino-5-(3-methoxy-phenyl)-thiazole-4-carbonyl]-cyclopropyl-amino}-ethyl)-amide.**
   LC-MS: t_{R} = 0.74 min; [M+H]⁺ = 497.17.
**Example 107:6-Methyl-imidazo[2,1-b]thiazole-5-carboxylic acid (2-{[2-amino-5-(4-fluoro-phenyl)-thiazole-4-carbonyl]-cyclopropyl-amino}-ethyl)-amide.**
   LC-MS: t_{R} = 0.74 min; [M+H]⁺ = 485.17.
**Example 108: 6-Methyl-imidazo[2,1-b]thiazole-5-carboxylic acid (2-{[2-amino-5-(3-chloro-phenyl)-thiazole-4-carbonyl]-cyclopropyl-amino}-ethyl)-amide.**
   LC-MS: t_{R} = 0.78 min; [M+H]⁺ = 500.53.
**Example 109: 6-Methyl-imidazo[2,1-b]thiazole-5-carboxylic acid {2-[(2-amino-5-phenyl-thiazole-4-carbonyl)-cyclopropyl-amino]-ethyl}-amide.**
   LC-MS: t_{R} = 0.73 min; [M+H]⁺ = 467.17.
**Example 110: 6-Methyl-imidazo[2,1-b]thiazole-5-carboxylic acid (2-{[2-bromo-5-(3-methoxy-phenyl)-thiazole-4-carbonyl]-cyclopropyl-amino}-ethyl)-amide.**
   LC-MS: t_{R} = 0.88 min; [M+H]⁺ = 560.05.
**Example 111: 6-Methyl-imidazo[2,1-b]thiazole-5-carboxylic acid {2-[(2-bromo-5-p-tolyl-thiazole-4-carbonyl)-cyclopropyl-amino]-ethyl}-amide.**
   LC-MS: t_{R} = 0.9 min; [M+H]⁺ = 546.07.
**Example 112:6-Methyl-imidazo[2,1-b]thiazole-5-carboxylic acid (2-{cyclopropyl-[5-(2-fluoro-phenyl)-2-methyl-thiazole-4-carbonyl]-amino}-ethyl)-amide.**
   LC-MS: t_{R} = 0.82 min; [M+H]⁺ = 484.16.
**Example 113:6-Methyl-imidazo[2,1-b]thiazole-5-carboxylic acid (2-{cyclopropyl-[2-methyl-5-(3-trifluoromethyl-phenyl)-thiazole-4-carbonyl]-amino}-ethyl)-amide.**
   LC-MS: t_{R} = 0.89 min; [M+H]⁺ = 534.08.
**Example 114:6-Methyl-imidazo[2,1-b]thiazole-5-carboxylic acid (2-{cyclopropyl-[5-(3-fluoro-phenyl)-2-methyl-thiazole-4-carbonyl]-amino}-ethyl)-amide.**
   LC-MS: t_{R} = 0.82 min; [M+H]⁺ = 484.17.
**Example 115: 6-Methyl-imidazo[2,1-b]thiazole-5-carboxylic acid (2-{cyclopropyl-[2-methyl-5-(4-trifluoromethyl-phenyl)-thiazole-4-carbonyl]-amino}-ethyl)-amide.**
   LC-MS: t_{R} = 0.89 min; [M+H]⁺ = 534.09.
**Example 116:6-Methyl-imidazo[2,1-b]thiazole-5-carboxylic acid (2-{[5-(4-chlorophenyl)-2-methyl-thiazole-4-carbonyl]-cyclopropyl-amino}-ethyl)-amide.**
   LC-MS: t_{R} = 0.85 min; [M+H]⁺ = 499.99.
**Example 117:6-Methyl-imidazo[2,1-b]thiazole-5-carboxylic acid (2-{cyclopropyl-[5-(3-methoxy-phenyl)-2-methyl-thiazole-4-carbonyl]-amino}-ethyl)-amide.**
   LC-MS: t_{R} = 0.82 min; [M+H]⁺ = 496.18.
**Example 118: 6-Methyl-imidazo[2,1-b]thiazole-5-carboxylic acid (2-{[5-(3-cyanophenyl)-2-methyl-thiazole-4-carbonyl]-cyclopropyl-amino}-ethyl)-amide.**
   LC-MS: t_{R} = 0.8 min; [M+H]⁺ = 491.17.
**Example 119: 6-Methyl-imidazo[2,1-b]thiazole-5-carboxylic acid (2-{cyclopropyl-[5-(3,4-dimethyl-phenyl)-2-methyl-thiazole-4-carbonyl]-amino}-ethyl)-amide.**
   LC-MS: t_{R} = 0.87 min; [M+H]⁺ = 494.19.
**Example 120: 6-Methyl-imidazo[2,1-b]thiazole-5-carboxylic acid {2-[cyclopropyl-(2-methyl-5-*p*-tolyl-thiazole-4-carbonyl)-amino]-ethyl}-amide.**
   LC-MS: t_{R} = 0.84 min; [M+H]⁺ = 480.19.
**Example 121: 6-Methyl-imidazo[2,1-b]thiazole-5-carboxylic acid {2-[cyclopropyl-(3'-methyl-biphenyl-2-carbonyl)-amino]-ethyl}-amide.**
   LC-MS: t_{R} = 0.9 min; [M+H]⁺ = 459.23.
**Example 122: 6-Methyl-imidazo[2,1-b]thiazole-5-carboxylic acid {2-[cyclopropyl-(4'-methyl-biphenyl-2-carbonyl)-amino]-ethyl}-amide.**
   LC-MS: t_{R} = 0.91 min; [M+H]⁺ = 459.22.
**Example 123:6-Methyl-imidazo[2,1-b]thiazole-5-carboxylic acid {2-[cyclopropyl-(4'-fluoro-biphenyl-2-carbonyl)-amino]-ethyl}-amide.**
   LC-MS: t_{R} = 0.88 min; [M+H]⁺ = 463.2.
**Example 124:1-Methyl-1*H*-indazole-3-carboxylic acid {2-[cyclopropyl-(2-cyclopropyl-5-phenyl-thiazole-4-carbonyl)-amino]-ethyl}-amide.**
   LC-MS: t_{R} = 1.01 min; [M+H]⁺ = 486.22.
**Example 125:1-Methyl-1*H*-indazole-3-carboxylic acid {2-[cyclopropyl-(2-cyclopropyl-5-*m*-tolyl-thiazole-4-carbonyl)-amino]-ethyl}-amide.**
   LC-MS: t_{R} = 1.04 min; [M+H]⁺ = 500.15.
**Example 126:1-Methyl-1*H*-indazole-3-carboxylic acid (2-{cyclopropyl-[2-cyclopropyl-5-(3-methoxy-phenyl)-thiazole-4-carbonyl]-amino}-ethyl)-amide.**
   LC-MS: t_{R} = 1.02 min; [M+H]⁺ = 516.22.
**Example 127:1-Methyl-1*H*-indazole-3-carboxylic acid {2-[(2-amino-5-m-tolyl-thiazole-4-carbonyl)-cyclopropyl-amino]ethyl}-amide.**
   LC-MS: t_{R} = 0.88 min; [M+H]⁺ = 475.23.
**Example 128:1-Methyl-1*H*-indazole-3-carboxylic acid (2-{[2-amino-5-(3-fluorophenyl)-thiazole-4-carbonyl]-cyclopropyl-amino}-ethyl)-amide.**
   LC-MS: t_{R} = 0.88 min; [M+H]⁺ = 479.2.
**Example 129:1-Methyl-1*H*-indazole-3-carboxylic acid (2-{[2-amino-5-(3-methoxy-phenyl)-thiazole-4-carbonyl]-cyclopropyl-amino}-ethyl)-amide.**
   LC-MS: t_{R} = 0.87 min; [M+H]⁺ = 491.21.
**Example 130:1-Methyl-1*H*-indazole-3-carboxylic acid (2-{[2-amino-5-(4-fluorophenyl)-thiazole-4-carbonyl]-cyclopropyl-amino}-ethyl)-amide.**
   LC-MS: t_{R} = 0.87 min; [M+H]⁺ = 479.2.
**Example 131:1-Methyl-1*H*-indazole-3-carboxylic acid (2-{[2-amino-5-(3-chlorophenyl)-thiazole-4-carbonyl]-cyclopropyl-amino}-ethyl)-amide.**
   LC-MS: t_{R} = 0.91 min; [M+H]⁺ = 495.17.
**Example 132:1-Methyl-1*H*-indazole-3-carboxylic acid {2-[(2-amino-5-phenyl-thiazole-4-carbonyl)-cyclopropyl-amino]-ethyl}-amide.**
   LC-MS: t_{R} = 0.86 min; [M+H]⁺ = 461.22.
**Example 133:1-Methyl-1*H*-indazole-3-carboxylic acid (2-{[2-bromo-5-(3-methoxy-phenyl)-thiazole-4-carbonyl]-cyclopropyl-amino}-ethyl)-amide.**
   LC-MS: t_{R} = 1.02 min; [M+H]⁺ = 554.1.
**Example 134: 1-Methyl-1*H*-indazole-3-carboxylic acid {2-[(2-bromo-5-p-tolyl-thiazole-4-carbonyl)-cyclopropyl-amino]-ethyl}-amide.**
   LC-MS: t_{R} = 1.04 min; [M+H]⁺ = 538.11.
**Example 135: 1-Methyl-1*H*-indazole-3-carboxylic acid (2-{cyclopropyl-[5-(2-fluoro-phenyl)-2-methyl-thiazole-4-carbonyl]-amino}-ethyl)-amide.**
   LC-MS: t_{R} = 0.96 min; [M+H]⁺ = 478.2.
**Example 136:1-Methyl-1*H*-indazole-3-carboxylic acid (2-{cyclopropyl-[2-methyl-5-(3-trifluoromethyl-phenyl)-thiazole-4-carbonyl]-amino}-ethyl)-amide.**
   LC-MS: t_{R} = 1.01 min; [M+H]⁺ = 528.18.
**Example 137:1-Methyl-1*H*-indazole-3-carboxylic acid (2-{cyclopropyl-[5-(3-fluoro-phenyl)-2-methyl-thiazole-4-carbonyl]-amino}-ethyl)-amide.**
   LC-MS: t_{R} = 0.96 min; [M+H]⁺ = 478.2.
**Example 138:1-Methyl-1*H*-indazole-3-carboxylic acid (2-{cyclopropyl-[2-methyl-5-(4-trifluoromethyl-phenyl)-thiazole-4-carbonyl]-amino}-ethyl)-amide.**
   LC-MS: t_{R} = 1.02 min; [M+H]⁺ = 528.16.
**Example 139:1-Methyl-1*H*-indazole-3-carboxylic acid (2-{[5-(4-chloro-phenyl)-2-methyl-thiazole-4-carbonyl]-cyclopropyl-amino}-ethyl)-amide.**
   LC-MS: t_{R} = 0.99 min; [M+H]⁺ = 494.16.
**Example 140:1-Methyl-1*H*-indazole-3-carboxylic acid (2-{cyclopropyl-[5-(3-methoxy-phenyl)-2-methyl-thiazole-4-carbonyl]-amino}-ethyl)-amide.**
   LC-MS: t_{R} = 0.96 min; [M+H]⁺ = 490.22.
**Example 141:1-Methyl-1*H*-indazole-3-carboxylic acid (2-{[5-(3-cyano-phenyl)-2-methyl-thiazole-4-carbonyl]-cyclopropyl-amino}-ethyl)-amide.**
   LC-MS: t_{R} = 0.93 min; [M+H]⁺ = 485.2.
**Example 142:1-Methyl-1*H*-indazole-3-carboxylic acid (2-{cyclopropyl-[5-(3,4-dimethyl-phenyl)-2-methyl-thiazole-4-carbonyl]-amino}-ethyl)-amide.**

LC-MS: t_{R} = 1.01 min; [M+H]⁺ = 488.23.
**Example 143:1-Methyl-1*H*-indazole-3-carboxylic acid {2-[cyclopropyl-(2-methyl-5-*p*-tolyl-thiazole-4-carbonyl)-amino]-ethyl}-amide.**
   LC-MS: t_{R} = 0.98 min; [M+H]⁺ = 474.23.
**Example 144:1-Methyl-1*H*-indazole-3-carboxylic acid {2-[cyclopropyl-(3'-methyl-biphenyl-2-carbonyl)-amino]-ethyl}-amide.**
   LC-MS: t_{R} = 1.03 min; [M+H]⁺ = 453.25.
**Example 145:1-Methyl-1*H*-indazole-3-carboxylic acid {2-[cyclopropyl-(4'-methyl-bi phenyl-2-carbonyl)-amino]-ethyl}-amide.**
   LC-MS: t_{R} = 1.03 min; [M+H]⁺ = 453.27.
**Example 146:1-Methyl-1*H*-indazole-3-carboxylic acid {2-[cyclopropyl-(4'-fluoro-biphenyl-2-carbonyl)-amino]-ethyl}-amide.**
   LC-MS: t_{R} = 1 min; [M+H]⁺ = 457.24.
**Example 147: 2-Cyclopropyl-5-phenyl-thiazole-4-carboxylic acid {2-[(benzofuran-4-carbonyl)-amino]-ethyl}-cyclopropyl-amide.**
   LC-MS: t_{R} = 1.01 min; [M+H]⁺ = 472.22.
**Example 148: 2-Cyclopropyl-5-*m*-tolyl-thiazole-4-carboxylic acid {2-[(benzofuran-4-carbonyl)-amino]-ethyl}-cyclopropyl-amide.**
   LC-MS: t_{R} = 1.04 min; [M+H]⁺ = 486.21.
**Example 149: 2-Cyclopropyl-5-(3-methoxy-phenyl)-thiazole-4-carboxylic acid {2-[(benzofuran-4-carbonyl)-amino]-ethyl}-cyclopropyl-amide.**
   LC-MS: t_{R} = 1.02 min; [M+H]⁺ = 502.12.
**Example 150:2-Amino-5-*m*-tolyl-thiazole-4-carboxylic acid {2-[(benzofuran-4-carbonyl)-amino]-ethyl}-cyclopropyl-amide.**
   LC-MS: t_{R} = 0.88 min; [M+H]⁺ = 461.19.
**Example 151:2-Amino-5-(3-fluoro-phenyl)-thiazole-4-carboxylic acid {2-[(benzofuran-4-carbonyl)-amino]-ethyl}-cyclopropyl-amide.**
   LC-MS: t_{R} = 0.88 min; [M+H]⁺ = 465.18.
**Example 152:2-Amino-5-(3-methoxy-phenyl)-thiazole-4-carboxylic acid {2-[(benzofuran-4-carbonyl)-amino]-ethyl}-cyclopropyl-amide.**
   LC-MS: t_{R} = 0.87 min; [M+H]⁺ = 477.19.
**Example 153: 2-Amino-5-(4-fluoro-phenyl)-thiazole-4-carboxylic acid {2-[(benzofuran-4-carbonyl)-amino]-ethyl}-cyclopropyl-amide.**
   LC-MS: t_{R} = 0.87 min; [M+H]⁺ = 465.18.
**Example 154:2-Amino-5-(3-chloro-phenyl)-thiazole-4-carboxylic acid {2-[(benzofuran-4-carbonyl)-amino]-ethyl}-cyclopropyl-amide.**
   LC-MS: t_{R} = 0.91 min; [M+H]⁺ = 481.14.
**Example 155: 2-Amino-5-phenyl-thiazole-4-carboxylic acid {2-[(benzofuran-4-carbonyl)-amino]-ethyl}-cyclopropyl-amide.**
   LC-MS: t_{R} = 0.85 min; [M+H]⁺ = 447.18.
**Example 156: 2-Bromo-5-(3-methoxy-phenyl)-thiazole-4-carboxylic acid {2-[(benzofuran-4-carbonyl)-amino]-ethyl}-cyclopropyl-amide.**
   LC-MS: t_{R} = 1.02 min; [M+H]⁺ = 540.08.
**Example 157: 2-Bromo-5-*p*-tolyl-thiazole-4-carboxylic acid {2-[(benzofuran-4-carbonyl)-amino]-ethyl}-cyclopropyl-amide.**
   LC-MS: t_{R} = 1.04 min; [M+H]⁺ = 524.06.
**Example 158: 5-(2-Fluoro-phenyl)-2-methyl-thiazole-4-carboxylic acid {2-[(benzofuran-4-carbonyl)-ami no]-ethyl}-cyclopropyl-amide.**
   LC-MS: t_{R} = 0.96 min; [M+H]⁺ = 464.18.
**Example 159:2-Methyl-5-(3-trifluoromethyl-phenyl)-thiazole-4-carboxylic acid {2-[(benzofuran-4-carbonyl)-amino]-ethyl}-cyclopropyl-amide.**
   LC-MS: t_{R} = 1.01 min; [M+H]⁺ = 514.14.
**Example 160: 5-(3-Fluoro-phenyl)-2-methyl-thiazole-4-carboxylic acid {2-[(benzofuran-4-carbonyl)-amino]-ethyl}-cyclopropyl-amide.**
   LC-MS: t_{R} = 0.97 min; [M+H]⁺ = 464.18.
**Example 161: 2-Methyl-5-(4-trifluoromethyl-phenyl)-thiazole-4-carboxylic acid {2-[(benzofuran-4-carbonyl)-amino]-ethyl}-cyclopropyl-amide.**
   LC-MS: t_{R} = 1.02 min; [M+H]⁺ = 514.14.
**Example 162: 5-(4-Chloro-phenyl)-2-methyl-thiazole-4-carboxylic acid {2-[(benzofuran-4-carbonyl)-amino]-ethyl}-cyclopropyl-amide.**
   LC-MS: t_{R} = 0.99 min; [M+H]⁺ = 480.15.
**Example 163: 5-(3-Methoxy-phenyl)-2-methyl-thiazole-4-carboxylic acid {2-[(benzofuran-4-carbonyl)-amino]-ethyl}-cyclopropyl-amide.**
   LC-MS: t_{R} = 0.96 min; [M+H]⁺ = 476.19.
**Example 164: 5-(3-Cyano-phenyl)-2-methyl-thiazole-4-carboxylic acid {2-[(benzofuran-4-carbonyl)-amino]-ethyl}-cyclopropyl-amide.**
   LC-MS: t_{R} = 0.94 min; [M+H]⁺ = 471.18.
**Example 165: 5-(3,4-Dimethyl-phenyl)-2-methyl-thiazole-4-carboxylic acid {2-[(benzofuran-4-carbonyl)-amino]-ethyl}-cyclopropyl-amide.**
   LC-MS: t_{R} = 1.01 min; [M+H]⁺ = 474.22.
**Example 166: 2-Methyl-5-*p*-tolyl-thiazole-4-carboxylic acid {2-[(benzofuran-4-carbonyl)-amino]-ethyl}-cyclopropyl-amide.**
   LC-MS: t_{R} = 0.99 min; [M+H]⁺ = 460.19.
**Example 167: Benzofuran-4-carboxylic acid {2-[cyclopropyl-(3'-methylbiphenyl-2-carbonyl)-amino]-ethyl}-amide.**
   LC-MS: t_{R} = 1.03 min; [M+H]⁺ = 439.21.
**Example 168: Benzofuran-4-carboxylic acid {2-[cyclopropyl-(4'-methylbiphenyl-2-carbonyl)-amino]-ethyl}-amide.**
   LC-MS: t_{R} = 1.03 min; [M+H]⁺ = 439.22.
**Example 169: Benzofuran-4-carboxylic acid {2-[cyclopropyl-(4'-fluoro-biphenyl-2-carbonyl)-amino]-ethyl}-amide.**
   LC-MS: t_{R} = 1 min; [M+H]⁺ = 443.2.
**Example 170:6-Methyl-imidazo[2,1-b]thiazole-5-carboxylic acid {2-[cyclopropylmethyl-(2-cyclopropyl-5-phenyl-thiazole-4-carbonyl)-amino]-ethyl}-**amide.
   LC-MS: t_{R} = 0.9 min; [M+H]⁺ = 506.17.
**Example 171: 6-Methyl-imidazo[2,1-b]thiazole-5-carboxylic acid {2-[cyclopropylmethyl-(2-cyclopropyl-5-*m*-tolyl-thiazole-4-carbonyl)-amino]-ethyl}-amide.**
   LC-MS: t_{R} = 0.94 min; [M+H]⁺ = 520.2.
**Example 172: 6-Methyl-imidazo[2,1-b]thiazole-5-carboxylic acid (2-{[2-cyclopropyl-5-(3-methoxy-phenyl)-thiazole-4-carbonyl]-cyclopropylmethyl-amino}-ethyl)-amide.**
   LC-MS: t_{R} = 0.91 min; [M+H]⁺ = 536.19.
**Example 173:6-Methyl-imidazo[2,1-b]thiazole-5-carboxylic acid {2-[(2-amino-5-*m*-tolyl-thiazole-4-carbonyl)-cyclopropylmethyl-amino]-ethyl}-amide.**
   LC-MS: t_{R} = 0.8 min; [M+H]⁺ = 495.2.
**Example 174:6-Methyl-imidazo[2,1-b]thiazole-5-carboxylic acid (2-{[2-amino-5-(3-fluoro-phenyl)-thiazole-4-carbonyl]-cyclopropylmethyl-amino}-ethyl)-amide.**
   LC-MS: t_{R} = 0.79 min; [M+H]⁺ = 499.15.
**Example 175: 6-Methyl-imidazo[2,1-b]thiazole-5-carboxylic acid (2-{[2-amino-5-(3-methoxy-phenyl)-thiazole-4-carbonyl]-cyclopropylmethyl-amino}-ethyl)-amide.**
   LC-MS: t_{R} = 0.77 min; [M+H]⁺ = 511.07.
**Example 176:6-Methyl-imidazo[2,1-b]thiazole-5-carboxylic acid (2-{[2-amino-5-(4-fluoro-phenyl)-thiazole-4-carbonyl]-cyclopropylmethyl-amino}-ethyl)-amide.**
   LC-MS: t_{R} = 0.78 min; [M+H]⁺ = 499.18.
**Example 177: 6-Methyl-imidazo[2,1-b]thiazole-5-carboxylic acid (2-{[2-amino-5-(3-chloro-phenyl)-thiazole-4-carbonyl]-cyclopropylmethyl-amino}-ethyl)-amide.**
   LC-MS: t_{R} = 0.81 min; [M+H]⁺ = 515.12.
**Example 178: 6-Methyl-imidazo[2,1-b]thiazole-5-carboxylic acid {2-[(2-amino-5-phenyl-thiazole-4-carbonyl)-cyclopropylmethyl-amino]-ethyl}-amide.**
   LC-MS: t_{R} = 0.77 min; [M+H]⁺ = 481.17.
**Example 179:6-Methyl-imidazo[2,1-b]thiazole-5-carboxylic acid (2-{[2-bromo-5-(3-methoxy-phenyl)-thiazole-4-carbonyl]-cyclopropylmethyl-amino}-ethyl)-amide.**
   LC-MS: t_{R} = 0.89 min; [M+H]⁺ = 574.04.
**Example 180:6-Methyl-imidazo[2,1-b]thiazole-5-carboxylic acid {2-[(2-bromo-5-*p*-tolyl-thiazole-4-carbonyl)-cyclopropylmethyl-amino]-ethyl}-amide.**
   LC-MS: t_{R} = 0.91 min; [M+H]⁺ = 558.04.
**Example 181:6-Methyl-imidazo[2,1-b]thiazole-5-carboxylic acid (2-{cyclopropylmethyl-[5-(2-fluoro-phenyl)-2-methyl-thiazole-4-carbonyl]-amino}-ethyl)-amide.**
   LC-MS: t_{R} = 0.86 min; [M+H]⁺ = 498.16.
**Example 182:6-Methyl-imidazo[2,1-b]thiazole-5-carboxylic acid (2-{cyclopropylmethyl-[2-methyl-5-(3-trifluoromethyl-phenyl)-thiazole-4-carbonyl]-amino}-ethyl)-amide.**
   LC-MS: t_{R} = 0.92 min; [M+H]⁺ = 548.15.
**Example 183: 6-Methyl-imidazo[2,1-b]thiazole-5-carboxylic acid (2-{cyclopropylmethyl-[5-(3-fluoro-phenyl)-2-methyl-thiazole-4-carbonyl]-amino}-ethyl)-amide.**
   LC-MS: t_{R} = 0.86 min; [M+H]⁺ = 498.17.
**Example 184:6-Methyl-imidazo[2,1-b]thiazole-5-carboxylic acid (2-{cyclopropylmethyl-[2-methyl-5-(4-trifluoromethyl-phenyl)-thiazole-4-carbonyl]-amino}-ethyl)-amide.**
   LC-MS: t_{R} = 0.92 min; [M+H]⁺ = 548.15.
**Example 185: 6-Methyl-imidazo[2,1-b]thiazole-5-carboxylic acid (2-{[5-(4-chlorophenyl)-2-methyl-thiazole-4-carbonyl]-cyclopropylmethyl-amino}-ethyl)-amide.**
   LC-MS: t_{R} = 0.88 min; [M+H]⁺ = 514.13.
**Example 186:6-Methyl-imidazo[2,1-b]thiazole-5-carboxylic acid (2-{cyclopropytmethyl-[5-(3-methoxy-phenyl)-2-methyl-thiazole-4-carbonyl]-amino}-ethyl)-amide.**
   LC-MS: t_{R} = 0.85 min; [M+H]⁺ = 510.13.
**Example 187:6-Methyl-imidazo[2,1-b]thiazole-5-carboxylic acid (2-{[5-(3-cyanophenyl)-2-methyl-thiazole-4-carbonyl]-cyclopropylmethyl-amino}-ethyl)-amide.**
   LC-MS: t_{R} = 0.83 min; [M+H]⁺ = 505.16.
**Example 188:6-Methyl-imidazo[2,1-b]thiazole-5-carboxylic acid (2-{cyclopropylmethyl-[5-(3,4-dimethyl-phenyl)-2-methyl-thiazole-4-carbonyl]-amino}-ethyl)-amide.**
   LC-MS: t_{R} = 0.9 min; [M+H]⁺ = 508.2.
**Example 189: 6-Methyl-imidazo[2,1-b]thiazole-5-carboxylic acid {2-[cyclopropylmethyl-(2-methyl-5-*p-*tolyl-thiazole-4-carbonyl)-amino]-ethyl}-amide.**
   LC-MS: t_{R} = 0.88 min; [M+H]⁺ = 494.2.
**Example 190: 6-Methyl-imidazo[2,1-b]thiazole-5-carboxylic acid {2-[cyclopropylmethyl-(3'-methyl-biphenyl-2-carbonyl)-amino]-ethyl}-amide.**
   LC-MS: t_{R} = 0.93 min; [M+H]⁺ = 473.23.
**Example 191: 6-Methyl-imidazo[2,1-b]thiazole-5-carboxylic acid {2-[cyclopropylmethyl-(4'-methyl-biphenyl-2-carbonyl)-amino]-ethyl}-amide.**
   LC-MS: t_{R} = 0.91 min; [M+H]⁺ = 473.23.
**Example 192: 1-Methyl-1*H-*indazole-3-carboxylic acid {2-[cyclopropylmethyl-(2-cyclopropyl-5-phenyl-thiazole-4-carbonyl)-amino]-ethyl}-amide.**
   LC-MS: t_{R} = 1.04 min; [M+H]⁺ = 500.07.
**Example 193: 1-Methyl-1*H-*indazole-3-carboxylic acid {2-[cyclopropylmethyl-(2-cyclopropyl-5-*m*-tolyl-thiazole-4-carbonyl)-amino]-ethyl}-amide.**
   LC-MS: t_{R} = 1.07 min; [M+H]⁺ = 514.25.
**Example 194: 1-Methyl-1*H-*indazole-3-carboxylic acid (2-{[2-cyclopropyl-5-(3-methoxy-phenyl)-thiazole-4-carbonyl]-cyclopropylmethyl-amino}-ethyl)-amide.**
   LC-MS: t_{R} = 1.04 min; [M+H]⁺ = 530.24.
**Example 195: 1-Methyl-1*H-*indazole-3-carboxylic acid {2-[(2-amino-5-*m-*tolyl-thiazole-4-carbonyl)-cyclopropylmethyl-amino]-ethyl}-amide.**
   LC-MS: t_{R} = 0.92 min; [M+H]⁺ = 489.23.
**Example 196: 1-Methyl-1*H-*indazole-3-carboxylic acid (2-{[2-amino-5-(3-fluoro-phenyl)-thiazole-4-carbonyl]-cyclopropylmethyl-amino}-ethyl)-amide.**
   LC-MS: t_{R} = 0.92 min; [M+H]⁺ = 493.2.
**Example 197: 1-Methyl-1*H-*indazole-3-carboxylic acid (2-{[2-amino-5-(3-methoxy-phenyl)-thiazole-4-carbonyl]-cyclopropylmethyl-amino}-ethyl)-amide.**
   LC-MS: t_{R} = 0.9 min; [M+H]⁺ = 505.22.
**Example 198: 1-Methyl-1*H-*indazole-3-carboxylic acid (2-{[2-amino-5-(4-fluoro-phenyl)-thiazole-4-carbonyl]-cyclopropylmethyl-amino}-ethyl)-amide.**
   LC-MS: t_{R} = 0.91 min; [M+H]⁺ = 493.2.
**Example 199: 1-Methyl-1*H-*indazole-3-carboxylic acid (2-{[2-amino-5-(3-chloro-phenyl)-thiazole-4-carbonyl]-cyclopropylmethyl-amino}-ethyl)-amide.**
   LC-MS: t_{R} = 0.95 min; [M+H]⁺ = 509.17.
**Example 200: 1-Methyl-1*H-*indazole-3-carboxylic acid {2-[(2-amino-5-phenyl-thiazole-4-carbonyl)-cyclopropylmethyl-amino]-ethyl}-amide.**
   LC-MS: t_{R} = 0.89 min; [M+H]⁺ = 475.23.
**Example 201:1-Methyl-1*H-*indazole-3-carboxylic acid (2-{[2-bromo-5-(3-methoxy-phenyl)-thiazole-4-carbonyl]-cyclopropylmethyl-amino}-ethyl)-amide.**
   LC-MS: t_{R} = 1.04 min; [M+H]⁺ = 570.12.
**Example 202: 1-Methyl-1*H-*indazole-3-carboxylic acid {2-[(2-bromo-5-*p-*tolyl-thiazole-4-carbonyl)-cyclopropylmethyl-amino]-ethyl}-amide.**
   LC-MS: t_{R} = 1.06 min; [M+H]⁺ = 554.11.
**Example 203: 1-Methyl-1*H-*indazole-3-carboxylic acid (2-{cyclopropylmethyl-[5-(2-fluoro-phenyl)-2-methyl-thiazole-4-carbonyl]-amino}-ethyl)-amide.**
   LC-MS: t_{R} = 1 min; [M+H]⁺ = 492.2.
**Example 204: 1-Methyl-1*H-*indazole-3-carboxylic acid (2-{cyclopropylmethyl-[2-methyl-5-(3-trifluoromethyl-phenyl)-thiazole-4-carbonyl]-amino}-ethyl)-amide.**
   LC-MS: t_{R} = 1.05 min; [M+H]⁺ = 542.11.
**Example 205: 1-Methyl-1*H-*indazole-3-carboxylic acid (2-{cyclopropylmethyl-[5-(3-fluoro-phenyl)-2-methyl-thiazole-4-carbonyl]-amino}-ethyl)-amide.**
   LC-MS: t_{R} = 0.99 min; [M+H]⁺ = 492.21.
**Example 206: 1-Methyl-1*H-*indazole-3-carboxylic acid (2-{cyclopropylmethyl-[2-methyl-5-(4-trifluoromethyl-phenyl)-thiazole-4-carbonyl]-amino}-ethyl)-amide.**
   LC-MS: t_{R} = 1.04 min; [M+H]⁺ = 542.12.
**Example 207: 1-Methyl-1*H-*indazole-3-carboxylic acid (2-{[5-(4-chloro-phenyl)-2-methyl-thiazole-4-carbonyl]-cyclopropylmethyl-amino}-ethyl)-amide.**
   LC-MS: t_{R} = 1.02 min; [M+H]⁺ = 508.18.
**Example 208: 1-Methyl-1*H-*indazole-3-carboxylic acid (2-{cyclopropylmethyl-[5-(3-methoxy-phenyl)-2-methyl-thiazole-4-carbonyl]-amino}-ethyl)-amide.**
   LC-MS: t_{R} = 0.99 min; [M+H]⁺ = 504.22.
**Example 209: 1-Methyl-1*H-*indazole-3-carboxylic acid (2-{[5-(3-cyano-phenyl)-2-methyl-thiazole-4-carbonyl]-cyclopropylmethyl-amino}-ethyl)-amide.**
   LC-MS: t_{R} = 0.97 min; [M+H]⁺ = 499.24.
**Example 210: 1-Methyl-1*H-*indazole-3-carboxylic acid (2-{cyclopropylmethyl-[5-(3,4-dimethyl-phenyl)-2-methyl-thiazole-4-carbonyl]-amino}-ethyl)-amide.**
   LC-MS: t_{R} = 1.03 min; [M+H]⁺ = 502.16.
**Example 211: 1-Methyl-1*H-*indazole-3-carboxylic acid {2-[cyclopropylmethyl-(2-methyl-5-*p-*tolyl-thiazole-4-carbonyl)-amino]-ethyl}-amide.**
   LC-MS: t_{R} = 1.01 min; [M+H]⁺ = 488.24.
**Example 212: 1-Methyl-1*H-*indazole-3-carboxylic acid {2-[cyclopropylmethyl-(3'-methyl-biphenyl-2-carbonyl)-amino]-ethyl}-amide.**
   LC-MS: t_{R} = 1.06 min; [M+H]⁺ = 467.27.
**Example 213: 1-Methyl-1*H-*indazole-3-carboxylic acid {2-[cyclopropylmethyl-(4'-methyl-biphenyl-2-carbonyl)-amino]-ethyl)-amide.**
   LC-MS: t_{R} = 1.06 min; [M+H]⁺ = 467.27.
**Example 214: 1-Methyl-1*H-*indazole-3-carboxylic acid {2-[cyclopropylmethyl-(4'-fluoro-biphenyl-2-carbonyl)-amino]-ethyl}-amide.**
   LC-MS: t_{R} = 1.04 min; [M+H]⁺ = 471.25.
**Example 215: 2-Cyclopropyl-5-phenyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.99 min; [M+H]⁺ = 490.2.
**Example 216: 2-Cyclopropyl-5-*m-*tolyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 1.02 min; [M+H]⁺ = 504.22.
**Example 217: 2-Cyclopropyl-5-(3-methoxy-phenyl)-thiazole-4-carboxylic acid cyclopropyl-{2-[(2,3-dihydro-benzo[1,4]dioxine-5-carbonyl)-amino]-ethyl}-amide.**
   LC-MS: t_{R} = 1 min; [M+H]⁺ = 520.21.
**Example 218: 2-Amino-5-*m-*tolyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.86 min; [M+H]⁺ = 479.21.
**Example 219: 2-Amino-5-(3-fluoro-phenyl)-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.87 min; [M+H]⁺ = 483.18.
**Example 220: 2-Amino-5-(3-methoxy-phenyl)-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.85 min; [M+H]⁺ = 495.19.
**Example 221: 2-Amino-5-(4-fluoro-phenyl)-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.85 min; [M+H]⁺ = 483.19.
**Example 222: 2-Amino-5-(3-chloro-phenyl)-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.89 min; [M+H]⁺ = 499.21.
**Example 223: 2-Amino-5-phenyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.84 min; [M+H]⁺ = 465.2.
**Example 224: 2-Bromo-5-(3-methoxy-phenyl)-thiazole-4-carboxylic acid cyclopropyl-{2-[(2,3-dihydro-benzo[1,4]dioxine-5-carbonyl)-amino]-ethyl}-amide.**
   LC-MS: t_{R} = 1.01 min; [M+H]⁺ = 558.05.
**Example 225: 2-Bromo-5-*p-*tolyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 1.03 min; [M+H]⁺ = 542.04.
**Example 226: 5-(2-Fluoro-phenyl)-2-methyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.94 min; [M+H]⁺ = 482.18.
**Example 227: 2-Methyl-5-(3-trifluoromethyl-phenyl)-thiazole-4-carboxylic acid cyclopropyl-{2-[(2,3-dihydro-benzo[1,4]dioxine-5-carbonyl)-amino]-ethyl}-amide.**
   LC-MS: t_{R} = 1 min; [M+H]⁺ = 532.17.
**Example 228: 5-(3-Fluoro-phenyl)-2-methyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.95 min; [M+H]⁺ = 482.18.
**Example 229: 5-(4-Chloro-phenyl)-2-methyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.98 min; [M+H]⁺ = 498.15.
**Example 230: 5-(3-Methoxy-phenyl)-2-methyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.94 min; [M+H]⁺ = 494.2.
**Example 231: 5-(3,4-Dimethyl-phenyl)-2-methyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.99 min; [M+H]⁺ = 492.23.
**Example 232: 2-Methyl-5-*p-*tolyl-thiazole-4-carboxylic acid cyclopropyl-{2-[(2,3-dihydro-benzo[1,4]dioxine-5-carbonyl)-amino]-ethyl}-amide.**
   LC-MS: t_{R} = 0.97 min; [M+H]⁺ = 478.21.
**Example 233: 2,3-Dihydro-benzo[1,4]dioxine-5-carboxylic acid {2-[cyclopropyl-(3'-methyl-biphenyl-2-carbonyl)-amino]-ethyl}-amide.**
   LC-MS: t_{R} = 1 min; [M+H]⁺ = 457.25.
**Example 234: 2,3-Dihydro-benzo[1,4]dioxine-5-carboxylic acid {2-[cyclopropyl-(4'-methyl-biphenyl-2-carbonyl)-amino]-ethyl}-amide.**
   LC-MS: t_{R} = 1.01 min; [M+H]⁺ = 457.24.
**Example 235: 2,3-Dihydro-benzo[1,4]dioxine-5-carboxylic acid {2-[cyclopropyl-(4'-fluoro-biphenyl-2-carbonyl)-amino]-ethyl}-amide.**
   LC-MS: t_{R} = 0.98 min; [M+H]⁺ = 461.22.
**Example 236: 2-Cyclopropyl-5-phenyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 1.02 min; [M+H]⁺ = 504.2.
**Example 237: 2-Cyclopropyl-5-*m-*tolyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 1.05 min; [M+H]⁺ = 518.15.
**Example 238: 2-Cyclopropyl-5-(3-methoxy-phenyl)-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 1.03 min; [M+H]⁺ = 534.13.
**Example 239: 2-Amino-5-*m-*tolyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 0.9 min; [M+H]⁺ = 493.21.
**Example 240: 2-Amino-5-(3-fluoro-phenyl)-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 0.91 min; [M+H]⁺ = 497.17.
**Example 241: 2-Amino-5-(3-methoxy-phenyl)-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 0.9 min; [M+H]⁺ = 509.19.
**Example 242: 2-Amino-5-phenyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 0.9 min; [M+H]⁺ = 479.2.
**Example 243: 2-Bromo-5-(3-methoxy-phenyl)-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 1.03 min; [M+H]⁺ = 572.09.
**Example 244: 2-Bromo-5-*p-*tolyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 1.06 min; [M+H]⁺ = 556.09.
**Example 245: 5-(2-Fluoro-phenyl)-2-methyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 0.98 min; [M+H]⁺ = 496.18.
**Example 246: 2-Methyl-5-(3-trifluoromethyl-phenyl)-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 1.03 min; [M+H]⁺ = 546.17.
**Example 247: 5-(3-Fluoro-phenyl)-2-methyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 0.98 min; [M+H]⁺ = 496.18.
**Example 248: 5-(3-Methoxy-phenyl)-2-methyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 0.97 min; [M+H]⁺ = 508.19.
**Example 249: 5-(3-Cyano-phenyl)-2-methyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 0.95 min; [M+H]⁺ = 503.09.
**Example 250: 5-(3,4-Dimethyl-phenyl)-2-methyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 1.02 min; [M+H]⁺ = 506.22.
**Example 251: 2-Methyl-5-*p-*tolyl-thiazole-4-carboxylic acid cyclopropylmethyl-{2-[(2,3-dihydro-benzo[1,4]dioxine-5-carbonyl)-amino]-ethyl}-amide.**
   LC-MS: t_{R} = 1 min; [M+H]⁺ = 492.22.
**Example 252: 2,3-Dihydro-benzo[1,4]dioxine-5-carboxylic acid {2-[cyclopropylmethyl-(3'-methyl-biphenyl-2-carbonyl)-amino]-ethyl}-amide.**
   LC-MS: t_{R} = 1.03 min; [M+H]⁺ = 471.25.
**Example 253: 2,3-Dihydro-benzo[1,4]dioxine-5-carboxylic acid {2-[cyclopropylmethyl-(4'-methyl-biphenyl-2-carbonyl)-amino]-ethyl}-amide.**
   LC-MS: t_{R} = 1.04 min; [M+H]⁺ = 471.25.
**Example 254: 2,3-Dihydro-benzo[1,4]dioxine-5-carboxylic acid {2-[cyclopropylmethyl-(4'-fluoro-biphenyl-2-carbonyl)-amino]-ethyl}-amide.**
   LC-MS: t_{R} = 1.02 min; [M+H]⁺ = 475.24.
**Example 255: 2-Cyclopropyl-5-phenyl-thiazole-4-carboxylic acid {2-[(benzofuran-4-carbonyl)-amino]-ethyl}-cyclopropylmethyl-amide.**
   LC-MS: t_{R} = 1.04 min; [M+H]⁺ = 486.2.
**Example 256: 2-Cyclopropyl-5-*m-*tolyl-thiazole-4-carboxylic acid {2-[(benzofuran-4-carbonyl)-amino]-ethyl}-cyclopropylmethyl-amide.**
   LC-MS: t_{R} = 1.06 min; [M+H]⁺ = 499.93.
**Example 257: 2-Cyclopropyl-5-(3-methoxy-phenyl)-thiazole-4-carboxylic acid {2-[(benzofuran-4-carbonyl)-amino]-ethyl}-cyclopropylmethyl-amide.**
   LC-MS: t_{R} = 1.04 min; [M+H]⁺ = 516.2.
**Example 258: 2-Amino-5-*m-*tolyl-thiazole-4-carboxylic acid {2-[(benzofuran-4-carbonyl)-amino]-ethyl}-cyclopropylmethyl-amide.**
   LC-MS: t_{R} = 0.92 min; [M+H]⁺ = 475.21.
**Example 259: 2-Amino-5-(3-fluoro-phenyl)-thiazole-4-carboxylic acid {2-[(benzofuran-4-carbonyl)-amino]-ethyl}-cyclopropylmethyl-amide.**
   LC-MS: t_{R} = 0.92 min; [M+H]⁺ = 479.18.
**Example 260: 2-Amino-5-(3-methoxy-phenyl)-thiazole-4-carboxylic acid {2-[(benzofuran-4-carbonyl)-amino]-ethyl}-cyclopropylmethyl-amide.**
   LC-MS: t_{R} = 0.9 min; [M+H]⁺ = 491.19.
**Example 261: 2-Amino-5-(4-fluoro-phenyl)-thiazole-4-carboxylic acid {2-[(benzofuran-4-carbonyl)-amino]-ethyl}-cyclopropylmethyl-amide.**
   LC-MS: t_{R} = 0.91 min; [M+H]⁺ = 479.18.
**Example 262: 2-Amino-5-(3-chloro-phenyl)-thiazole-4-carboxylic acid {2-[(benzofuran-4-carbonyl)-amino]-ethyl}-cyclopropylmethyl-amide.**
   LC-MS: t_{R} = 0.95 min; [M+H]⁺ = 495.14.
**Example 263: 2-Amino-5-phenyl-thiazole-4-carboxylic acid {2-[(benzofuran-4-carbonyl)-amino]-ethyl}-cyclopropylmethyl-amide.**
   LC-MS: t_{R} = 0.89 min; [M+H]⁺ = 461.19.
**Example 264: 2-Bromo-5-(3-methoxy-phenyl)-thiazole-4-carboxylic acid {2-[(benzofuran-4-carbonyl)-amino]-ethyl}-cyclopropylmethyl-amide.**
   LC-MS: t_{R} = 1.05 min; [M+H]⁺ = 556.07.
**Example 265: 2-Bromo-5-*p-*tolyl-thiazole-4-carboxylic acid {2-[(benzofuran-4-carbonyl)-amino]-ethyl}-cyclopropylmethyl-amide.**
   LC-MS: t_{R} = 1.07 min; [M+H]⁺ = 540.08.
**Example 266: 5-(2-Fluoro-phenyl)-2-methyl-thiazole-4-carboxylic acid {2-[(benzofuran-4-carbonyl)-amino]-ethyl}-cyclopropylmethyl-amide.**
   LC-MS: t_{R} = 1 min; [M+H]⁺ = 478.17.
**Example 267: 2-Methyl-5-(3-trifluoromethyl-phenyl)-thiazole-4-carboxylic acid {2-[(benzofuran-4-carbonyl)-amino]-ethyl}-cyclopropylmethyl-amide.**
   LC-MS: t_{R} = 1.04 min; [M+H]⁺ = 528.16.
**Example 268: 5-(3-Fluoro-phenyl)-2-methyl-thiazole-4-carboxylic acid {2-[(benzofuran-4-carbonyl)-amino]-ethyl}-cyclopropylmethyl-amide.**
   LC-MS: t_{R} = 1 min; [M+H]⁺ = 478.19.
**Example 269: 2-Methyl-5-(4-trifluoromethyl-phenyl)-thiazole-4-carboxylic acid {2-[(benzofuran-4-carbonyl)-amino]-ethyl}-cyclopropylmethyl-amide.**
   LC-MS: t_{R} = 1.05 min; [M+H]⁺ = 528.16.
**Example 270: 5-(4-Chloro-phenyl)-2-methyl-thiazole-4-carboxylic acid {2-[(benzofuran-4-carbonyl)-amino]-ethyl}-cyclopropylmethyl-amide.**
   LC-MS: t_{R} = 1.02 min; [M+H]⁺ = 494.15.
**Example 271: 5-(3-Methoxy-phenyl)-2-methyl-thiazole-4-carboxylic acid {2-[(benzofuran-4-carbonyl)-amino]-ethyl}-cyclopropylmethyl-amide.**
   LC-MS: t_{R} = 1 min; [M+H]⁺ = 490.22.
**Example 272: 5-(3-Cyano-phenyl)-2-methyl-thiazole-4-carboxylic acid {2-[(benzofuran-4-carbonyl)-amino]-ethyl}-cyclopropylmethyl-amide.**
   LC-MS: t_{R} = 0.95 min; [M+H]⁺ = 485.18.
**Example 273: 5-(3,4-Dimethyl-phenyl)-2-methyl-thiazole-4-carboxylic acid {2-[(benzofuran-4-carbonyl)-amino]-ethyl}-cyclopropylmethyl-amide.**
   LC-MS: t_{R} = 1.04 min; [M+H]⁺ = 488.22.
**Example 274: 2-Methyl-5-*p-*tolyl-thiazole-4-carboxylic acid {2-[(benzofuran-4-carbonyl)-amino]-ethyl}-cyclopropylmethyl-amide.**
   LC-MS: t_{R} = 1.02 min; [M+H]⁺ = 474.22.
**Example 275: Benzofuran-4-carboxylic acid {2-[cyclopropylmethyl-(3'-methyl-biphenyl-2-carbonyl)-amino]-ethyl}-amide.**
   LC-MS: t_{R} = 1.05 min; [M+H]⁺ = 453.25.
**Example 276: Benzofuran-4-carboxylic acid {2-[cyclopropylmethyl-(4'-methyl-biphenyl-2-carbonyl)-amino]-ethyl}-amide.**
   LC-MS: t_{R} = 1.05 min; [M+H]⁺ = 453.25.
**Example 277: Benzofuran-4-carboxylic acid {2-[cyclopropylmethyl-(4'-fluoro-biphenyl-2-carbonyl)-amino]-ethyl}-amide.**
   LC-MS: t_{R} = 1.01 min; [M+H]⁺ = 457.22.

### II. Biological assays

### In vitro assay

The orexin receptor antagonistic activity of the compounds of formula (I) is determined in accordance with one of the following experimental methods.

### Experimental method:

Chinese hamster ovary (CHO) cells expressing the human orexin-1 receptor and the human orexin-2 receptor, respectively, are grown in culture medium (Ham F-12 with L-Glutamine) containing 300 µg/ml G418, 100 U/ml penicillin, 100 µg/ml streptomycin and 10 % heat inactivated fetal calf serum (FCS). The cells are seeded at 20'000 cells / well into 384-well black clear bottom sterile plates (Greiner). The seeded plates are incubated overnight at 37°C in 5% CO₂.

Human orexin-A as an agonist is prepared as 1 mM stock solution in MeOH: water (1:1), diluted in HBSS containing 0.1 % bovine serum albumin (BSA), NaHCO₃: 0.375g/l and 20 mM HEPES for use in the assay at a final concentration of 3 nM.

Antagonists are prepared as 10 mM stock solution in DMSO, then diluted in 384-well plates, first in DMSO, then in HBSS containing 0.1 % bovine serum albumin (BSA), NaHCO₃: 0.375g/l and 20 mM HEPES.

On the day of the assay, 50 µl of staining buffer (HBSS containing 1% FCS, 20 mM HEPES, NaHCO₃: 0.375g/l, 5 mM probenecid (Sigma) and 3 µM of the fluorescent calcium indicator fluo-4 AM (1 mM stock solution in DMSO, containing 10% pluronic) is added to each well.

The 384-well cell-plates are incubated for 50 min at 37° C in 5% CO₂ followed by equilibration at RT for 30 - 120 min before measurement.

Within the Fluorescent Imaging Plate Reader (FLIPR2 or FLIPR Tetra, Molecular Devices), antagonists are added to the plate in a volume of 10 µl/well, incubated for 10 min and finally 10 µl/well of agonist is added. Fluorescence is measured for each well at 1 second intervals, and the height of each fluorescence peak is compared to the height of the fluorescence peak induced by 3 nM orexin-A with vehicle in place of antagonist. For each antagonist, the IC₅₀ value (the concentration of compound needed to inhibit 50 % of the agonistic response) is determined. Optimized conditions may be achieved by adjustment of pipetting speed and cell splitting regime. The calculated IC₅₀ values of the compounds may fluctuate depending on the daily cellular assay performance. Fluctuations of this kind are known to those skilled in the art.

Antagonistic activities (IC₅₀ values) of all exemplified compounds are below 1000 nM with respect to the OX1 and/or the OX2 receptor. IC₅₀ values of 267 exemplified compounds are in the range of 1.9-8626 nM with respect to the OX1 receptor; 10 compounds have been measured with an IC₅₀ value >10000 nM in this assay. IC₅₀ values of 277 exemplified compounds are in the range of 0.6-1475 nM with respect to the OX2 receptor; 2 compounds have been measured with an IC₅₀ value >10000 nM in this assay. Antagonistic activities of selected compounds are displayed in Table *1.*

**Table 1**

| **Compound of Example** | **OX₁ IC₅₀ (nM)** | **OX₂ IC₅₀ (nM)** |
|---|---|---|
| **2** | 242¹⁾ | 35¹⁾ |
| **3** | 150¹⁾ | 27¹⁾ |
| **18** | 7.8 | 11 |
| **25** | 75 | 12 |
| **31** | 504 | 85 |
| **47** | 112 | 50 |
| **49** | 37 | 33 |
| **71** | 27 | 50 |
| **73** | 119 | 19 |
| **83** | 437 | 303 |
| **88** | 84 | 46 |
| **97** | 5.7 | 5.8 |
| **99** | 114 | 718 |
| **111** | 39* | 23* |
| **140** | 98* | 68* |
| **144** | 95* | 20* |
| **165** | 2.0* | 4.3* |
| **171** | 16* | 12* |
| **179** | 10* | 10* |
| **187** | 17* | 28* |
| **195** | 57* | **12*** |
| **248** | 434* | 29* |
| **252** | 807* | 203* |
| **261** | 263* | 4.2* |
| **262** | 144* | 17* |

| | | |
|---|---|---|
| IC₅₀ values measured with FLIPR 2 or, if marked with *, with FLIPR Tetra; ¹⁾ geometric mean from n=2 values; | | |

## Claims

1. A compound of formula (I) 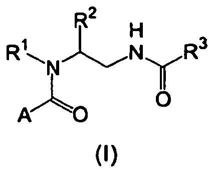 wherein
R¹ represents cyclopropyl or cyclopropyl-methyl;
R² represents hydrogen, (C₁₋₄)alkyl, cyclopropyl, or phenyl-(C₁₋₄)alkyl, wherein the phenyl is unsubstituted, or mono-, di-, or tri-substituted, wherein the substituents are independently selected from the group consisting of (C₁₋₄)alkyl, (C₁₋₄)alkoxy, trifluoromethyl, and halogen;
R³ represents heterocyclyl, wherein said heterocyclyl is an 8- to 10-membered bicyclic ring containing 1, 2 or 3 heteroatoms, each independently selected from oxygen, nitrogen and sulfur, wherein at least one ring of said bicyclic ring system is aromatic and the bond with which the heterocyclyl is attached to the rest of the molecule is positioned on an aromatic carbon atom of said bicyclic ring system in alpha position to a bridgehead atom; wherein said heterocyclyl is unsubstituted, mono-, di-, or tri-substituted, wherein the substituents are independently selected from the group consisting of (C₁₋₄)alkyl, (C₁₋₄)alkoxy, halogen, and trifluoromethyl;
A represents 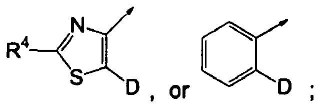 R⁴ represents (C₁₋₄)alkyl, bromo, cyclopropyl, or -NR⁵R 6;
R⁵ represents hydrogen, or (C₁₋₄)alkyl;
R⁶ represents hydrogen, or (C₁₋₄)alkyl; and
D represents phenyl, which is unsubstituted, mono-, di-, or tri-substituted, wherein the substituents are independently selected from the group consisting of (C₁₋₄)alkyl, (C₁₋₄)alkoxy, trifluoromethyl, trifluoromethoxy, cyano, and halogen;
or a salt of such a compound.

2. A compound of formula (I) according to claim 1, wherein A represents 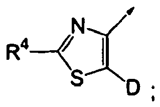 or a salt of such a compound.

3. A compound of formula (I) according to claim 1 or 2, wherein R⁴ represents methyl, -NH₂, bromo, or cyclopropyl;
or a salt of such a compound.

4. A compound of formula (I) according to any one of claims 1 to 3, wherein D represents phenyl, which is unsubstituted, mono- or di-substituted, wherein the substituents are independently selected from the group consisting of (C₁₋₄)alkyl, (C₁₋₄)alkoxy, trifluoromethyl, cyano, and halogen;
or a salt of such a compound.

5. A compound according to any one of claims 1 to 4, wherein R² represents hydrogen, methyl, cyclopropyl, or unsubstituted phenyl-(C₁₋₂)alkyl;
or a salt of such a compound.

6. A compound according to any one of claims 1 to 5, wherein R³ represents a group selected from quinoline-8-yl, isoquinoline-1-yl, indol-3-yl, indol-4-yl, indol-7-yl, indazol-3-yl, indazol-4-yl, indazol-7-yl, benzofuran-4-yl, benzofuran-7-yl, benzisoxazol-3-yl, benzisoxazol-4-yl, benzisoxazol-7-yl, benzoxazol-4-yl, benzoxazol-7-yl, benzoxadiazol-4-yl, benzoxadiazol-7-yl, benzothiophene-3-yl, benzothiophene-4-yl, benzothiophene-7-yl, benzthiazol-4-yl, benzthiazol-7-yl, benzoisothiazol-3-yl, benzoisothiazol-4-yl, benzoisothiazol-7-yl, benzothiadiazol-4-yl, benzothiadiazol-7-yl, benzimidazol-4-yl, benzimidazol-7-yl, imidazo[2,1-b]thiazol-3-yl, imidazo[2,1-b]thiazol-5-yl, pyrazolo[1,5-a]pyridine-3-yl, imidazo[1,2-a]pyridine-3-yl, 2,3-dihydro-thieno[3,4-b][1,4]dioxine-5-yl, benzo[1,3]dioxol-4-yl, 2,3-dihydrobenzofuran-4-yl, 2,3-dihydro-benzofuran-7-yl, 4H-benzo[1,3]dioxin-8-yl, 4H-benzo[1,3]dioxin-5-yl, and 2,3-dihydro-benzo[1,4]dioxin-5-yl; wherein said group is unsubstituted, mono-, or di-substituted, wherein the substituents are independently selected from the group consisting of methyl, methoxy, halogen, and trifluoromethyl; or a salt of such a compound.

7. A compound according to any one of claims 1 to 5, wherein R³ represents 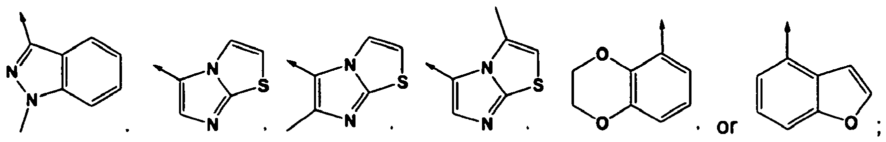 or a salt of such a compound.

8. A compound according to claim 1 selected from the group consisting of:
2-Methyl-5-phenyl-thiazole-4-carboxylic acid {2-[(benzofuran-4-carbonyl)-amino]-ethyl}-cyclopropylmethyl-amide;
6-Methyl-imidazo[2,1-b]thiazole-5-carboxylic acid {2-[cyclopropylmethyl-(2-methyl-5-phenyl-thiazole-4-carbonyl)-amino]-ethyl}-amide;
2-Methyl-5-phenyl-thiazole-4-carboxylic acid cyclopropylmethyl-{2-[(2,3-dihydro-benzo[1,4]dioxine-5-carbonyl)-amino]-ethyl}-amide;
2-Methyl-5-phenyl-thiazole-4-carboxylic acid {2-[(benzofuran-4-carbonyl)-amino]-ethyl}-cyclopropyl-amide;
6-Methyl-imidazo[2,1-b]thiazole-5-carboxylic acid {2-[cyclopropyl-(2-methyl-5-phenyl-thiazole-4-carbonyl)-amino]-ethyl}-amide;
2-Methyl-5-phenyl-thiazole-4-carboxylic acid cyclopropyl-{2-[(2,3-dihydro-benzo[1,4]dioxine-5-carbonyl)-amino]-ethyl}-amide;
6-Methyl-imidazo[2, 1-b]thiazole-5-carboxylic acid {2-[cyclopropyl-(2-cyclopropyl-5-phenyl-thiazole-4-carbonyl)-amino]-ethyl}-amide;
6-Methyl-imidazo[2, 1-b]thiazole-5-carboxylic acid {2-[cyclopropyl-(2-cyclopropyl-5-*m-*tolyl-thiazole-4-carbonyl)-amino]-ethyl}-amide;
6-Methyl-imidazo[2,1-b]thiazole-5-carboxylic acid (2-{cyclopropyl-[2-cyclopropyl-5-(3-methoxy-phenyl)-thiazole-4-carbonyl]-amino}-ethyl)-amide;
6-Methyl-imidazo[2,1-b]thiazole-5-carboxylic acid {2-[(2-amino-5-*m-*tolyl-thiazole-4-carbonyl)-cyclopropyl-amino]-ethyl}-amide;
6-Methyl-imidazo[2,1-b]thiazole-5-carboxylic acid (2-{[2-amino-5-(3-fluoro-phenyl)-thiazole-4-carbonyl]-cyclopropyl-amino}-ethyl)-amide;
6-Methyl-imidazo[2,1-b]thiazole-5-carboxylic acid (2-{[2-amino-5-(3-methoxy-phenyl)-thiazole-4-carbonyl]-cyclopropyl-amino}-ethyl)-amide;
6-Methyl-imidazo[2,1-b]thiazole-5-carboxylic acid (2-{[2-amino-5-(4-fluoro-phenyl)-thiazole-4-carbonyl]-cyclopropyl-amino}-ethyl)-amide;
6-Methyl-imidazo[2,1-b]thiazole-5-carboxylic acid (2-{[2-amino-5-(3-chloro-phenyl)-thiazole-4-carbonyl]-cyclopropyl-amino}-ethyl)-amide;
6-Methyl-imidazo[2,1-b]thiazole-5-carboxylic acid {2-[(2-amino-5-phenyl-thiazole-4-carbonyl)-cyclopropyl-amino]-ethyl}-amide;
6-Methyl-imidazo[2,1-b]thiazole-5-carboxylic acid (2-{[2-bromo-5-(3-methoxy-phenyl)-thiazole-4-carbonyl]-cyclopropyl-amino}-ethyl)-amide;
6-Methyl-imidazo[2,1-b]thiazole-5-carboxylic acid {2-[(2-bromo-5-*p-*tolyl-thiazole-4-carbonyl)-cyclopropyl-amino]-ethyl}-amide;
6-Methyl-imidazo[2,1-b]thiazole-5-carboxylic acid (2-{cyclopropyl-[5-(2-fluoro-phenyl)-2-methyl-thiazole-4-carbonyl]-amino}-ethyl)-amide;
6-Methyl-imidazo[2,1-b]thiazole-5-carboxylic acid (2-{cyclopropyl-[2-methyl-5-(3-trifluoromethyl-phenyl)-thiazole-4-carbonyl]-amino}-ethyl)-amide;
6-Methyl-imidazo[2,1-b]thiazole-5-carboxylic acid (2-{cyclopropyl-[5-(3-fluoro-phenyl)-2-methyl-thiazole-4-carbonyl]-amino}-ethyl)-amide;
6-Methyl-imidazo[2,1-b]thiazole-5-carboxylic acid (2-{cyclopropyl-[2-methyl-5-(4-trifluoromethyl-phenyl)-thiazole-4-carbonyl]-amino}-ethyl)-amide;
6-Methyl-imidazo[2,1-b]thiazole-5-carboxylic acid (2-{[5-(4-chloro-phenyl)-2-methylthiazole-4-carbonyl]-cyclopropyl-amino}-ethyl)-amide;
6-Methyl-imidazo[2,1-b]thiazole-5-carboxylic acid (2-{cyclopropyl-[5-(3-methoxyphenyl)-2-methyl-thiazole-4-carbonyl]-amino}-ethyl)-amide;
6-Methyl-imidazo[2,1-b]thiazole-5-carboxylic acid (2-{[5-(3-cyano-phenyl)-2-methylthiazole-4-carbonyl]-cyclopropyl-amino}-ethyl)-amide;
6-Methyl-imidazo[2,1-b]thiazole-5-carboxylic acid (2-{clyclopropyl-[5-(3,4-dimethylphenyl)-2-methyl-thiazole-4-carbonyl]-amino}-ethyl)-amide;
6-Methyl-imidazo[2,1-b]thiazole-5-carboxylic acid {2-[cyclopropyl-(2-methyl-5-*p-*tolyl-thiazole-4-carbonyl)-amino]-ethyl}amide;
6-Methyl-imidazo[2,1-b]thiazole-5-carboxylic acid {2-[cyclopropyl-(3'-methyl-biphenyl-2-carbonyl)-amino]-ethyl}-amide;
6-Methyl-imidazo[2,1-b]thiazole-5-carboxylic acid {2-[cyclopropyl-(4'-methyl-biphenyl-2-carbonyl)-amino]-ethyl}-amide;
6-Methyl-imidazo[2,1-b]thiazole-5-carboxylic acid {2-[cyclopropyl-(4'-fluoro-biphenyl-2-carbonyl)-amino]-ethyl}-amide;
1-Methyl-1*H-*indazole-3-carboxylic acid {2-[cyclopropyl-(2-cyclopropyl-5-phenyl-thiazole-4-carbonyl)-amino]-ethyl}-amide;
1-Methyl-1*H-*indazole-3-carboxylic acid {2-[cyclopropyl-(2-cyclopropyl-5-*m-*tolyl-thiazole-4-carbonyl)-amino]-ethyl}-amide;
1-Methyl-1*H-*indazole-3-carboxylic acid (2-{cyclopropyl-[2-cyclopropyl-5-(3-methoxyphenyl)-thiazole-4-carbonyl]-amino}-ethyl)-amide;
1-Methyl-1*H-*indazole-3-carboxylic acid {2-[(2-amino-5-*m-*tolyl-thiazole-4-carbonyl)-cyclopropyl-amino]-ethyl}-amide;
1-Methyl-1H indazole-3-carboxylic acid (2-{[2-amino-5-(3-fluoro-phenyl)-thiazole-4-carbonyl]-cyclopropyl-amino}-ethyl)-amide;
1-Methy)-1*H-*indazole-3-carboxylic acid (2-{[2-amino-5-(3-methoxy-phenyl)-thiazole-4-carbonyl]-cyclopropyl-amino}-ethyl)-amide;
1-Methyl-1*H-*indazole-3-carboxylic acid (2-{[2-amino-5-(4-fluoro-phenyl)-thiazole-4-carbonyl]-cyclopropyl-amino}-ethyl)-amide;
1-Methyl-1*H-*indazole-3-carboxylic acid (2-{[2-amino-5-(3-chloro-phenyl)-thiazole-4-carbonyl]-cyclopropyl-amino}-ethyl)-amide;
1-Methyl-1*H-*indazole-3-carboxylic acid {2-[(2-amino-5-phenyl-thiazole-4-carbonyl)-cyclopropyl-amino]-ethyl}-amide;
1-Methyl-1*H-*indazole-3-carboxylic acid (2-{[2-bromo-5-(3-methoxy-phenyl)-thiazole-4-carbonyl]-cyclopropyl-amino}-ethyl)amide;
1-Methyl-1*H-*indazole-3-carboxylic acid {2-[(2-bromo-5-*p-*tolyl-thiazole-4-carbonyl)-cyclopropyl-amino]-ethyl}-amide;
1-Methyl-1*H-*indazole-3-carboxylic acid (2-{cyclopropyl-[5-(2-fluoro-phenyl)-2-methylthiazole-4-carbonyl]-amino}-ethyl)-amide;
1-Methyl-1*H-*indazole-3-carboxylic acid (2-{cyclopropyl-[2-methyl-5-(3-trifluoromethylphenyl)-thiazole-4-carbonyl]-amino}-ethyl)-amide;
1-Methyl-1*H-*indazole-3-carboxylic acid (2-{cyclopropyl-[5-(3-fluoro-phenyl)-2-methylthiazole-4-carbonyl]-amino}-ethyl)-amide;
1-Methyl-1*H-*indazole-3-carboxylic acid (2-{cyclopropyl-[2-methyl-5-(4-trifluoromethylphenyl)-thiazole-4-carbonyl]-amino}-ethyl)-amide;
1-Methyl-1*H-*indazole-3-carboxylic acid (2-{[5-(4-chloro-phenyl)-2-methyl-thiazole-4-carbonyl]-cyclopropyl-amino}-ethyl)-amide;
1 -Methyl-1*H-*indazole-3-carboxylic acid (2-{cyclopropyl-[5-(3-methoxy-phenyl)-2-methyl-thiazole-4-carbonyl]-amino}-ethyl)-amide;
1-Methyl-1*H-*indazole-3-carboxylic acid (2-{[5-(3-cyano-phenyl)-2-methyl-thiazole-4-carbonyl]-cyclopropyl-amino}-ethyl)-amide;
1-Methyl-1*H-*indazole-3-carboxylic acid (2-{cyclopropyl-[5-(3,4-dimethyl-phenyl)-2-methyl-thiazole-4-carbonyl]-amino}-ethyl)-amide;
1-Methyl-1*H-*indazole-3-carboxylic acid {2-[cyclopropyl-(2-methyl-5-*p-*tolyl-thiazole-4-carbonyl)-amino]-ethyl}-amide;
1-Methyl-1*H-*indazole-3-carboxylic acid {2-[cyclopropyl-(3'-methyl-biphenyl-2-carbonyl)-amino]-ethyl}-amide;
1-Methyl-1*H-*indazole-3-carboxylic acid {2-[cyclopropyl-(4'-methy)-biphenyl-2-carbonyl)-amino]-ethyl}-amide;
1-Methyl-1*H-*indazole-3-carboxylic acid {2-[cyclopropyl-(4'-fluoro-biphenyl-2-carbonyl)-amino]-ethyl}-amide;
2-Cyclopropyl-5-phenyl-thiazole-4-carboxylic acid {2-[(benzofuran-4-carbonyl)-amino]-ethyl}-cyclopropyl-amide;
2-Cyclopropyl-5-*m-*tolyl-thiazole-4-carboxylic acid {2-[(benzofuran-4-carbonyl)-amino]-ethyl}-cyclopropyl-amide;
2-Cyclopropyl-5-(3-methoxy-phenyl)-thiazole-4-carboxylic acid {2-[(benzofuran-4-carbonyl)-amino]-ethyl}-cyclopropyl-amide;
2-Amino-5-*m-*tolyl-thiazole-4-carboxylic acid {2-[(benzofuran-4-carbonyl)-amino]-ethyl}-cyclopropyl-amide;
2-Amino-5-(3-fluoro-phenyl)-thiazole-4-carboxylic acid {2-[(benzofuran-4-carbonyl)-amino]-ethyl}-cyclopropyl-amide;
2-Amino-5-(3-methoxy-phenyl)-thiazole-4-carboxylic acid {2-[(benzofuran-4-carbonyl)-amino]-ethyl}-cyclopropyl-amide;
2-Amino-5-(4-fluoro-phenyl)-thiazole-4-carboxylic acid {2-[(benzofuran-4-carbonyl)-amino]-ethyl}-cyclopropyl-amide;
2-Amino-5-(3-chloro-phenyl)-thiazole-4-carboxylic acid {2-[(benzofuran-4-carbonyl)-amino]-ethyl}-cyclopropyl-amide;
2-Amino-5-phenyl-thiazole-4-carboxylic acid {2-[(benzofuran-4-carbonyl)-amino]-ethyl}-cyclopropyl-amide;
2-Bromo-5-(3-methoxy-phenyl)-thiazole-4-carboxylic acid {2-[(benzofuran-4-carbonyl)-amino]-ethyl}-cyclopropyl-amide;
2-Bromo-5-*p-*tolyl-thiazole-4-carboxylic acid {2-[(benzofuran-4-carbonyl)-amino]-ethyl}-cyclopropyl-amide;
5-(2-Fluoro-phenyl)-2-methyl-thiazole-4-carboxylic acid {2-[(benzofuran-4-carbonyl)-amino]-ethyl}-cyclopropyl-amide;
2-Methyl-5-(3-trifluoromethyl-phenyl)-thiazole-4-carboxylic acid {2-[(benzofuran-4-carbonyl)-amino]-ethyl}-cyclopropyl-amide;
5-(3-Fluoro-phenyl)-2-methyl-thiazole-4-carboxylic acid {2-[(benzofuran-4-carbonyl)-amino]-ethyl}-cyclopropyl-amide;
2-Methyl-5-(4-trifluoromethyl-phenyl)-thiazole-4-carboxylic acid {2-[(benzofuran-4-carbonyl)-amino]-ethyl}-cyclopropyl-amide;
5-(4-Chloro-phenyl)-2-methyl-thiazole-4-carboxylic acid {2-[(benzofuran-4-carbonyl)-amino]-ethyl}-cyclopropyl-amide;
5-(3-Methoxy-phenyl)-2-methyl-thiazole-4-carboxylic acid {2-[(benzofuran-4-carbonyl)-amino]-ethyl}-cyclopropyl-amide;
5-(3-Cyano-phenyl)-2-methyl-thiazole-4-carboxylic acid {2-[(benzofuran-4-carbonyl)-amino]-ethyl}-cyclopropyl-amide;
5-(3,4-Dimethyl-phenyl)-2-methyl-thiazole-4-carboxylic acid {2-[(benzofuran-4-carbonyl)-amino]-ethyl}-cyclopropyl-amide;
2-Methyl-5-*p-*tolyl-thiazole-4-carboxylic acid {2-[(benzofuran-4-carbonyl)-amino]-ethyl}-cyclopropyl-amide;
Benzofuran-4-carboxylic acid {2-[cyclopropyl-(3'-methyl-biphenyl-2-carbonyl)-amino]-ethyl}-amide;
Benzofuran-4-carboxylic acid {2-[cyclopropyl-(4'-methyl-biphenyl-2-carbonyl)-amino]-ethyl}-amide;
Benzofuran-4-carboxylic acid {2-[cyclopropyl-(4'-fluoro-biphenyl-2-carbonyl)-amino]-ethyl}-amide;
6-Methyl-imidazo[2,1-b]thiazole-5-carboxylic acid {2-[cyclopropylmethyl-(2-cyclopropyl-5-phenyl-thiazole-4-carbonyl)-amino]-ethyl}-amide;
6-Methyl-imidazo[2,1-b]thiazole-5-carboxylic acid {2-[cyclopropylmethyl-(2-cyclopropyl-5-*m-*tolyl-thiazole-4-carbonyl)-amino]-ethyl}-amide;
6-Methyl-imidazo[2,1-b]thiazole-5-carboxylic acid (2-{[2-cyclopropyl-5-(3-methoxyphenyl)-thiazole-4-carbonyl]-cyclopropylmethyl-amino}-ethyl)-amide;
6-Methyl-imidazo[2,1-b]thiazole-5-carboxylic acid {2-[(2-amino-5-*m-*tolyl-thiazole-4-carbonyl)-cyclopropylmethyl-amino]-ethyl}-amide;
6-Methyl-imidazo[2,1-b]thiazole-5-carboxylic acid (2-{[2-amino-5-(3-fluoro-phenyl)-thiazole-4-carbonyl]-cyclopropylmethyl-amino}-ethyl)-amide;
6-Methyl-imidazo[2,1-b]thiazole-5-carboxylic acid (2-{[2-amino-5-(3-methoxy-phenyl)-thiazole-4-carbonyl]-cyclopropylmethyl-amino}-ethyl)-amide;
6-Methyl-imidazo[2,1-b]thiazole-5-carboxylic acid (2-{[2-amino-5-(4-fluoro-phenyl)-thiazole-4-carbonyl]-cyclopropylmethyl-amino}-ethyl)-amide;
6-Methyl-imidazo[2,1-b]thiazole-5-carboxylic acid (2-{[2-amino-5-(3-chloro-phenyl)-thiazole-4-carbonyl]-cyclopropylmethyl-amino}-ethyl)-amide;
6-Methyl-imidazo[2,1-b]thiazole-5-carboxylic acid {2-[(2-amino-5-phenyl-thiazole-4-carbonyl)-cyclopropylmethyl-amino]-ethyl}-amide;
6-Methyl-imidazo[2,1-b]thiazole-5-carboxylic acid (2-{[2-bromo-5-(3-methoxy-phenyl)-thiazole-4-carbonyl]-cyclopropylmethyl-amino}-ethyl)-amide;
6-Methyl-imidazo[2,1-b]thiazole-5-carboxylic acid {2-[(2-bromo-5-*p-*tolyl-thiazole-4-carbonyl)-cyclopropylmethyl-amino]-ethyl}-amide;
6-Methyl-imidazo[2,1-b]thiazole-5-carboxylic acid (2-{cyclopropylmethyl-[5-(2-fluorophenyl)-2-methyl-thiazole-4-carbonyl]-amino}-ethyl)-amide;
6-Methyl-imidazo[2,1-b]thiazole-5-carboxylic acid (2-{cyclopropylmethyl-[2-methyl-5-(3-trifluoromethyl-phenyl)-thiazole-4carbonyl]-amino}-ethyl)-amide;
6-Methyl-imidazo[2,1-b]thiazole-5-carboxylic acid (2-{cyclopropylmethyl-[5-(3-fluorophenyl)-2-methyl-thiazole-4-carbonyl]-amino}-ethyl)-amide;
6-Methyl-imidazo[2,1-b]thiazole-5-carboxylic acid (2-{cyclopropylmethyl-[2-methyl-5-(4-trifluoromethyl-phenyl)-thiazole-4-carbonyl]-amino}ethyl)-amide;
6-Methyl-imidazo[2,1-b]thiazole-5-carboxylic acid (2-{[5-(4-chloro-phenyl)-2-methylthiazole-4-carbonyl]-cyclopropylmethyl-amino}-ethyl)-amide;
6-Methyl-imidazo[2,1-b]thiazole-5-carboxylic acid (2-{cyclopropylmethyl-[5-(3-methoxy-phenyl)-2-methyl-thiazole-4-carbonyl]-amino}-ethyl)-amide;
6-Methyl-imidazo[2,1-b]thiazole-5-carboxylic acid (2-{[5-(3-cyano-phenyl)-2-methylthiazole-4-carbonyl]-cyclopropylmethyl-amino}-ethyl)-amide;
6-Methyl-imidazo[2,1-b]thiazole-5-carboxylic acid (2-{cyclopropylmethyl-[5-(3,4-dimethyl-phenyl)-2-methyl-thiazole-4-carbonyl]-amino}-ethyl)-amide;
6-Methyl-imidazo[2,1-b]thiazole-5-carboxylic acid {2-[cyclopropylmethyl-(2-methyl-5-*p-*tolyl-thiazole-4-carbonyl)-amino]-ethyl}-amide;
6-Methyl-imidazo[2,1-b]thiazole-5-carboxylic acid {2-[cyclopropylmethyl-(3'-methylbiphenyl-2-carbonyl)-amino]-ethyl}-amide;
6-Methyl-imidazo[2,1-b]thiazole-5-carboxylic acid {2-[cyclopropylmethyl-(4'-methylbiphenyl-2-carbonyl)-amino]-ethyl}-amide;
1-Methyl-1*H-*indazole-3-carboxylic acid {2-[cyclopropylmethyl-(2-cyclopropyl-5-phenyl-thiazole-4-carbonyl)-amino]-ethyl}-amide;
1-Methyl-1*H-*indazole-3-carboxylic acid {2-[cyclopropylmethyl-(2-cyclopropyl-5-*m-*tolyl-thiazole-4-carbonyl)-amino]-ethyl}-amide;
1-Methyl-1*H-*indazole-3-carboxylic acid (2-{[2-cyclopropyl-5-(3-methoxy-phenyl)-thiazole-4-carbonyl]-cyclopropylmethyl-amino}-ethyl)-amide;
1-Methyl-1*H-*indazole-3-carboxylic acid {2-[(2-amino-5-*m-*tolyl-thiazole-4-carbonyl)-cyclopropylmethyl-amino]-ethyl}-amide;
1-Methyl-1*H-*indazole-3-carboxylic acid (2-{[2-amino-5-(3-fluoro-phenyl)-thiazole-4-carbonyl]-cyclopropylmethyl-amino}-ethyl)-amide;
1-Methyl-1*H-*indazole-3-carboxylic acid (2-{[2-amino-5-(3-methoxy-phenyl)-thiazole-4-carbonyl]-cyclopropylmethyl-amino}-ethyl)-amide;
1-Methyl-1*H-*indazole-3-carboxylic acid (2-{[2-amino-5-(4-fluoro-phenyl)-thiazole-4-carbonyl]-cyclopropylmethyl-amino}-ethyl)-amide;
1-Methyl-1*H-*indazole-3-carboxylic acid (2-{[2-amino-5-(3-chloro-phenyl)-thiazole-4-carbonyl]-cyclopropylmethyl-amino}-ethyl)-amide;
1-Methyl-1*H-*indazole-3-carboxylic acid {2-[(2-amino-5-phenyl-thiazole-4-carbonyl)-cyclopropylmethyl-amino]-ethyl}-amide;
1-Methyl-1*H-*indazole-3-carboxylic acid (2-{[2-bromo-5-(3-methoxy-phenyl)-thiazole-4-carbonyl]-cyclopropylmethyl-amino}-ethyl)-amide;
1-Methyl-1*H-*indazole-3-carboxylic acid {2-[(2-bromo-5-*p-*tolyl-thiazole-4-carbonyl)-cyclopropylmethyl-amino]-ethyl}-amide;
1-Methyl-1*H-*indazole-3-carboxylic acid (2-{cyclopropylmethyl-[5-(2-fluoro-phenyl)-2-methyl-thiazole-4-carbonyl]-amino}-ethyl)-amide;
1-Methyl-1*H-*indazole-3-carboxylic acid (2-{cyclopropylmethyl-[2-methyl-5-(3-trifluoromethyl-phenyl)-thiazole-4-carbonyl]-amino}-ethyl)-amide;
1-Methyl-1*H-*indazole-3-carboxylic acid (2-{cyclopropylmethyl-[5-(3-fluoro-phenyl)-2-methyl-thiazole-4-carbonyl]-amino}-ethyl)-amide;
1-Methyl-1*H-*indazole-3-carboxylic acid (2-{cyclopropylmethyl-[2-methyl-5-(4-trifluoromethyl-phenyl)-thiazole-4-carbonyl]-amino}-ethyl)-amide;
1-Methyl-1*H-*indazole-3-carboxylic acid (2-{[5-(4-chloro-phenyl)-2-methyl-thiazole-4-carbonyl]-cyclopropylmethyl-amino}-ethyl)-amide;
1-Methyl-1*H-*indazole-3-carboxylic acid (2-{cyclopropylmethyl-[5-(3-methoxy-phenyl)-2-methyl-thiazole-4-carbonyl]-amino}-ethyl)-amide;
1-Methy)-1*H-*indazole-3-carboxylic acid (2-{[5-(3-cyano-phenyl)-2-methyl-thiazole-4-carbonyl]-cyclopropylmethyl-amino}-ethyl)-amide;
1-Methyl-1*H-*indazole-3-carboxylic acid (2-{cyclopropylmethyl-[5-(3,4-dimethylphenyl)-2-methyl-thiazole-4-carbonyl]-amino}-ethyl)-amide;
1-Methyl-1*H-*indazole-3-carboxylic acid {2-[cyclopropylmethyl-(2-methyl-5-*p-*tolyl-thiazole-4-carbonyl)-amino]-ethyl}-amide;
1-Methyl-1*H-*indazole-3-carboxylic acid {2-[cyclopropylmethyl-(3'-methyl-biphenyl-2-carbonyl)-amino]-ethyl}-amide;
1-Methyl-1*H-*indazole-3-carboxylic acid {2-[cyclopropylmethyl-(4'-methyl-biphenyl-2-carbonyl)-amino]-ethyl}-amide;
1-Methyl-1*H-*indazole-3-carboxylic acid {2-[cyclopropylmethyl-(4'-fluoro-biphenyl-2-carbonyl)-amino]-ethyl}-amide;
2-Cyclopropyl-5-phenyl-thiazole-4-carboxylic acid cyclopropyl-{2-[(2,3-dihydro-benzo[1,4]dioxine-5-carbonyl)-amino]-ethyl}-amide;
2-Cyclopropyl-5-*m-*tolyl-thiazole-4-carboxylic acid cyclopropyl-{2-[(2,3-dihydro-benzo[1,4]dioxine-5-carbonyl)-amino]-ethyl}-amide;
2-Cyclopropyl-5-(3-methoxy-phenyl)-thiazole-4-carboxylic acid cyclopropyl-{2-[(2,3-dihydro-benzo[1,4]dioxine-5-carbonyl)-amino]-ethyl}-amide;
2-Amino-5-*m-*tolyl-thiazole-4-carboxylic acid cyclopropyl-{2-[(2,3-dihydro-benzo[1,4]dioxine-5-carbonyl)-amino]-ethyl}-amide;
2-Amino-5-(3-fluoro-phenyl)-thiazole-4-carboxylic acid cyclopropyl-{2-[(2,3-dihydro-benzo[1,4]dioxine-5-carbonyl)-amino]-ethyl}-amide;
2-Amino-5-(3-methoxy-phenyl)-thiazole-4-carboxylic acid cyclopropyl-{2-[(2,3-dihydro-benzo[1,4]dioxine-5-carbonyl)-amino]-ethyl}-amide;
2-Amino-5-(4-fluoro-phenyl)-thiazole-4-carboxylic acid cyclopropyl-{2-[(2,3-dihydro-benzo[1,4]dioxine-5-carbonyl)-amino]-ethyl}-amide;
2-Amino-5-(3-chloro-phenyl)-thiazole-4-carboxylic acid cyclopropyl-{2-[(2,3-dihydro-benzo[1,4]dioxine-5-carbonyl)-amino]-ethyl}-amide;
2-Amino-5-phenyl-thiazole-4-carboxylic acid cyclopropyl-{2-[(2,3-dihydro-benzo[1,4]dioxine-5-carbonyl)-amino]-ethyl}-amide;
2-Bromo-5-(3-methoxy-phenyl)-thiazole-4-carboxylic acid cyclopropyl-{2-[(2,3-dihydro-benzo[1,4]dioxine-5-carbonyl)-amino]-ethyl}-amide;
2-Bromo-5-*p-*tolyl-thiazole-4-carboxylic acid cyclopropyl-{2-[(2,3-dihydro-benzo[1,4]dioxine-5-carbonyl)-amino]-ethyl}-amide;
5-(2-Fluoro-phenyl)-2-methyl-thiazole-4-carboxylic acid cyclopropyl-{2-[(2,3-dihydro-benzo[1,4]dioxine-5-carbonyl)-amino]-ethyl}-amide;
2-Methyl-5-(3-trifluoromethyl-phenyl)-thiazole-4-carboxylic acid cyclopropyl-{2-[(2,3-dihydro-benzo[1,4]dioxine-5-carbonyl)-amino]-ethyl}-amide;
5-(3-Fluoro-phenyl)-2-methyl-thiazole-4-carboxylic acid cyclopropyl-{2-[(2,3-dihydro-benzo[1,4]dioxine-5-carbonyl)-amino]-ethyl}-amide;
5-(4-Chloro-phenyl)-2-methyl-thiazole-4-carboxylic acid cyclopropyl-{2-[(2,3-dihydro-benzo[1,4]dioxine-5-carbonyl)-amino]-ethyl}-amide;
5-(3-Methoxy-phenyl)-2-methyl-thiazole-4-carboxylic acid cyclopropyl-{2-[(2,3-dihydro-benzo[1,4]dioxine-5-carbonyl)-amino]-ethyl}-amide;
5-(3,4-Dimethyl-phenyl)-2-methyl-thiazole-4-carboxylic acid cyclopropyl-{2-[(2,3-dihydro-benzo[1,4]dioxine-5-carbonyl)-amino]-ethyl}-amide;
2-Methyl-5-*p-*tolyl-thiazole-4-carboxylic acid cyclopropyl-{2-[(2,3-dihydro-benzo[1,4]dioxine-5-carbonyl)-amino]-ethyl}-amide;
2,3-Dihydro-benzo[1,4]dioxine-5-carboxylic acid {2-[cyclopropyl-(3'-methyl-biphenyl-2-carbonyl)-amino]-ethyl}-amide;
2,3-Dihydro-benzo[1,4]dioxine-5-carboxylic acid {2-[cyclopropyl-(4'-methyl-biphenyl-2-carbonyl)-amino]-ethyl}-amide;
2,3-Dihydro-benzo[1,4]dioxine-5-carboxylic acid {2-[cyclopropyl-(4'-fluoro-biphenyl-2-carbonyl)-amino]-ethyl}-amide;
2-Cyclopropyl-5-phenyl-thiazole-4-carboxylic acid cyclopropylmethyl-{2-[(2,3-dihydro-benzo[1,4]dioxine-5-carbonyl)-amino]-ethyl}-amide;
2-Cyclopropyl-5-*m*-tolyl-thiazole-4-carboxylic acid cyclopropylmethyl-{2-[(2,3-dihydro-benzo[1,4]dioxine-5-carbonyl)-amino]-ethyl}-amide;
2-Cyclopropyl-5-(3-methoxy-phenyl)-thiazole-4-carboxylic acid cyclopropylmethyl-{2-[(2,3-dihydro-benzo[1,4]dioxine-5-carbonyl)-amino]-ethyl}-amide;
2-Amino-5-*m*-tolyl-thiazole-4-carboxylic acid cyclopropylmethyl-{2-[(2,3-dihydro-benzo[1,4]dioxine-5-carbonyl)-amino]-ethyl}-amide;
2-Amino-5-(3-fluoro-phenyl)-thiazole-4-carboxylic acid cyclopropylmethyl-{2-[(2,3-dihydro-benzo[1,4]dioxine-5-carbonyl)-amino]-ethyl}-amide;
2-Amino-5-(3-methoxy-phenyl)-thiazole-4-carboxylic acid cyclopropylmethyl-{2-[(2,3-dihydro-benzo[1,4]dioxine-5-carbonyl)-amino]-ethyl}-amide;
2-Amino-5-phenyl-thiazole-4-carboxylic acid cyclopropylmethyl-{2-[(2,3-dihydro-benzo[1,4]dioxine-5-carbonyl)-amino]-ethyl}-amide;
2-Bromo-5-(3-methoxy-phenyl)-thiazole-4-carboxylic acid cyclopropylmethyl-{2-[(2,3-dihydro-benzo[1,4]dioxine-5-carbonyl)-amino]-ethyl}-amide;
2-Bromo-5-*p*-tolyl-thiazole-4-carboxylic acid cyclopropylmethyl-{2-[(2,3-dihydro-benzo[1,4]dioxine-5-carbonyl)-amino]-ethyl}-amide;
5-(2-Fluoro-phenyl)-2-methyl-thiazole-4-carboxylic acid cyclopropylmethyl-{2-[(2,3-dihydro-benzo[1,4]dioxine-5-carbonyl)-amino]-ethyl}-amide;
2-Methyl-5-(3-trifluoromethyl-phenyl)-thiazole-4-carboxylic acid cyclopropylmethyl-{2-[(2,3-dihydro-benzo[1,4]dioxine-5-carbonyl)-amino]-ethyl}-amide;
5-(3-Fluoro-phenyl)-2-methyl-thiazole-4-carboxylic acid cyclopropylmethyl-{2-[(2,3-dihydro-benzo[1,4]dioxine-5-carbonyl)-amino]-ethyl}-amide;
5-(3-Methoxy-phenyl)-2-methyl-thiazole-4-carboxylic acid cyclopropylmethyl-{2-[(2,3-dihydro-benzo[1,4]dioxine-5-carbonyl)-amino]-ethyl}-amide;
5-(3-Cyano-phenyl)-2-methyl-thiazole-4-carboxylic acid cyclopropylmethyl-{2-[(2,3-dihydro-benzo[1,4]dioxine-5-carbonyl)-amino]-ethyl}-amide;
5-(3,4-Dimethyl-phenyl)-2-methyl-thiazole-4-carboxylic acid cyclopropylmethyl-{2-[(2,3-dihydro-benzo[1,4]dioxine-5-carbonyl)-amino]-ethyl}-amide;
2-Methyl-5-*p*-tolyl-thiazole-4-carboxylic acid cyclopropylmethyl-{2-[(2,3-dihydro-benzo[1,4]dioxine-5-carbonyl)-amino]-ethyl}-amide;
2,3-Dihydro-benzo[1,4]dioxine-5-carboxylic acid {2-[cyclopropylmethyl-(3'-methylbiphenyl-2-carbonyl)-amino]-ethyl}-amide;
2,3-Dihydro-benzo[1,4]dioxine-5-carboxylic acid {2-[cyclopropylmethyl-(4'-methylbiphenyl-2-carbonyl)-amino]-ethyl}-amide;
2,3-Dihydro-benzo[1,4]dioxine-5-carboxylic acid {2-[cyclopropylmethyl-(4'-fluoro-biphenyl-2-carbonyl)-amino]-ethyl}-amide;
2-Cyclopropyl-5-phenyl-thiazole-4-carboxylic acid {2-[(benzofuran-4-carbonyl)-amino]-ethyl}-cyclopropylmethyl-amide;
2-Cyclopropyl-5-*m*-tolyl-thiazole-4-carboxylic acid {2-[(benzofuran-4-carbonyl)-amino]-ethyl}-cyclopropylmethyl-amide;
2-Cyclopropyl-5-(3-methoxy-phenyl)-thiazole-4-carboxylic acid {2-[(benzofuran-4-carbonyl)-amino]-ethyl}-cyclopropylmethyl-amide;
2-Amino-5-*m*-tolyl-thiazole-4-carboxylic acid {2-[(benzofuran-4-carbonyl)-amino]-ethyl}-cyclopropylmethyl-amide;
2-Amino-5-(3-fluoro-phenyl)-thiazole-4-carboxylic acid {2-[(benzofuran-4-carbonyl)-amino]-ethyl}-cyclopropylmethyl-amide;
2-Amino-5-(3-methoxy-phenyl)-thiazole-4-carboxylic acid {2-[(benzofuran-4-carbonyl)-amino]-ethyl}-cyclopropylmethyl-amide;
2-Amino-5-(4-fluoro-phenyl)-thiazole-4-carboxylic acid {2-[(benzofuran-4-carbonyl)-amino]-ethyl}-cyclopropylmethyl-amide;
2-Amino-5-(3-chloro-phenyl)-thiazole-4-carboxylic acid {2-[(benzofuran-4-carbonyl)-amino]-ethyl}-cyclopropylmethyl-amide;
2-Amino-5-phenyl-thiazole-4-carboxylic acid {2-[(benzofuran-4-carbonyl)-amino]-ethyl}-cyclopropylmethyl-amide;
2-Bromo-5-(3-methoxy-phenyl)-thiazole-4-carboxylic acid {2-[(benzofuran-4-carbonyl)-amino]-ethyl}-cyclopropylmethyl-amide;
2-Bromo-5-*p*-tolyl-thiazole-4-carboxylic acid {2-[(benzofuran-4-carbonyl)-amino]-ethyl}-cyclopropylmethyl-amide;
5-(2-Fluoro-phenyl)-2-methyl-thiazole-4-carboxylic acid {2-[(benzofuran-4-carbonyl)-amino]-ethyl}-cyclopropylmethyl-amide;
2-Methyl-5-(3-trifluoromethyl-phenyl)-thiazole-4-carboxylic acid {2-[(benzofuran-4-carbonyl)-amino]-ethyl}-cyclopropylmethyl-amide;
5-(3-Fluoro-phenyl)-2-methyl-thiazole-4-carboxylic acid {2-[(benzofuran-4-carbonyl)-amino]-ethyl}-cyclopropylmethyl-amide;
2-Methyl-5-(4-trifluoromethyl-phenyl)-thiazole-4-carboxylic acid {2-[(benzofuran-4-carbonyl)-amino]-ethyl}-cyclopropylmethyl-amide;
5-(4-Chloro-phenyl)-2-methyl-thiazole-4-carboxylic acid {2-[(benzofuran-4-carbonyl)-amino]-ethyl}-cyclopropylmethyl-amide;
5-(3-Methoxy-phenyl)-2-methyl-thiazole-4-carboxylic acid {2-[(benzofuran-4-carbonyl)-amino]-ethyl}-cyclopropylmethyl-amide;
5-(3-Cyano-phenyl)-2-methyl-thiazole-4-carboxylic acid {2-[(benzofuran-4-carbonyl)-amino]-ethyl}-cyclopropylmethyl-amide;
5-(3,4-Dimethyl-phenyl)-2-methyl-thiazole-4-carboxylic acid {2-[(benzofuran-4-carbonyl)-amino]-ethyl}-cyclopropylmethyl-amide;
2-Methyl-5-*p*-tolyl-thiazole-4-carboxylic acid {2-[(benzofuran-4-carbonyl)-amino]-ethyl}-cyclopropylmethyl-amide;
Benzofuran-4-carboxylic acid {2-[cyclopropylmethyl-(3'-methyl-biphenyl-2-carbonyl)-amino]-ethyl}-amide;
Benzofuran-4-carboxylic acid {2-[cyclopropylmethyl-(4'-methyl-biphenyl-2-carbonyl)-amino]-ethyl}-amide; and
Benzofuran-4-carboxylic acid {2-[cyclopropylmethyl-(4'-fluoro-biphenyl-2-carbonyl)-amino]-ethyl}-amide;
or a salt of such a compound.

9. A pharmaceutical composition containing, as active principle, a compound of formula (I) according to any one of claims 1 to 8, or a pharmaceutically acceptable salt thereof, and at least one therapeutically inert excipient.

10. A compound of any one of claims 1 to 8, or a pharmaceutically acceptable salt thereof, for use as medicament.

11. A compound according to any of claims 1 to 8, or a pharmaceutically acceptable salt thereof, for the the prevention or treatment of diseases selected from the group consisting of all types of sleep disorders, of stress-related syndromes, of psychoactive substance use and abuse, of cognitive dysfunctions in the healthy population and in psychiatric and neurologic disorders, of eating or drinking disorders.

12. Use of a compound according to any of claims 1 to 8, or of a pharmaceutically acceptable salt thereof, for the preparation of a medicament for the prevention or treatment of diseases selected from the group consisting of all types of sleep disorders, of stress-related syndromes, of psychoactive substance use and abuse, of cognitive dysfunctions in the healthy population and in psychiatric and neurologic disorders, of eating or drinking disorders.

## Patentansprüche

1. Verbindung der Formel (I) 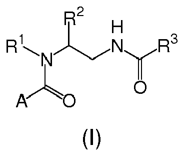 wobei
R¹ Cyclopropyl oder Cyclopropyl-methyl repräsentiert;
R² Wasserstoff, (C₁₋₄)Alkyl, Cyclopropyl oder Phenyl-(C₁₋₄)alkyl repräsentiert, wobei das Phenyl unsubstituiert oder mono-, di- oder tri-substituiert ist, wobei die Substituenten unabhängig ausgewählt sind aus der Gruppe bestehend aus (C₁₋₄)Alkyl, (C₁₋₄)Alkoxy, Trifluormethyl und Halogen;
R³ Heterocyclyl repräsentiert, wobei das genannte Heterocyclyl ein 8- bis 10-gliedriger bicyclischer Ring ist, der 1, 2 oder 3 Heteroatome enthält, die jeweils unabhängig ausgewählt sind aus Sauerstoff, Stickstoff und Schwefel, wobei wenigstens ein Ring des genannten bicyclischen Ringsystems aromatisch ist und die Bindung, mit der das Heterocyclyl am Rest des Moleküls angefügt ist, auf einem aromatischen Kohlenstoffatom des genannten bicyclischen Ringsystems in Alpha-Position zu einem Brückenkopfatom positioniert ist; wobei das genannte Heterocyclyl unsubstituiert, mono-, di- oder tri-substituiert ist, wobei die Substituenten unabhängig ausgewählt sind aus der Gruppe bestehend aus (C₁₋₄)Alkyl, (C₁₋₄)Alkoxy, Halogen und Trifluormethyl;
A Folgendes repräsentiert 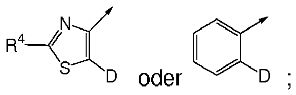 R⁴ (C₁₋₄)Alkyl, Brom, Cyclopropyl oder -NR⁵R⁶ repräsentiert;
R⁵ Wasserstoff oder (C₁₋₄)Alkyl repräsentiert;
R⁶ Wasserstoff oder (C₁₋₄)Alkyl repräsentiert; und
D Phenyl repräsentiert, das unsubstituiert, mono-, di- oder tri-substituiert ist, wobei die Substituenten unabhängig ausgewählt sind aus der Gruppe bestehend aus (C₁₋₄)Alkyl, (C₁₋₄)Alkoxy, Trifluormethyl, Trifluormethoxy, Cyano und Halogen;
oder ein Salz einer solchen Verbindung.

2. Verbindung der Formel (I) nach Anspruch 1, wobei A Folgendes repräsentiert 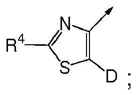 oder ein Salz einer solchen Verbindung.

3. Verbindung der Formel (I) nach Anspruch 1 oder 2, wobei R⁴ Methyl, -NH₂, Brom oder Cyclopropyl repräsentiert;
oder ein Salz einer solchen Verbindung.

4. Verbindung der Formel (I) nach einem der Ansprüche 1 bis 3, wobei D Phenyl repräsentiert, das unsubstituiert, mono- oder di-substituiert ist, wobei die Substituenten unabhängig ausgewählt sind aus der Gruppe bestehend aus (C₁₋₄)Alkyl, (C₁₋₄)Alkoxy, Trifluormethyl, Cyano und Halogen;
oder ein Salz einer solchen Verbindung.

5. Verbindung nach einem der Ansprüche 1 bis 4, wobei R² Wasserstoff, Methyl, Cyclopropyl oder unsubstituiertes Phenyl-(C₁₋₂)alkyl repräsentiert;
oder ein Salz einer solchen Verbindung.

6. Verbindung nach einem der Ansprüche 1 bis 5, wobei R³ eine Gruppe repräsentiert, die ausgewählt ist aus Chinolin-8-yl, Isochinolin-1-yl, Indol-3-yl, Indol-4-yl, Indol-7-yl, Indazol-3-yl, Indazol-4-yl, Indazol-7-yl, Benzofuran-4-yl, Benzofuran-7-yl, Benzisoxazol-3-yl, Benzisoxazol-4-yl, Benzisoxazol-7-yl, Benzoxazol-4-yl, Benzoxazol-7-yl, Benzoxadiazol-4-yl, Benzoxadiazol-7-yl, Benzothiophen-3-yl, Benzothiophen-4-yl, Benzothiophen-7-yl, Benzthiazol-4-yl, Benzthiazol-7-yl, Benzoisothiazol-3-yl, Benzoisothiazol-4-yl, Benzoisothiazol-7-yl, Benzothiadiazol-4-yl, Benzothiadiazol-7-yl, Benzimidazol-4-yl, Benzimidazol-7-yl, Imidazo[2,1-b]thiazol-3-yl, Imidazo[2,1-b]thiazol-5-yl, Pyrazolo[1,5-a]pyridin-3-yl, Imidazo[1,2-a]pyridin-3-yl, 2,3-Dihydro-thieno[3,4-b][1,4]dioxin-5-yl, Benzo[1,3]dioxol-4-yl, 2,3-Dihydrobenzofuran-4-yl, 2,3-Dihydro-benzofuran-7-yl, 4H-Benzo[1,3]dioxin-8-yl, 4H-Benzo[1,3]dioxin-5-yl und 2,3-Dihydro-benzo[1,4]dioxin-5-yl; wobei die genannte Gruppe unsubstituiert, mono- oder di-substituiert ist, wobei die Substituenten unabhängig ausgewählt sind aus der Gruppe bestehend aus Methyl, Methoxy, Halogen und Trifluormethyl;
oder ein Salz einer solchen Verbindung.

7. Verbindung nach einem der Ansprüche 1 bis 5, wobei R³ Folgendes repräsentiert 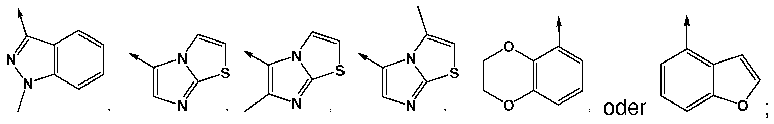 oder ein Salz einer solchen Verbindung.

8. Verbindung nach Anspruch 1, die ausgewählt ist aus der Gruppe bestehend aus:
2-Methyl-5-phenyl-thiazol-4-carbonsäure {2-[(benzofuran-4-carbonyl)-amino]-ethyl}-cyclopropylmethyl-amid;
6-Methyl-imidazo[2,1-b]thiazol-5-carbonsäure {2-[cyclopropylmethyl-(2-methyl-5-phenyl-thiazol-4-carbonyl)-amino]-ethyl}-amid;
2-Methyl-5-phenyl-thiazol-4-carbonsäurecyclopropylmethyl-{2-[(2,3-dihydro-benzo[1,4]dioxin-5-carbonyl)-amino]-ethyl}-amid;
2-Methyl-5-phenyl-thiazol-4-carbonsäure {2-[(benzofuran-4-carbonyl)-amino]-ethyl}-cyclopropyl-amid;
6-Methyl-imidazo[2,1-b]thiazol-5-carbonsäure {2-[cyclopropyl-(2-methyl-5-phenyl-thiazol-4-carbonyl)-amino]-ethyl}-amid;
2-Methyl-5-phenyl-thiazol-4-carbonsäurecyclopropyl-{2-[(2,3-dihydro-benzo[1,4]dioxin-5-carbonyl)-amino]-ethyl}-amid;
6-Methyl-imidazo[2,1-b]thiazol-5-carbonsäure {2-[cyclopropyl-(2-cyclopropyl-5-phenyl-thiazol-4-carbonyl)-amino]-ethyl]-amid;
6-Methyl-imidazo[2,1-b]thiazol-5-carbonsäure {2-[cyclopropyl-(2-cyclopropyl-5-*m-*tolyl-thiazol-4-carbonyl)-amino]-ethyl}-amid;
6-Methyl-imidazo[2,1-b]thiazol-5-carbonsäure (2-{cyclopropyl-[2-cyclopropyl-5-(3-methoxy-phenyl)-thiazol-4-carbonyl]-amino}-ethyl)-amid;
6-Methyl-imidazo[2,1-b]thiazol-5-carbonsäure {2-[(2-amino-5-*m*-tolyl-thiazol-4-carbonyl)-cyclopropyl-amino]-ethyl}-amid;
6-Methyl-imidazo[2,1-b]thiazol-5-carbonsäure (2-{[2-amino-5-(3-fluor-phenyl)-thiazol-4-carbonyl]-cyclopropyl-amino}-ethyl)-amid;
6-Methyl-imidazo[2,1-b]thiazol-5-carbonsäure (2-{[2-amino-5-(3-methoxy-phenyl)-thiazol-4-carbonyl]-cyclopropyl-amino}-ethyl)-amid;
6-Methyl-imidazo[2,1-b]thiazol-5-carbonsäure (2-{[2-amino-5-(4-fluor-phenyl)-thiazol-4-carbonyl]-cyclopropyl-amino}-ethyl)-amid;
6-Methyl-imidazo[2,1-b]thiazol-5-carbonsäure (2-{[2-amino-5-(3-chlor-phenyl)-thiazol-4-carbonyl]-cyclopropyl-amino}-ethyl)-amid;
6-Methyl-imidazo[2,1-b]thiazol-5-carbonsäure {2-[(2-amino-5-phenyl-thiazol-4-carbonyl)-cyclopropyl-amino]-ethyl}-amid;
6-Methyl-imidazo[2,1-b]thiazol-5-carbonsäure (2-{[2-brom-5-(3-methoxy-phenyl)-thiazol-4-carbonyl]-cyclopropyl-amino}-ethyl)-amid;
6-Methyl-imidazo[2,1-b]thiazol-5-carbonsäure {2-[(2-brom-5-*p*-tolyl-thiazol-4-carbonyl)-cyclopropyl-amino]-ethyl}-amid;
6-Methyl-imidazo[2,1-b]thiazol-5-carbonsäure (2-{cyclopropyl-[5-(2-fluor-phenyl)-2-methyl-thiazol-4-carbonyl]-amino}-ethyl)-amid;
6-Methyl-imidazo[2,1-b]thiazol-5-carbonsäure (2-{cyclopropyl-[2-methyl-5-(3-trifluormethyl-phenyl)-thiazol-4-carbonyl]-amino}-ethyl)-amid;
6-Methyl-imidazo[2,1-b]thiazol-5-carbonsäure (2-{cyclopropyl-[5-(3-fluor-phenyl)-2-methyl-thiazol-4-carbonyl]-amino}-ethyl)-amid;
6-Methyl-imidazo[2,1-b]thiazol-5-carbonsäure (2-{cyclopropyl-[2-methyl-5-(4-trifluormethyl-phenyl)-thiazol-4-carbonyl]-amino}-ethyl)-amid;
6-Methyl-imidazo[2,1-b]thiazol-5-carbonsäure (2-{[5-(4-chlor-phenyl)-2-methyl-thiazol-4-carbonyl]-cyclopropyl-amino}-ethyl)-amid;
6-Methyl-imidazo[2,1-b]thiazol-5-carbonsäure (2-{cyclopropyl-[5-(3-methoxy-phenyl)-2-methyl-thiazol-4-carbonyl]-amino}-ethyl)-amid;
6-Methyl-imidazo[2,1-b]thiazol-5-carbonsäure (2-{[5-(3-cyano-phenyl)-2-methyl-thiazol-4-carbonyl]-cyclopropyl-amino}-ethyl)-amid;
6-Methyl-imidazo[2,1-b]thiazol-5-carbonsäure (2-{cyclopropyl-[5-(3,4-dimethylphenyl)-2-methyl-thiazol-4-carbonyl]-amino}-ethyl)-amid;
6-Methyl-imidazo[2,1-b]thiazol-5-carbonsäure {2-[cyclopropyl-(2-methyl-5-*p*-tolyl-thiazol-4-carbonyl)-amino]-ethyl}-amid;
6-Methyl-imidazo[2,1-b]thiazol-5-carbonsäure {2-[cyclopropyl-(3'-methyl-biphenyl-2-carbonyl)-amino]-ethyl}-amid;
6-Methyl-imidazo[2,1-b]thiazol-5-carbonsäure {2-[cyclopropyl-(4'-methyl-biphenyl-2-carbonyl)-amino]-ethyl}-amid;
6-Methyl-imidazo[2,1-b]thiazol-5-carbonsäure {2-[cyclopropyl-(4'-fluor-biphenyl-2-carbonyl)-amino]-ethyl}-amid;
1-Methyl-1*H*-indazol-3-carbonsäure {2-[cyclopropyl-(2-cyclopropyl-5-phenyl-thiazol-4-carbonyl)-amino]-ethyl}-amid;
1-Methyl-1*H*-indazol-3-carbonsäure {2-[cyclopropyl-(2-cyclopropyl-5-*m*-tolyl-thiazol-4-carbonyl)-amino]-ethyl}-amid;
1-Methyl-1*H*-indazol-3-carbonsäure (2-{cyclopropyl-[2-cyclopropyl-5-(3-methoxyphenyl)-thiazol-4-carbonyl]-amino}-ethyl)-amid;
1-Methyl-1*H*-indazol-3-carbonsäure {2-[(2-amino-5-*m*-tolyl-thiazol-4-carbonyl)-cyclopropyl-amino]-ethyl}-amid;
1-Methyl-1*H*-indazol-3-carbonsäure (2-{[2-amino-5-(3-fluor-phenyl)-thiazol-4-carbonyl]-cyclopropyl-amino}-ethyl)-amid;
1-Methyl-1*H*-indazol-3-carbonsäure (2-{[2-amino-5-(3-methoxy-phenyl)-thiazol-4-carbonyl]-cyclopropyl-amino}-ethyl)-amid;
1-Methyl-1*H*-indazol-3-carbonsäure (2-{[2-amino-5-(4-fluor-phenyl)-thiazol-4-carbonyl]-cyclopropyl-amino}-ethyl)-amid;
1-Methyl-1*H*-indazol-3-carbonsäure (2-{[2-amino-5-(3-chlor-phenyl)-thiazol-4-carbonyl]-cyclopropyl-amino}-ethyl)-amid;
1-Methyl-1*H*-indazol-3-carbonsäure {2-[(2-amino-5-phenyl-thiazol-4-carbonyl)-cyclopropyl-amino]-ethyl}-amid;
1-Methyl-1*H*-indazol-3-carbonsäure (2-{[2-brom-5-(3-methoxy-phenyl)-thiazol-4-carbonyl]-cyclopropyl-amino}-ethyl)-amid;
1-Methyl-1*H*-indazol-3-carbonsäure {2-[(2-brom-5-*p*-tolyl-thiazol-4-carbonyl)-cyclopropyl-amino]-ethyl}-amid;
1-Methyl-1*H*-indazol-3-carbonsäure (2-{cyclopropyl-[5-(2-fluor-phenyl)-2-methyl-thiazol-4-carbonyl]-amino}-ethyl)-amid;
1-Methyl-1*H*-indazol-3-carbonsäure (2-{cyclopropyl-[2-methyl-5-(3-trifluormethylphenyl)-thiazol-4-carbonyl]-amino}-ethyl)-amid;
1-Methyl-1*H*-indazol-3-carbonsäure (2-{cyclopropyl-[5-(3-fluor-phenyl)-2-methyl-thiazol-4-carbonyl]-amino}-ethyl)-amid;
1-Methyl-1*H*-indazol-3-carbonsäure (2-{cyclopropyl-[2-methyl-5-(4-trifluormethylphenyl)-thiazol-4-carbonyl]-amino}-ethyl)-amid;
1-Methyl-1*H*-indazol-3-carbonsäure (2-{[5-(4-chlor-phenyl)-2-methyl-thiazol-4-carbonyl]-cyclopropyl-amino}-ethyl)-amid;
1-Methyl-1*H*-indazol-3-carbonsäure (2-{cyclopropyl-[5-(3-methoxy-phenyl)-2-methyl-thiazol-4-carbonyl]-amino}-ethyl)-amid;
1-Methyl-1*H*-indazol-3-carbonsäure (2-{[5-(3-cyano-phenyl)-2-methyl-thiazol-4-carbonyl]-cyclopropyl-amino}-ethyl)-amid;
1-Methyl-1*H*-indazol-3-carbonsäure (2-{cyclopropyl-[5-(3,4-dimethyl-phenyl)-2-methyl-thiazol-4-carbonyl]-amino}-ethyl)-amid;
1-Methyl-1*H*-indazol-3-carbonsäure {2-[cyclopropyl-(2-methyl-5-*p*-tolyl-thiazol-4-carbonyl)-amino]-ethyl}-amid;
1-Methyl-1*H*-indazol-3-carbonsäure {2-[cyclopropyl-(3'-methyl-biphenyl-2-carbonyl)-amino]-ethyl}-amid;
1-Methyl-1*H*-indazol-3-carbonsäure {2-[cyclopropyl-(4'-methyl-biphenyl-2-carbonyl)-amino]-ethyl}-amid;
1-Methyl-1*H*-indazol-3-carbonsäure {2-[cyclopropyl-(4'-fluor-biphenyl-2-carbonyl)-amino]-ethyl}-amid;
2-Cyclopropyl-5-phenyl-thiazol-4-carbonsäure {2-[(benzofuran-4-carbonyl)-amino]-ethyl}-cyclopropyl-amid;
2-Cyclopropyl-5-*m*-tolyl-thiazol-4-carbonsäure {2-[(benzofuran-4-carbonyl)-amino]-ethyl}-cyclopropyl-amid;
2-Cyclopropyl-5-(3-methoxy-phenyl)-thiazol-4-carbonsäure {2-[(benzofuran-4-carbonyl)-amino]-ethyl}-cyclopropyl-amid;
2-Amino-5-*m*-tolyl-thiazol-4-carbonsäure {2-[(benzofuran-4-carbonyl)-amino]-ethyl}-cyclopropyl-amid;
2-Amino-5-(3-fluor-phenyl)-thiazol-4-carbonsäure {2-[(benzofuran-4-carbonyl)-amino]-ethyl}-cyclopropyl-amid;
2-Amino-5-(3-methoxy-phenyl)-thiazol-4-carbonsäure {2-[(benzofuran-4-carbonyl)-amino]-ethyl}-cyclopropyl-amid;
2-Amino-5-(4-fluor-phenyl)-thiazol-4-carbonsäure {2-[(benzofuran-4-carbonyl)-amino]-ethyl}-cyclopropyl-amid;
2-Amino-5-(3-chlor-phenyl)-thiazol-4-carbonsäure {2-[(benzofuran-4-carbonyl)-amino]-ethyl}-cyclopropyl-amid;
2-Amino-5-phenyl-thiazol-4-carbonsäure {2-[(benzofuran-4-carbonyl)-amino]-ethyl}-cyclopropyl-amid;
2-Brom-5-(3-methoxy-phenyl)-thiazol-4-carbonsäure {2-[(benzofuran-4-carbonyl)-amino]-ethyl}-cyclopropyl-amid;
2-Brom-5-*p*-tolyl-thiazol-4-carbonsäure {2-[(benzofuran-4-carbonyl)-amino]-ethyl}-cyclopropyl-amid;
5-(2-Fluor-phenyl)-2-methyl-thiazol-4-carbonsäure {2-[(benzofuran-4-carbonyl)-amino]-ethyl}-cyclopropyl-amid;
2-Methyl-5-(3-trifluormethyl-phenyl)-thiazol-4-carbonsäure {2-[(benzofuran-4-carbonyl)-amino]-ethyl}-cyclopropyl-amid;
5-(3-Fluor-phenyl)-2-methyl-thiazol-4-carbonsäure {2-[(benzofuran-4-carbonyl)-amino]-ethyl}-cyclopropyl-amid;
2-Methyl-5-(4-trifluormethyl-phenyl)-thiazol-4-carbonsäure {2-[(benzofuran-4-carbonyl)-amino]-ethyl}-cyclopropyl-amid;
5-(4-Chlor-phenyl)-2-methyl-thiazol-4-carbonsäure {2-[(benzofuran-4-carbonyl)-amino]-ethyl}-cyclopropyl-amid;
5-(3-Methoxy-phenyl)-2-methyl-thiazol-4-carbonsäure {2-[(benzofuran-4-carbonyl)-amino]-ethyl}-cyclopropyl-amid;
5-(3-Cyano-phenyl)-2-methyl-thiazol-4-carbonsäure {2-[(benzofuran-4-carbonyl)-amino]-ethyl}-cyclopropyl-amid;
5-(3,4-Dimethyl-phenyl)-2-methyl-thiazol-4-carbonsäure {2-[(benzofuran-4-carbonyl)-amino]-ethyl}-cyclopropyl-amid;
2-Methyl-5-*p*-tolyl-thiazol-4-carbonsäure {2-[(benzofuran-4-carbonyl)-amino]-ethyl}-cyclopropyl-amid;
Benzofuran-4-carbonsäure {2-[cyclopropyl-(3'-methyl-biphenyl-2-carbonyl)-amino]-ethyl}-amid;
Benzofuran-4-carbonsäure {2-[cyclopropyl-(4'-methyl-biphenyl-2-carbonyl)-amino]-ethyl}-amid;
Benzofuran-4-carbonsäure {2-[cyclopropyl-(4'-fluor-biphenyl-2-carbonyl)-amino]-ethyl}-amid;
6-Methyl-imidazo[2,1-b]thiazol-5-carbonsäure {2-[cyclopropylmethyl-(2-cyclopropyl-5-phenyl-thiazol-4-carbonyl)-amino]-ethyl}-amid;
6-Methyl-imidazo[2,1-b]thiazol-5-carbonsäure {2-[cyclopropylmethyl-(2-cyclopropyl-5-*m*-tolyl-thiazol-4-carbonyl)-amino]-ethyl}-amid;
6-Methyl-imidazo[2,1-b]thiazol-5-carbonsäure (2-{[2-cyclopropyl-5-(3-methoxyphenyl)-thiazol-4-carbonyl]-cyclopropylmethyl-amino}-ethyl)-amid;
6-Methyl-imidazo[2,1-b]thiazol-5-carbonsäure {2-[(2-amino-5-*m*-tolyl-thiazol-4-carbonyl)-cyclopropylmethyl-amino]-ethyl}-amid;
6-Methyl-imidazo[2,1-b]thiazol-5-carbonsäure (2-{[2-amino-5-(3-fluor-phenyl)-thiazol-4-carbonyl]-cyclopropylmethyl-amino}-ethyl)-amid;
6-Methyl-imidazo[2,1-b]thiazol-5-carbonsäure (2-{[2-amino-5-(3-methoxy-phenyl)-thiazol-4-carbonyl]-cyclopropylmethyl-amino}-ethyl)-amid;
6-Methyl-imidazo[2,1-b]thiazol-5-carbonsäure (2-{[2-amino-5-(4-fluor-phenyl)-thiazol-4-carbonyl]-cyclopropylmethyl-amino}-ethyl)-amid;
6-Methyl-imidazo[2,1-b]thiazol-5-carbonsäure (2-{[2-amino-5-(3-chlor-phenyl)-thiazol-4-carbonyl]-cyclopropylmethyl-amino}-ethyl)-amid;
6-Methyl-imidazo[2,1-b]thiazol-5-carbonsäure {2-[(2-amino-5-phenyl-thiazol-4-carbonyl)-cyclopropylmethyl-amino]-ethyl}-amid;
6-Methyl-imidazo[2,1-b]thiazol-5-carbonsäure (2-{[2-brom-5-(3-methoxy-phenyl)-thiazol-4-carbonyl]-cyclopropylmethyl-amino}-ethyl)-amid;
6-Methyl-imidazo[2,1-b]thiazol-5-carbonsäure {2-[(2-brom-5-*p*-tolyl-thiazol-4-carbonyl)-cyclopropylmethyl-amino]-ethyl}-amid;
6-Methyl-imidazo[2,1-b]thiazol-5-carbonsäure (2-{cyclopropylmethyl-[5-(2-fluorphenyl)-2-methyl-thiazol-4-carbonyl]-amino}-ethyl)-amid;
6-Methyl-imidazo[2,1-b]thiazol-5-carbonsäure (2-{cyclopropylmethyl-[2-methyl-5-(3-trifluormethyl-phenyl)-thiazol-4-carbonyl]-amino}-ethyl)-amid;
6-Methyl-imidazo[2,1-b]thiazol-5-carbonsäure (2-{cyclopropylmethyl-[5-(3-fluorphenyl)-2-methyl-thiazol-4-carbonyl]-amino}-ethyl)-amid;
6-Methyl-imidazo[2,1-b]thiazol-5-carbonsäure (2-{cyclopropylmethyl-[2-methyl-5-(4-trifluormethyl-phenyl)-thiazol-4-carbonyl]-amino}-ethyl)-amid;
6-Methyl-imidazo[2,1-b]thiazol-5-carbonsäure (2-{[5-(4-chlor-phenyl)-2-methyl-thiazol-4-carbonyl]-cyclopropylmethyl-amino}-ethyl)-amid;
6-Methyl-imidazo[2,1-b]thiazol-5-carbonsäure (2-{cyclopropylmethyl-[5-(3-methoxyphenyl)-2-methyl-thiazol-4-carbonyl]-amino}-ethyl)-amid;
6-Methyl-imidazo[2,1-b]thiazol-5-carbonsäure (2-{[5-(3-cyano-phenyl)-2-methyl-thiazol-4-carbonyl]-cyclopropylmethyl-amino}-ethyl)-amid;
6-Methyl-imidazo[2,1-b]thiazol-5-carbonsäure (2-{cyclopropylmethyl-[5-(3,4-dimethylphenyl)-2-methyl-thiazol-4-carbonyl]-amino}-ethyl)-amid;
6-Methyl-imidazo[2,1-b]thiazol-5-carbonsäure {2-[cyclopropylmethyl-(2-methyl-5-*p-*tolyl-thiazol-4-carbonyl)-amino]-ethyl}-amid;
6-Methyl-imidazo[2,1-b]thiazol-5-carbonsäure {2-[cyclopropylmethyl-(3'-methyl-biphenyl-2-carbonyl)-amino]-ethyl}-amid;
6-Methyl-imidazo[2,1-b]thiazol-5-carbonsäure {2-[cyclopropylmethyl-(4'-methyl-biphenyl-2-carbonyl)-amino]-ethyl}-amid;
1-Methyl-1*H*-indazol-3-carbonsäure {2-[cyclopropylmethyl-(2-cyclopropyl-5-phenyl-thiazol-4-carbonyl)-amino]-ethyl}-amid;
1-Methyl-1*H*-indazol-3-carbonsäure {2-[cyclopropylmethyl-(2-cyclopropyl-5-*m*-tolyl-thiazol-4-carbonyl)-amino]-ethyl}-amid;
1-Methyl-1*H*-indazol-3-carbonsäure (2-{[2-cyclopropyl-5-(3-methoxy-phenyl)-thiazol-4-carbonyl]-cyclopropylmethyl-amino}-ethyl)-amid;
1-Methyl-1*H*-indazol-3-carbonsäure {2-[(2-amino-5-*m*-tolyl-thiazol-4-carbonyl)-cyclopropylmethyl-amino]-ethyl}-amid;
1-Methyl-1*H*-indazol-3-carbonsäure (2-{[2-amino-5-(3-fluor-phenyl)-thiazol-4-carbonyl]-cyclopropylmethyl-amino}-ethyl)-amid;
1-Methyl-1*H*-indazol-3-carbonsäure (2-{[2-amino-5-(3-methoxy-phenyl)-thiazol-4-carbonyl]-cyclopropylmethyl-amino}-ethyl)-amid;
1-Methyl-1*H*-indazol-3-carbonsäure (2-{[2-amino-5-(4-fluor-phenyl)-thiazol-4-carbonyl]-cyclopropylmethyl-amino}-ethyl)-amid;
1-Methyl-1*H*-indazol-3-carbonsäure (2-{[2-amino-5-(3-chlor-phenyl)-thiazol-4-carbonyl]-cyclopropylmethyl-amino}-ethyl)-amid;
1-Methyl-1*H*-indazol-3-carbonsäure {2-[(2-amino-5-phenyl-thiazol-4-carbonyl)-cyclopropylmethyl-amino]-ethyl}-amid;
1-Methyl-1*H*-indazol-3-carbonsäure (2-{[2-brom-5-(3-methoxy-phenyl)-thiazol-4-carbonyl]-cyclopropylmethyl-amino}-ethyl)-amid;
1-Methyl-1*H*-indazol-3-carbonsäure {2-[(2-brom-5-*p*-tolyl-thiazol-4-carbonyl)-cyclopropylmethyl-amino]-ethyl}-amid;
1-Methyl-1*H*-indazol-3-carbonsäure (2-{cyclopropylmethyl-[5-(2-fluor-phenyl)-2-methyl-thiazol-4-carbonyl]-amino}-ethyl)-amid;
1-Methyl-1*H*-indazol-3-carbonsäure (2-{cyclopropylmethyl-[2-methyl-5-(3-trifluormethyl-phenyl)-thiazol-4-carbonyl]-amino}-ethyl)-amid;
1-Methyl-1*H*-indazol-3-carbonsäure (2-{cyclopropylmethyl-[5-(3-fluor-phenyl)-2-methyl-thiazol-4-carbonyl]-amino}-ethyl)-amid;
1-Methyl-1*H*-indazol-3-carbonsäure (2-{cyclopropylmethyl-[2-methyl-5-(4-trifluormethyl-phenyl)-thiazol-4-carbonyl]-amino}-ethyl)-amid;
1-Methyl-1*H*-indazol-3-carbonsäure (2-{[5-(4-chlor-phenyl)-2-methyl-thiazol-4-carbonyl]-cyclopropylmethyl-amino}-ethyl)-amid;
1-Methyl-1*H*-indazol-3-carbonsäure (2-{cyclopropylmethyl-[5-(3-methoxy-phenyl)-2-methyl-thiazol-4-carbonyl]-amino}-ethyl)-amid;
1-Methyl-1*H*-indazol-3-carbonsäure (2-{[5-(3-cyano-phenyl)-2-methyl-thiazol-4-carbonyl]-cyclopropylmethyl-amino}-ethyl)-amid;
1-Methyl-1*H*-indazol-3-carbonsäure (2-{cyclopropylmethyl-[5-(3,4-dimethyl-phenyl)-2-methyl-thiazol-4-carbonyl]-amino}-ethyl)-amid;
1-Methyl-1*H*-indazol-3-carbonsäure {2-[cyclopropylmethyl-(2-methyl-5-*p*-tolyl-thiazol-4-carbonyl)-amino]-ethyl}-amid;
1-Methyl-1*H*-indazol-3-carbonsäure {2-[cyclopropylmethyl-(3'-methyl-biphenyl-2-carbonyl)-amino]-ethyl}-amid;
1-Methyl-1*H*-indazol-3-carbonsäure {2-[cyclopropylmethyl-(4'-methyl-biphenyl-2-carbonyl)-amino]-ethyl}-amid;
1-Methyl-1*H*-indazol-3-carbonsäure {2-[cyclopropylmethyl-(4'-fluor-biphenyl-2-carbonyl)-amino]-ethyl}-amid;
2-Cyclopropyl-5-phenyl-thiazol-4-carbonsäurecyclopropyl-{2-[(2,3-dihydro-benzo[1,4]dioxin-5-carbonyl)-amino]-ethyl}-amid;
2-Cyclopropyl-5-*m*-tolyl-thiazol-4-carbonsäurecyclopropyl-{2-[(2,3-dihydro-benzo[1,4]dioxin-5-carbonyl)-amino]-ethyl}-amid;
2-Cyclopropyl-5-(3-methoxy-phenyl)-thiazol-4-carbonsäurecyclopropyl-{2-[(2,3-dihydro-benzo[1,4]dioxin-5-carbonyl)-amino]-ethyl}-amid;
2-Amino-5-*m*-tolyl-thiazol-4-carbonsäurecyclopropyl-{2-[(2,3-dihydro-benzo[1,4]dioxin-5-carbonyl)-amino]-ethyl}-amid;
2-Amino-5-(3-fluor-phenyl)-thiazol-4-carbonsäurecyclopropyl-{2-[(2,3-dihydro-benzo[1,4]dioxin-5-carbonyl)-amino]-ethyl}-amid;
2-Amino-5-(3-methoxy-phenyl)-thiazol-4-carbonsäurecyclopropyl-{2-[(2,3-dihydro-benzo[1,4]dioxin-5-carbonyl)-amino]-ethyl}-amid;
2-Amino-5-(4-fluor-phenyl)-thiazol-4-carbonsäurecyclopropyl-{2-[(2,3-dihydro-benzo[1,4]dioxin-5-carbonyl)-amino]-ethyl}-amid;
2-Amino-5-(3-chlor-phenyl)-thiazol-4-carbonsäurecyclopropyl-{2-[(2,3-dihydro-benzo[1,4]dioxin-5-carbonyl)-amino]-ethyl}-amid;
2-Amino-5-phenyl-thiazol-4-carbonsäurecyclopropyl-{2-[(2,3-dihydro-benzo[1,4]dioxin-5-carbonyl)-amino]-ethyl}-amid;
2-Brom-5-(3-methoxy-phenyl)-thiazol-4-carbonsäurecyclopropyl-{2-[(2,3-dihydro-benzo[1,4]dioxin-5-carbonyl)-amino]-ethyl}-amid;
2-Brom-5-*p*-tolyl-thiazol-4-carbonsäurecyclopropyl-{2-[(2,3-dihydro-benzo[1,4]dioxin-5-carbonyl)-amino]-ethyl}-amid;
5-(2-Fluor-phenyl)-2-methyl-thiazol-4-carbonsäurecyclopropyl-{2-[(2,3-dihydro-benzo[1,4]dioxin-5-carbonyl)-amino]-ethyl}-amid;
2-Methyl-5-(3-trifluormethyl-phenyl)-thiazol-4-carbonsäurecyclopropyl-{2-[(2,3-dihydro-benzo[1,4]dioxin-5-carbonyl)-amino]-ethyl}-amid;
5-(3-Fluor-phenyl)-2-methyl-thiazol-4-carbonsäurecyclopropyl-{2-[(2,3-dihydro-benzo[1,4]dioxin-5-carbonyl)-amino]-ethyl}-amid;
5-(4-Chlor-phenyl)-2-methyl-thiazol-4-carbonsäurecyclopropyl-{2-[(2,3-dihydro-benzo[1,4]dioxin-5-carbonyl)-amino]-ethyl}-amid;
5-(3-Methoxy-phenyl)-2-methyl-thiazol-4-carbonsäurecyclopropyl-{2-[(2,3-dihydro-benzo[1,4]dioxin-5-carbonyl)-amino]-ethyl}-amid;
5-(3,4-Dimethyl-phenyl)-2-methyl-thiazol-4-carbonsäurecyclopropyl-{2-[(2,3-dihydro-benzo[1,4]dioxin-5-carbonyl)-amino]-ethyl}-amid;
2-Methyl-5-*p*-tolyl-thiazol-4-carbonsäurecyclopropyl-{2-[(2,3-dihydro-benzo[1,4]dioxin-5-carbonyl)-amino]-ethyl}-amid;
2,3-Dihydro-benzo[1,4]dioxin-5-carbonsäure {2-[cyclopropyl-(3'-methyl-biphenyl-2-carbonyl)-amino]-ethyl}-amid;
2,3-Dihydro-benzo[1,4]dioxin-5-carbonsäure {2-[cyclopropyl-(4'-methyl-biphenyl-2-carbonyl)-amino]-ethyl}-amid;
2,3-Dihydro-benzo[1,4]dioxin-5-carbonsäure {2-[cyclopropyl-(4'-fluor-biphenyl-2-carbonyl)-amino]-ethyl}-amid;
2-Cyclopropyl-5-phenyl-thiazol-4-carbonsäurecyclopropylmethyl-{2-[(2,3-dihydro-benzo[1,4]dioxin-5-carbonyl)-amino]-ethyl}-amid;
2-Cyclopropyl-5-*m*-tolyl-thiazol-4-carbonsäurecyclopropylmethyl-{2-[(2,3-dihydro-benzo[1,4]dioxin-5-carbonyl)-amino]-ethyl}-amid;
2-Cyclopropyl-5-(3-methoxy-phenyl)-thiazol-4-carbonsäurecyclopropylmethyl-{2-[(2,3-dihydro-benzo[1,4]dioxin-5-carbonyl)-amino]-ethyl}-amid;
2-Amino-5-*m*-tolyl-thiazol-4-carbonsäurecyclopropylmethyl-{2-[(2,3-dihydro-benzo[1,4]dioxin-5-carbonyl)-amino]-ethyl}-amid;
2-Amino-5-(3-fluor-phenyl)-thiazol-4-carbonsäurecyclopropylmethyl-{2-[(2,3-dihydro-benzo[1,4]dioxin-5-carbonyl)-amino]-ethyl}-amid;
2-Amino-5-(3-methoxy-phenyl)-thiazol-4-carbonsäurecyclopropylmethyl-{2-[(2,3-dihydro-benzo[1,4]dioxin-5-carbonyl)-amino]-ethyl}-amid;
2-Amino-5-phenyl-thiazol-4-carbonsäurecyclopropylmethyl-{2-[(2,3-dihydro-benzo[1,4]dioxin-5-carbonyl)-amino]-ethyl}-amid;
2-Brom-5-(3-methoxy-phenyl)-thiazol-4-carbonsäurecyclopropylmethyl-{2-[(2,3-dihydro-benzo[1,4]dioxin-5-carbonyl)-amino]-ethyl}-amid;
2-Brom-5-*p*-tolyl-thiazol-4-carbonsäurecyclopropylmethyl-{2-[(2,3-dihydro-benzo[1,4]dioxin-5-carbonyl)-amino]-ethyl}-amid;
5-(2-Fluor-phenyl)-2-methyl-thiazol-4-carbonsäurecyclopropylmethyl-{2-[(2,3-dihydro-benzo[1,4]dioxin-5-carbonyl)-amino]-ethyl}-amid;
2-Methyl-5-(3-trifluormethyl-phenyl)-thiazol-4-carbonsäurecyclopropylmethyl-{2-[(2,3-dihydro-benzo[1,4]dioxin-5-carbonyl)-amino]-ethyl}-amid;
5-(3-Fluor-phenyl)-2-methyl-thiazol-4-carbonsäurecyclopropylmethyl-{2-[(2,3-dihydro-benzo[1,4]dioxin-5-carbonyl)-amino]-ethyl}-amid;
5-(3-Methoxy-phenyl)-2-methyl-thiazol-4-carbonsäurecyclopropylmethyl-{2-[(2,3-dihydro-benzo[1,4]dioxin-5-carbonyl)-amino]-ethyl}-amid;
5-(3-Cyano-phenyl)-2-methyl-thiazol-4-carbonsäurecyclopropylmethyl-{2-[(2,3-dihydro-benzo[1,4]dioxin-5-carbonyl)-amino]-ethyl}-amid;
5-(3,4-Dimethyl-phenyl)-2-methyl-thiazol-4-carbonsäurecyclopropylmethyl-{2-[(2,3-dihydro-benzo[1,4]dioxin-5-carbonyl)-amino]-ethyl}-amid;
2-Methyl-5-*p*-tolyl-thiazol-4-carbonsäurecyclopropylmethyl-{2-[(2,3-dihydro-benzo[1,4]dioxin-5-carbonyl)-amino]-ethyl}-amid;
2,3-Dihydro-benzo[1,4]dioxin-5-carbonsäure {2-[cyclopropylmethyl-(3'-methyl-biphenyl-2-carbonyl)-amino]-ethyl}-amid;
2,3-Dihydro-benzo[1,4]dioxin-5-carbonsäure {2-[cyclopropylmethyl-(4'-methyl-biphenyl-2-carbonyl)-amino]-ethyl}-amid;
2,3-Dihydro-benzo[1,4]dioxin-5-carbonsäure {2-[cyclopropylmethyl-(4'-fluor-biphenyl-2-carbonyl)-amino]-ethyl}-amid;
2-Cyclopropyl-5-phenyl-thiazol-4-carbonsäure {2-[(benzofuran-4-carbonyl)-amino]-ethyl}-cyclopropylmethyl-amid;
2-Cyclopropyl-5-*m*-tolyl-thiazol-4-carbonsäure {2-[(benzofuran-4-carbonyl)-amino]-ethyl}-cyclopropylmethyl-amid;
2-Cyclopropyl-5-(3-methoxy-phenyl)-thiazol-4-carbonsäure {2-[(benzofuran-4-carbonyl)-amino]-ethyl}-cyclopropylmethyl-amid;
2-Amino-5-*m*-tolyl-thiazol-4-carbonsäure {2-[(benzofuran-4-carbonyl)-amino]-ethyl}-cyclopropylmethyl-amid;
2-Amino-5-(3-fluor-phenyl)-thiazol-4-carbonsäure {2-[(benzofuran-4-carbonyl)-amino]-ethyl}-cyclopropylmethyl-amid;
2-Amino-5-(3-methoxy-phenyl)-thiazol-4-carbonsäure {2-[(benzofuran-4-carbonyl)-amino]-ethyl}-cyclopropylmethyl-amid;
2-Amino-5-(4-fluor-phenyl)-thiazol-4-carbonsäure {2-[(benzofuran-4-carbonyl)-amino]-ethyl}-cyclopropylmethyl-amid;
2-Amino-5-(3-chlor-phenyl)-thiazol-4-carbonsäure {2-[(benzofuran-4-carbonyl)-amino]-ethyl}-cyclopropylmethyl-amid;
2-Amino-5-phenyl-thiazol-4-carbonsäure {2-[(benzofuran-4-carbonyl)-amino]-ethyl}-cyclopropylmethyl-amid;
2-Brom-5-(3-methoxy-phenyl)-thiazol-4-carbonsäure {2-[(benzofuran-4-carbonyl)-amino]-ethyl}-cyclopropylmethyl-amid;
2-Brom-5-*p*-tolyl-thiazol-4-carbonsäure {2-[(benzofuran-4-carbonyl)-amino]-ethyl}-cyclopropylmethyl-amid;
5-(2-Fluor-phenyl)-2-methyl-thiazol-4-carbonsäure {2-[(benzofuran-4-carbonyl)-amino]-ethyl}-cyclopropylmethyl-amid;
2-Methyl-5-(3-trifluormethyl-phenyl)-thiazol-4-carbonsäure {2-[(benzofuran-4-carbonyl)-amino]-ethyl}-cyclopropylmethyl-amid;
5-(3-Fluor-phenyl)-2-methyl-thiazol-4-carbonsäure {2-[(benzofuran-4-carbonyl)-amino]-ethyl}-cyclopropylmethyl-amid;
2-Methyl-5-(4-trifluormethyl-phenyl)-thiazol-4-carbonsäure {2-[(benzofuran-4-carbonyl)-amino]-ethyl}-cyclopropylmethyl-amid;
5-(4-Chlor-phenyl)-2-methyl-thiazol-4-carbonsäure {2-[(benzofuran-4-carbonyl)-amino]-ethyl}-cyclopropylmethyl-amid;
5-(3-Methoxy-phenyl)-2-methyl-thiazol-4-carbonsäure {2-[(benzofuran-4-carbonyl)-amino]-ethyl}-cyclopropylmethyl-amid;
5-(3-Cyano-phenyl)-2-methyl-thiazol-4-carbonsäure {2-[(benzofuran-4-carbonyl)-amino]-ethyl}-cyclopropylmethyl-amid;
5-(3,4-Dimethyl-phenyl)-2-methyl-thiazol-4-carbonsäure {2-[(benzofuran-4-carbonyl)-amino]-ethyl}-cyclopropylmethyl-amid;
2-Methyl-5-*p*-tolyl-thiazol-4-carbonsäure {2-[(benzofuran-4-carbonyl)-amino]-ethyl}-cyclopropylmethyl-amid;
Benzofuran-4-carbonsäure {2-[cyclopropylmethyl-(3'-methyl-biphenyl-2-carbonyl)-amino]-ethyl}-amid;
Benzofuran-4-carbonsäure {2-[cyclopropylmethyl-(4'-methyl-biphenyl-2-carbonyl)-amino]-ethyl}-amid; und
Benzofuran-4-carbonsäure {2-[cyclopropylmethyl-(4'-fluor-biphenyl-2-carbonyl)-amino]-ethyl}-amid;
oder ein Salz einer solchen Verbindung.

9. Pharmazeutische Zusammensetzung, die als Wirkstoff eine Verbindung der Formel (I) nach einem der Ansprüche 1 bis 8 oder ein pharmazeutisch akzeptables Salz davon und wenigstens einen therapeutisch inerten Exzipienten enthält.

10. Verbindung nach einem der Ansprüche 1 bis 8 oder ein pharmazeutisch akzeptables Salz davon zur Verwendung als Medikament.

11. Verbindung nach einem der Ansprüche 1 bis 8 oder ein pharmazeutisch akzeptables Salz davon zur Verhütung oder Behandlung von Krankheiten, die ausgewählt sind aus der Gruppe bestehend aus allen Arten von Schlafstörungen, stressbedingten Syndromen, Psychotropikumgebrauch und -missbrauch, kognitiven Fehlfunktionen bei der gesunden Bevölkerung und bei psychiatrischen und neurologischen Störungen, Ess- oder Trinkstörungen.

12. Verwendung einer Verbindung nach einem der Ansprüche 1 bis 8 oder eines pharmazeutisch akzeptablen Salzes davon zur Herstellung eines Medikaments zur Verhütung oder Behandlung von Krankheiten, die ausgewählt sind aus der Gruppe bestehend aus allen Arten von Schlafstörungen, stressbedingten Syndromen, Psychotropikumgebrauch und -missbrauch, kognitiven Fehlfunktionen bei der gesunden Bevölkerung und bei psychiatrischen und neurologischen Störungen, Ess- oder Trinkstörungen.

## Revendications

1. Composé de formule (I) 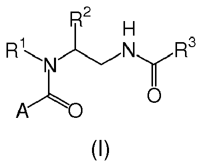 où
R¹ représente un cyclopropyle ou un cyclopropyl-méthyle ;
R² représente un hydrogène, (C₁₋₄)alkyle, cyclopropyle ou phényl-(C₁₋₄)alkyle, où le phényle est non substitué ou mono-, di- ou trisubstitué par des substituants indépendamment sélectionnés dans le groupe consistant en un (C₁₋₄)alkyle, (C₁₋₄)alkoxy, trifluorométhyle et halogène ;
R³ représente un hétérocyclyle, ledit hétérocyclyle étant un système bicyclique à 8 à 10 chaînons contenant 1, 2 ou 3 hétéroatomes qui sont chacun indépendamment sélectionnés parmi l'oxygène, l'azote et le soufre, où au moins un noyau dudit système bicyclique est aromatique et où la liaison par laquelle l'hétérocyclyle est rattaché au restant de la molécule est positionnée sur un atome de carbone aromatique dudit système bicyclique en position alpha par rapport à un atome en tête de pont ; où ledit hétérocyclyle est non substitué ou mono-, di- ou trisubstitué par des substituants indépendamment sélectionnés dans le groupe consistant en un (C₁₋₄)alkyle, (C₁₋₄)alkoxy, halogène et trifluorométhyle ;
A représente 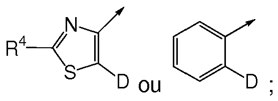 R⁴ représente un (C₁₋₄)alkyle, bromo, cyclopropyle ou -NR⁵R⁶ ;
R⁵ représente un hydrogène ou un (C₁₋₄)alkyle ;
R⁶ représente un hydrogène ou un (C₁₋₄)alkyle ; et
D représente un phényle qui est non substitué ou mono-, di- ou trisubstitué par des substituants indépendamment sélectionnés dans le groupe consistant en un (C₁₋₄)alkyle, (C₁₋₄)alkoxy, trifluorométhyle, trifluorométhoxy, cyano et halogène ;
ou sel de celui-ci.

2. Composé de formule (I) selon la revendication 1, où A représente 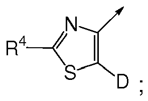 ou sel de celui-ci.

3. Composé de formule (I) selon la revendication 1 ou 2, où R⁴ représente un méthyle, -NH₂, bromo ou cyclopropyle ;
ou sel de celui-ci.

4. Composé de formule (I) selon l'une quelconque des revendications 1 à 3, où D représente un phényle qui est non substitué ou mono- ou disubstitué par des substituants indépendamment sélectionnés dans le groupe consistant en un (C₁₋₄)alkyle, (C₁₋₄)alkoxy, trifluorométhyle, cyano et halogène ;
ou sel de celui-ci.

5. Composé selon l'une quelconque des revendications 1 à 4, où R² représente un hydrogène, méthyle, cyclopropyle ou phényl non substitué-(C₁₋₂)alkyle ;
ou sel de celui-ci.

6. Composé selon l'une quelconque des revendications 1 à 5, où R³ représente un groupement sélectionné parmi les suivants : quinoléin-8-yle, isoquinoléin-1-yle, indol-3-yle, indol-4-yle, indol-7-yle, indazol-3-yle, indazol-4-yle, indazol-7-yle, benzofuran-4-yle, benzofuran-7-yle, benzisoxazol-3-yle, benzisoxazol-4-yle, benzisoxazol-7-yle, benzoxazol-4-yle, benzoxazol-7-yle, benzoxadiazol-4-yle, benzoxadiazol-7-yle, benzothiophén-3-yle, benzothiophén-4-yle, benzothiophén-7-yle, benzothiazol-4-yle, benzothiazol-7-yle, benzoisothiazol-3-yle, benzoisothiazol-4-yle, benzoisothiazol-7-yle, benzothiadiazol-4-yle, benzothiadiazol-7-yle, benzimidazol-4-yle, benzimidazol-7-yle, imidazo[2,1-b]thiazol-3-yle, imidazo[2,1-b]thiazol-5-yle, pyrazolo[1,5-a]pyridine-3-yle, imidazo[1,2-a]pyridine-3-yle, 2,3-dihydro-thiéno[3,4-b][1,4]dioxine-5-yle, benzo[1,3]dioxol-4-yle, 2,3-dihydro-benzofuran-4-yle, 2,3-dihydro-benzofuran-7-yle, 4*H*-benzo[1,3]dioxin-8-yle, 4*H-*benzo[1,3]dioxin-5-yle et 2,3-dihydro-benzo[1,4]dioxin-5-yle ; où ledit groupement est non substitué ou mono- ou disubstitué par des substituants indépendamment sélectionnés dans le groupe consistant en un méthyle, méthoxy, halogène et trifluorométhyle ;
ou sel de celui-ci.

7. Composé selon l'une quelconque des revendications 1 à 5, où R³ représente 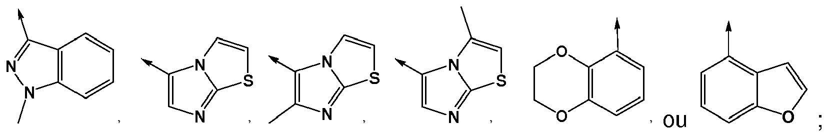 ou sel de celui-ci.

8. Composé selon la revendication 1, sélectionné parmi le groupe consistant en les suivants :
{2-[(Benzofuran-4-carbonyl)-amino]-éthyl}-cyclopropylméthyl-amide de l'acide 2-méthyl-5-phényl-thiazole-4-carboxylique ;
{2-[Cyclopropylméthyl-(2-méthyl-5-phényl-thiazole-4-carbonyl)-amino]-éthyl}-amide de l'acide 6-méthyl-imidazo[2,1-b]thiazole-5-carboxylique ;
Cyclopropylméthyl-{2-[(2,3-dihydro-benzo[1,4]dioxine-5-carbonyl)-amino]-éthyl}-amide de l'acide 2-méthyl-5-phényl-thiazole-4-carboxylique ;
{2-[(Benzofuran-4-carbonyl)-amino]-éthyl}-cyclopropyl-amide de l'acide 2-méthyl-5-phényl-thiazole-4-carboxylique ;
{2-[Cyclopropyl-(2-méthyl-5-phényl-thiazole-4-carbonyl)-amino]-éthyl}-amide de l'acide 6-méthyl-imidazo[2,1-b]thiazole-5-carboxylique ;
Cyclopropyl-{2-[(2,3-dihydro-benzo[1,4]dioxine-5-carbonyl)-amino]-éthyl}-amide de l'acide 2-méthyl-5-phényl-thiazole-4-carboxylique ;
{2-[Cyclopropyl-(2-cyclopropyl-5-phényl-thiazole-4-carbonyl)-amino]-éthyl}-amide de l'acide 6-méthyl-imidazo[2,1-b]thiazole-5-carboxylique ;
{2-[Cyclopropyl-(2-cyclopropyl-5-*m*-tolyl-thiazole-4-carbonyl)-amino]-éthyl}-amide de l'acide 6-méthyl-imidazo[2,1-b]thiazole-5-carboxylique ;
(2-{Cyclopropyl-[2-cyclopropyl-5-(3-méthoxy-phényl)-thiazole-4-carbonyl]-amino}-éthyl)-amide de l'acide 6-méthyl-imidazo[2,1-b]thiazole-5-carboxylique ;
{2-[(2-Amino-5-*m*-tolyl-thiazole-4-carbonyl)-cyclopropyl-amino]-éthyl}-amide de l'acide 6-méthyl-imidazo[2,1-b]thiazole-5-carboxylique ;
(2-{[2-Amino-5-(3-fluoro-phényl)-thiazole-4-carbonyl]-cyclopropyl-amino}-éthyl)-amide de l'acide 6-méthyl-imidazo[2,1-b]thiazole-5-carboxylique ;
(2-{[2-Amino-5-(3-méthoxy-phényl)-thiazole-4-carbonyl]-cyclopropyl-amino}-éthyl)-amide de l'acide 6-méthyl-imidazo[2,1-b]thiazole-5-carboxylique ;
(2-{[2-Amino-5-(4-fluoro-phényl)-thiazole-4-carbonyl]-cyclopropyl-amino}-éthyl)-amide de l'acide 6-méthyl-imidazo[2,1-b]thiazole-5-carboxylique ;
(2-{[2-Amino-5-(3-chloro-phényl)-thiazole-4-carbonyl]-cyclopropyl-amino}-éthyl)-amide de l'acide 6-méthyl-imidazo[2,1-b]thiazole-5-carboxylique ;
{2-[(2-Amino-5-phényl-thiazole-4-carbonyl)-cyclopropyl-amino]-éthyl}-amide de l'acide 6-méthyl-imidazo[2,1-b]thiazole-5-carboxylique ;
(2-{[2-Bromo-5-(3-méthoxy-phényl)-thiazole-4-carbonyl]-cyclopropyl-amino}-éthyl)-amide de l'acide 6-méthyl-imidazo[2,1-b]thiazole-5-carboxylique ;
{2-[(2-Bromo-5-*p*-tolyl-thiazole-4-carbonyl)-cyclopropyl-amino]-éthyl}-amide de l'acide 6-méthyl-imidazo[2,1-b]thiazole-5-carboxylique ;
(2-{Cyclopropyl-[5-(2-fluoro-phényl)-2-méthyl-thiazole-4-carbonyl]-amino}-éthyl)-amide de l'acide 6-méthyl-imidazo[2,1-b]thiazole-5-carboxylique ;
(2-{Cyclopropyl-[2-méthyl-5-(3-trifluorométhyl-phényl)-thiazole-4-carbonyl]-amino}-éthyl)-amide de l'acide 6-méthyl-imidazo[2,1-b]thiazole-5-carboxylique ;
(2-{Cyclopropyl-[5-(3-fluoro-phényl)-2-méthyl-thiazole-4-carbonyl]-amino}-éthyl)-amide de l'acide 6-méthyl-imidazo[2,1-b]thiazole-5-carboxylique ;
(2-{Cyclopropyl-[2-méthyl-5-(4-trifluorométhyl-phényl)-thiazole-4-carbonyl]-amino}-éthyl)-amide de l'acide 6-méthyl-imidazo[2,1-b]thiazole-5-carboxylique ;
(2-{[5-(4-Chloro-phényl)-2-méthyl-thiazole-4-carbonyl]-cyclopropyl-amino}-éthyl)-amide de l'acide 6-méthyl-imidazo[2,1-b]thiazole-5-carboxylique ;
(2-{Cyclopropyl-[5-(3-méthoxy-phényl)-2-méthyl-thiazole-4-carbonyl]-amino}-éthyl)-amide de l'acide 6-méthyl-imidazo[2,1-b]thiazole-5-carboxylique ;
(2-{[5-(3-Cyano-phényl)-2-méthyl-thiazole-4-carbonyl]-cyclopropyl-amino}-éthyl)-amide de l'acide 6-méthyl-imidazo[2,1-b]thiazole-5-carboxylique ;
(2-{Cyclopropyl-[5-(3,4-diméthyl-phényl)-2-méthyl-thiazole-4-carbonyl]-amino}-éthyl)-amide de l'acide 6-méthyl-imidazo[2,1-b]thiazole-5-carboxylique ;
{2-[Cyclopropyl-(2-méthyl-5-*p*-tolyl-thiazole-4-carbonyl)-amino]-éthyl}-amide de l'acide 6-méthyl-imidazo[2,1-b]thiazole-5-carboxylique ;
{2-[Cyclopropyl-(3'-méthyl-biphényl-2-carbonyl)-amino]-éthyl}-amide de l'acide 6-méthyl-imidazo[2,1-b]thiazole-5-carboxylique ;
{2-[Cyclopropyl-(4'-méthyl-biphényl-2-carbonyl)-amino]-éthyl}-amide de l'acide 6-méthyl-imidazo[2,1-b]thiazole-5-carboxylique ;
{2-[Cyclopropyl-(4'-fluoro-biphényl-2-carbonyl)-amino]-éthyl}-amide de l'acide 6-méthyl-imidazo[2,1-b]thiazole-5-carboxylique ;
{2-[Cyclopropyl-(2-cyclopropyl-5-phényl-thiazole-4-carbonyl)-amino]-éthyl}-amide de l'acide 1-méthyl-1*H*-indazole-3-carboxylique ;
{2-[Cyclopropyl-(2-cyclopropyl-5-*m*-tolyl-thiazole-4-carbonyl)-amino]-éthyl}-amide de l'acide 1-méthyl-1*H*-indazole-3-carboxylique ;
(2-{Cyclopropyl-[2-cyclopropyl-5-(3-méthoxy-phényl)-thiazole-4-carbonyl]-amino}-éthyl)-amide de l'acide 1-méthyl-1*H*-indazole-3-carboxylique ;
{2-[(2-Amino-5-*m*-tolyl-thiazole-4-carbonyl)-cyclopropyl-amino]-éthyl}-amide de l'acide 1-méthyl-1*H*-indazole-3-carboxylique ;
(2-{[2-Amino-5-(3-fluoro-phényl)-thiazole-4-carbonyl]-cyclopropyl-amino}-éthyl)-amide de l'acide 1-méthyl-1*H*-indazole-3-carboxylique ;
(2-{[2-Amino-5-(3-méthoxy-phényl)-thiazole-4-carbonyl]-cyclopropyl-amino}-éthyl)-amide de l'acide 1-méthyl-1*H*-indazole-3-carboxylique ;
(2-{[2-Amino-5-(4-fluoro-phényl)-thiazole-4-carbonyl]-cyclopropyl-amino}-éthyl)-amide de l'acide 1-méthyl-1*H*-indazole-3-carboxylique ;
(2-{[2-Amino-5-(3-chloro-phényl)-thiazole-4-carbonyl]-cyclopropyl-amino}-éthyl)-amide de l'acide 1-méthyl-1*H*-indazole-3-carboxylique ;
{2-[(2-Amino-5-phényl-thiazole-4-carbonyl)-cyclopropyl-amino]-éthyl}-amide de l'acide 1-méthyl-1*H*-indazole-3-carboxylique ;
(2-{[2-Bromo-5-(3-méthoxy-phényl)-thiazole-4-carbonyl]-cyclopropyl-amino}-éthyl)-amide de l'acide 1-méthyl-1*H*-indazole-3-carboxylique ;
{2-[(2-Bromo-5-*p*-tolyl-thiazole-4-carbonyl)-cyclopropyl-amino]-éthyl}-amide de l'acide 1-méthyl-1*H*-indazole-3-carboxylique ;
(2-{cyclopropyl-[5-(2-fluoro-phényl)-2-méthyl-thiazole-4-carbonyl]-amino}-éthyl)-amide de l'acide 1-méthyl-1*H*-indazole-3-carboxylique ;
(2-{Cyclopropyl-[2-méthyl-5-(3-trifluorométhyl-phényl)-thiazole-4-carbonyl]-amino}-éthyl)-amide de l'acide 1-méthyl-1*H*-indazole-3-carboxylique ;
(2-{Cyclopropyl-[5-(3-fluoro-phényl)-2-méthyl-thiazole-4-carbonyl]-amino}-éthyl)-amide de l'acide 1-méthyl-1*H*-indazole-3-carboxylique ;
(2-{Cyclopropyl-[2-méthyl-5-(4-trifluorométhyl-phényl)-thiazole-4-carbonyl]-amino}-éthyl)-amide de l'acide 1-méthyl-1*H*-indazole-3-carboxylique ;
(2-{[5-(4-Chloro-phényl)-2-méthyl-thiazole-4-carbonyl]-cyclopropyl-amino}-éthyl)-amide de l'acide 1-méthyl-1*H*-indazole-3-carboxylique ;
(2-{Cyclopropyl-[5-(3-méthoxy-phényl)-2-méthyl-thiazole-4-carbonyl]-amino}-éthyl)-amide de l'acide 1-méthyl-1*H*-indazole-3-carboxylique ;
(2-{[5-(3-Cyano-phényl)-2-méthyl-thiazole-4-carbonyl]-cyclopropyl-amino}-éthyl)-amide de l'acide 1-méthyl-1*H*-indazole-3-carboxylique ;
(2-{Cyclopropyl-[5-(3,4-diméthyl-phényl)-2-méthyl-thiazole-4-carbonyl]-amino}-éthyl)-amide de l'acide 1-méthyl-1*H*-indazole-3-carboxylique ;
{2-[Cyclopropyl-(2-méthyl-5-*p*-tolyl-thiazole-4-carbonyl)-amino]-éthyl}-amide de l'acide 1-méthyl-1*H*-indazole-3-carboxylique ;
{2-[Cyclopropyl-(3'-méthyl-biphényl-2-carbonyl)-amino]-éthyl}-amide de l'acide 1-méthyl-1*H*-indazole-3-carboxylique ;
{2-[Cyclopropyl-(4'-méthyl-biphényl-2-carbonyl)-amino]-éthyl}-amide de l'acide 1-méthyl-1*H*-indazole-3-carboxylique ;
{2-[Cyclopropyl-(4'-fluoro-biphényl-2-carbonyl)-amino]-éthyl}-amide de l'acide 1-méthyl-1*H*-indazole-3-carboxylique ;
{2-[(Benzofuran-4-carbonyl)-amino]-éthyl}-cyclopropyl-amide de l'acide 2-cyclopropyl-5-phényl-thiazole-4-carboxylique ;
{2-[(Benzofuran-4-carbonyl)-amino]-éthyl}-cyclopropyl-amide de l'acide 2-cyclopropyl-5-*m*-tolyl-thiazole-4-carboxylique ;
{2-[(Benzofuran-4-carbonyl)-amino]-éthyl}-cyclopropyl-amide de l'acide 2-cyclopropyl-5-(3-méthoxy-phényl)-thiazole-4-carboxylique ;
{2-[(Benzofuran-4-carbonyl)-amino]-éthyl}-cyclopropyl-amide de l'acide 2-amino-5-*m-*tolyl-thiazole-4-carboxylique ;
{2-[(Benzofuran-4-carbonyl)-amino]-éthyl}-cyclopropyl-amide de l'acide 2-amino-5-(3-fluoro-phényl)-thiazole-4-carboxylique ;
{2-[(Benzofuran-4-carbonyl)-amino]-éthyl}-cyclopropyl-amide de l'acide 2-amino-5-(3-méthoxy-phényl)-thiazole-4-carboxylique ;
{2-[(Benzofuran-4-carbonyl)-amino]-éthyl}-cyclopropyl-amide de l'acide 2-amino-5-(4-fluoro-phényl)-thiazole-4-carboxylique ;
{2-[(Benzofuran-4-carbonyl)-amino]-éthyl}-cyclopropyl-amide de l'acide 2-amino-5-(3-chloro-phényl)-thiazole-4-carboxylique ;
{2-[(Benzofuran-4-carbonyl)-amino]-éthyl}-cyclopropyl-amide de l'acide 2-amino-5-phényl-thiazole-4-carboxylique ;
{2-[(Benzofuran-4-carbonyl)-amino]-éthyl}-cyclopropyl-amide de l'acide 2-bromo-5-(3-méthoxy-phényl)-thiazole-4-carboxylique ;
{2-[(Benzofuran-4-carbonyl)-amino]-éthyl}-cyclopropyl-amide de l'acide 2-bromo-5-*p-*tolyl-thiazole-4-carboxylique ;
{2-[(Benzofuran-4-carbonyl)-amino]-éthyl}-cyclopropyl-amide de l'acide 5-(2-fluoro-phényl)-2-méthyl-thiazole-4-carboxylique ;
{2-[(Benzofuran-4-carbonyl)-amino]-éthyl}-cyclopropyl-amide de l'acide 2-méthyl-5-(3-trifluorométhyl-phényl)-thiazole-4-carboxylique ;
{2-[(Benzofuran-4-carbonyl)-amino]-éthyl}-cyclopropyl-amide de l'acide 5-(3-fluoro-phényl)-2-méthyl-thiazole-4-carboxylique ;
{2-[(Benzofuran-4-carbonyl)-amino]-éthyl}-cyclopropyl-amide de l'acide 2-méthyl-5-(4-trifluorométhyl-phényl)-thiazole-4-carboxylique ;
{2-[(Benzofuran-4-carbonyl)-amino]-éthyl}-cyclopropyl-amide de l'acide 5-(4-chloro-phényl)-2-méthyl-thiazole-4-carboxylique ;
{2-[(Benzofuran-4-carbonyl)-amino]-éthyl}-cyclopropyl-amide de l'acide 5-(3-méthoxy-phényl)-2-méthyl-thiazole-4-carboxylique ;
{2-[(Benzofuran-4-carbonyl)-amino]-éthyl}-cyclopropyl-amide de l'acide 5-(3-cyano-phényl)-2-méthyl-thiazole-4-carboxylique ;
{2-[(Benzofuran-4-carbonyl)-amino]-éthyl}-cyclopropyl-amide de l'acide 5-(3,4-diméthyl-phényl)-2-méthyl-thiazole-4-carboxylique ;
{2-[(Benzofuran-4-carbonyl)-amino]-éthyl}-cyclopropyl-amide de l'acide 2-méthyl-5-*p-*tolyl-thiazole-4-carboxylique ;
{2-[Cyclopropyl-(3'-méthyl-biphényl-2-carbonyl)-amino]-éthyl}-amide de l'acide benzofuran-4-carboxylique ;
{2-[Cyclopropyl-(4'-méthyl-biphényl-2-carbonyl)-amino]-éthyl}-amide de l'acide benzofuran-4-carboxylique ;
{2-[Cyclopropyl-(4'-fluoro-biphényl-2-carbonyl)-amino]-éthyl}-amide de l'acide benzofuran-4-carboxylique ;
{2-[Cyclopropylméthyl-(2-cyclopropyl-5-phényl-thiazole-4-carbonyl)-amino]-éthyl}-amide de l'acide 6-méthyl-imidazo[2,1-b]thiazole-5-carboxylique ;
{2-[Cyclopropylméthyl-(2-cyclopropyl-5-*m*-tolyl-thiazole-4-carbonyl)-amino]-éthyl}-amide de l'acide 6-méthyl-imidazo[2,1-b]thiazole-5-carboxylique ;
(2-{[2-Cyclopropyl-5-(3-méthoxy-phényl)-thiazole-4-carbonyl]-cyclopropylméthyl-amino}-éthyl)-amide de l'acide 6-méthyl-imidazo[2,1-b]thiazole-5-carboxylique ;
{2-[(2-Amino-5-*m*-tolyl-thiazole-4-carbonyl)-cyclopropylméthyl-amino]-éthyl}-amide de l'acide 6-méthyl-imidazo[2,1-b]thiazole-5-carboxylique ;
(2-{[2-Amino-5-(3-fluoro-phényl)-thiazole-4-carbonyl]-cyclopropylméthyl-amino}-éthyl)-amide de l'acide 6-méthyl-imidazo[2,1-b]thiazole-5-carboxylique ;
(2-{[2-Amino-5-(3-méthoxy-phényl)-thiazole-4-carbonyl]-cyclopropylméthyl-amino}-éthyl)-amide de l'acide 6-méthyl-imidazo[2,1-b]thiazole-5-carboxylique ;
(2-{[2-Amino-5-(4-fluoro-phényl)-thiazole-4-carbonyl]-cyclopropylméthyl-amino}-éthyl)-amide de l'acide 6-méthyl-imidazo[2,1-b]thiazole-5-carboxylique ;
(2-{[2-Amino-5-(3-chloro-phényl)-thiazole-4-carbonyl]-cyclopropylméthyl-amino}-éthyl)-amide de l'acide 6-méthyl-imidazo[2,1-b]thiazole-5-carboxylique ;
{2-[(2-Amino-5-phényl-thiazole-4-carbonyl)-cyclopropylméthyl-amino]-éthyl}-amide de l'acide 6-méthyl-imidazo[2,1-b]thiazole-5-carboxylique ;
(2-{[2-Bromo-5-(3-méthoxy-phényl)-thiazole-4-carbonyl]-cyclopropylméthyl-amino}-éthyl)-amide de l'acide 6-méthyl-imidazo[2,1-b]thiazole-5-carboxylique ;
{2-[(2-Bromo-5-*p*-tolyl-thiazole-4-carbonyl)-cyclopropylméthyl-amino]-éthyl}-amide de l'acide 6-méthyl-imidazo[2,1-b]thiazole-5-carboxylique ;
(2-{Cyclopropylméthyl-[5-(2-fluoro-phényl)-2-méthyl-thiazole-4-carbonyl]-amino}-éthyl)-amide de l'acide 6-méthyl-imidazo[2,1-b]thiazole-5-carboxylique ;
(2-{Cyclopropylméthyl-[2-méthyl-5-(3-trifluorométhyl-phényl)-thiazole-4-carbonyl]-amino}-éthyl)-amide de l'acide 6-méthyl-imidazo[2,1-b]thiazole-5-carboxylique ;
(2-{Cyclopropylméthyl-[5-(3-fluoro-phényl)-2-méthyl-thiazole-4-carbonyl]-amino}-éthyl)-amide de l'acide 6-méthyl-imidazo[2,1-b]thiazole-5-carboxylique ;
(2-{Cyclopropylméthyl-[2-méthyl-5-(4-trifluorométhyl-phényl)-thiazole-4-carbonyl]-amino}-éthyl)-amide de l'acide 6-méthyl-imidazo[2,1-b]thiazole-5-carboxylique ;
(2-{[5-(4-Chloro-phényl)-2-méthyl-thiazole-4-carbonyl]-cyclopropylméthyl-amino}-éthyl)-amide de l'acide 6-méthyl-imidazo[2,1-b]thiazole-5-carboxylique ;
(2-{Cyclopropylméthyl-[5-(3-méthoxy-phényl)-2-méthyl-thiazole-4-carbonyl]-amino}-éthyl)-amide de l'acide 6-méthyl-imidazo[2,1-b]thiazole-5-carboxylique ;
(2-{[5-(3-Cyano-phényl)-2-méthyl-thiazole-4-carbonyl]-cyclopropylméthyl-amino}-éthyl)-amide de l'acide 6-méthyl-imidazo[2,1-b]thiazole-5-carboxylique ;
(2-{Cyclopropylméthyl-[5-(3,4-diméthyl-phényl)-2-méthyl-thiazole-4-carbonyl]-amino}-éthyl)-amide de l'acide 6-méthyl-imidazo[2,1-b]thiazole-5-carboxylique ;
{2-[Cyclopropylméthyl-(2-méthyl-5-*p*-tolyl-thiazole-4-carbonyl)-amino]-éthyl}-amide de l'acide 6-méthyl-imidazo[2,1-b]thiazole-5-carboxylique ;
{2-[Cyclopropylméthyl-(3'-méthyl-biphényl-2-carbonyl)-amino]-éthyl}-amide de l'acide 6-méthyl-imidazo[2,1-b]thiazole-5-carboxylique ;
{2-[Cyclopropylméthyl-(4'-méthyl-biphényl-2-carbonyl)-amino]-éthyl}-amide de l'acide 6-méthyl-imidazo[2,1-b]thiazole-5-carboxylique ;
{2-[Cyclopropylméthyl-(2-cyclopropyl-5-phényl-thiazole-4-carbonyl)-amino]-éthyl}-amide de l'acide 1-méthyl-1*H*-indazole-3-carboxylique ;
{2-[Cyclopropylméthyl-(2-cyclopropyl-5-*m*-tolyl-thiazole-4-carbonyl)-amino]-éthyl}-amide de l'acide 1-méthyl-1*H*-indazole-3-carboxylique ;
(2-{[2-Cyclopropyl-5-(3-méthoxy-phényl)-thiazole-4-carbonyl]-cyclopropylméthyl-amino}-éthyl)-amide de l'acide 1-méthyl-1*H*-indazole-3-carboxylique ;
{2-[(2-Amino-5-*m*-tolyl-thiazole-4-carbonyl)-cyclopropylméthyl-amino]-éthyl}-amide de l'acide 1-méthyl-1*H*-indazole-3-carboxylique ;
(2-{[2-Amino-5-(3-fluoro-phényl)-thiazole-4-carbonyl]-cyclopropylméthyl-amino}-éthyl)-amide de l'acide 1-méthyl-1*H*-indazole-3-carboxylique ;
(2-{[2-Amino-5-(3-méthoxy-phényl)-thiazole-4-carbonyl]-cyclopropylméthyl-amino}-éthyl)-amide de l'acide 1-méthyl-1*H*-indazole-3-carboxylique ;
(2-{[2-Amino-5-(4-fluoro-phényl)-thiazole-4-carbonyl]-cyclopropylméthyl-amino}-éthyl)-amide de l'acide 1-méthyl-1*H*-indazole-3-carboxylique ;
(2-{[2-Amino-5-(3-chloro-phényl)-thiazole-4-carbonyl]-cyclopropylméthyl-amino}-éthyl)-amide de l'acide 1-méthyl-1*H*-indazole-3-carboxylique ;
{2-[(2-Amino-5-phényl-thiazole-4-carbonyl)-cyclopropylméthyl-amino]-éthyl}-amide de l'acide 1-méthyl-1*H*-indazole-3-carboxylique ;
(2-{[2-Bromo-5-(3-méthoxy-phényl)-thiazole-4-carbonyl]-cyclopropylméthyl-amino}-éthyl)-amide de l'acide 1-méthyl-1*H*-indazole-3-carboxylique ;
{2-[(2-Bromo-5-*p*-tolyl-thiazole-4-carbonyl)-cyclopropylméthyl-amino]-éthyl}-amide de l'acide 1-méthyl-1*H*-indazole-3-carboxylique ;
(2-{Cyclopropylméthyl-[5-(2-fluoro-phényl)-2-méthyl-thiazole-4-carbonyl]-amino}-éthyl)-amide de l'acide 1-méthyl-1*H*-indazole-3-carboxylique ;
(2-{Cyclopropylméthyl-[2-méthyl-5-(3-trifluorométhyl-phényl)-thiazole-4-carbonyl]-amino}-éthyl)-amide de l'acide 1-méthyl-1*H*-indazole-3-carboxylique ;
(2-{Cyclopropylméthyl-[5-(3-fluoro-phényl)-2-méthyl-thiazole-4-carbonyl]-amino}-éthyl)-amide de l'acide 1-méthyl-1*H*-indazole-3-carboxylique ;
(2-{Cyclopropylméthyl-[2-méthyl-5-(4-trifluorométhyl-phényl)-thiazole-4-carbonyl]-amino}-éthyl)-amide de l'acide 1-méthyl-1*H*-indazole-3-carboxylique ;
(2-{[5-(4-Chloro-phényl)-2-méthyl-thiazole-4-carbonyl]-cyclopropylméthyl-amino}-éthyl)-amide de l'acide 1-méthyl-1*H*-indazole-3-carboxylique ;
(2-{Cyclopropylméthyl-[5-(3-méthoxy-phényl)-2-méthyl-thiazole-4-carbonyl]-amino}-éthyl)-amide de l'acide 1-méthyl-1*H*-indazole-3-carboxylique ;
(2-{[5-(3-Cyano-phényl)-2-méthyl-thiazole-4-carbonyl]-cyclopropylméthyl-amino}-éthyl)-amide de l'acide 1-méthyl-1*H*-indazole-3-carboxylique ;
(2-{Cyclopropylméthyl-[5-(3,4-diméthyl-phényl)-2-méthyl-thiazole-4-carbonyl]-amino}-éthyl)-amide de l'acide 1-méthyl-1*H*-indazole-3-carboxylique ;
{2-[Cyclopropylméthyl-(2-méthyl-5-*p*-tolyl-thiazole-4-carbonyl)-amino]-éthyl}-amide de l'acide 1-méthyl-1*H*-indazole-3-carboxylique ;
{2-[Cyclopropylméthyl-(3'-méthyl-biphényl-2-carbonyl)-amino]-éthyl}-amide de l'acide 1-méthyl-1*H*-indazole-3-carboxylique ;
{2-[Cyclopropylméthyl-(4'-méthyl-biphényl-2-carbonyl)-amino]-éthyl}-amide de l'acide 1-méthyl-1*H*-indazole-3-carboxylique ;
{2-[Cyclopropylméthyl-(4'-fluoro-biphényl-2-carbonyl)-amino]-éthyl}-amide de l'acide 1-méthyl-1*H*-indazole-3-carboxylique ;
Cyclopropyl-{2-[(2,3-dihydro-benzo[1,4]dioxine-5-carbonyl)-amino]-éthyl}-amide de l'acide 2-cyclopropyl-5-phényl-thiazole-4-carboxylique ;
Cyclopropyl-{2-[(2,3-dihydro-benzo[1,4]dioxine-5-carbonyl)-amino]-éthyl}-amide de l'acide 2-cyclopropyl-5-*m*-tolyl-thiazole-4-carboxylique ;
Cyclopropyl-{2-[(2,3-dihydro-benzo[1,4]dioxine-5-carbonyl)-amino]-éthyl}-amide de l'acide 2-cyclopropyl-5-(3-méthoxy-phényl)-thiazole-4-carboxylique ;
Cyclopropyl-{2-[(2,3-dihydro-benzo[1,4]dioxine-5-carbonyl)-amino]-éthyl}-amide de l'acide 2-amino-5-*m*-tolyl-thiazole-4-carboxylique ;
Cyclopropyl-{2-[(2,3-dihydro-benzo[1,4]dioxine-5-carbonyl)-amino]-éthyl}-amide de l'acide 2-amino-5-(3-fluoro-phényl)-thiazole-4-carboxylique ;
Cyclopropyl-{2-[(2,3-dihydro-benzo[1,4]dioxine-5-carbonyl)-amino]-éthyl}-amide de l'acide 2-amino-5-(3-méthoxy-phényl)-thiazole-4-carboxylique ;
Cyclopropyl-{2-[(2,3-dihydro-benzo[1,4]dioxine-5-carbonyl)-amino]-éthyl}-amide de l'acide 2-amino-5-(4-fluoro-phényl)-thiazole-4-carboxylique ;
Cyclopropyl-{2-[(2,3-dihydro-benzo[1,4]dioxine-5-carbonyl)-amino]-éthyl}-amide de l'acide 2-amino-5-(3-chloro-phényl)-thiazole-4-carboxylique ;
Cyclopropyl-{2-[(2,3-dihydro-benzo[1,4]dioxine-5-carbonyl)-amino]-éthyl}-amide de l'acide 2-amino-5-phényl-thiazole-4-carboxylique ;
Cyclopropyl-{2-[(2,3-dihydro-benzo[1,4]dioxine-5-carbonyl)-amino]-éthyl}-amide de l'acide 2-bromo-5-(3-méthoxy-phényl)-thiazole-4-carboxylique ;
Cyclopropyl-{2-[(2,3-dihydro-benzo[1,4]dioxine-5-carbonyl)-amino]-éthyl}-amide de l'acide 2-bromo-5-*p*-tolyl-thiazole-4-carboxylique ;
Cyclopropyl-{2-[(2,3-dihydro-benzo[1,4]dioxine-5-carbonyl)-amino]-éthyl}-amide de l'acide 5-(2-fluoro-phényl)-2-méthyl-thiazole-4-carboxylique ;
Cyclopropyl-{2-[(2,3-dihydro-benzo[1,4]dioxine-5-carbonyl)-amino]-éthyl}-amide de l'acide 2-méthyl-5-(3-trifluorométhyl-phényl)-thiazole-4-carboxylique ;
Cyclopropyl-{2-[(2,3-dihydro-benzo[1,4]dioxine-5-carbonyl)-amino]-éthyl}-amide de l'acide 5-(3-fluoro-phényl)-2-méthyl-thiazole-4-carboxylique ;
Cyclopropyl-{2-[(2,3-dihydro-benzo[1,4]dioxine-5-carbonyl)-amino]-éthyl}-amide de l'acide 5-(4-chloro-phényl)-2-méthyl-thiazole-4-carboxylique ;
Cyclopropyl-{2-[(2,3-dihydro-benzo[1,4]dioxine-5-carbonyl)-amino]-éthyl}-amide de l'acide 5-(3-méthoxy-phényl)-2-méthyl-thiazole-4-carboxylique ;
Cyclopropyl-{2-[(2,3-dihydro-benzo[1,4]dioxine-5-carbonyl)-amino]-éthyl}-amide de l'acide 5-(3,4-diméthyl-phényl)-2-méthyl-thiazole-4-carboxylique ;
Cyclopropyl-{2-[(2,3-dihydro-benzo[1,4]dioxine-5-carbonyl)-amino]-éthyl}-amide de l'acide 2-méthyl-5-*p*-tolyl-thiazole-4-carboxylique ;
{2-[Cyclopropyl-(3'-méthyl-biphényl-2-carbonyl)-amino]-éthyl}-amide de l'acide 2,3-dihydro-benzo[1,4]dioxine-5-carboxylique ;
{2-[Cyclopropyl-(4'-méthyl-biphényl-2-carbonyl)-amino]-éthyl}-amide de l'acide 2,3-dihydro-benzo[1,4]dioxine-5-carboxylique ;
{2-[Cyclopropyl-(4'-fluoro-biphényl-2-carbonyl)-amino]-éthyl}-amide de l'acide 2,3-dihydro-benzo[1,4]dioxine-5-carboxylique ;
Cyclopropylméthyl-{2-[(2,3-dihydro-benzo[1,4]dioxine-5-carbonyl)-amino]-éthyl}-amide de l'acide 2-cyclopropyl-5-phényl-thiazole-4-carboxylique ;
Cyclopropylméthyl-{2-[(2,3-dihydro-benzo[1,4]dioxine-5-carbonyl)-amino]-éthyl}-amide de l'acide 2-cyclopropyl-5-*m*-tolyl-thiazole-4-carboxylique ;
Cyclopropylméthyl-{2-[(2,3-dihydro-benzo[1,4]dioxine-5-carbonyl)-amino]-éthyl}-amide de l'acide 2-cyclopropyl-5-(3-méthoxy-phényl)-thiazole-4-carboxylique ;
Cyclopropylméthyl-{2-[(2,3-dihydro-benzo[1,4]dioxine-5-carbonyl)-amino]-éthyl}-amide de l'acide 2-amino-5-*m*-tolyl-thiazole-4-carboxylique ;
Cyclopropylméthyl-{2-[(2,3-dihydro-benzo[1,4]dioxine-5-carbonyl)-amino]-éthyl}-amide de l'acide 2-amino-5-(3-fluoro-phényl)-thiazole-4-carboxylique ;
Cyclopropylméthyl-{2-[(2,3-dihydro-benzo[1,4]dioxine-5-carbonyl)-amino]-éthyl}-amide de l'acide 2-amino-5-(3-méthoxy-phényl)-thiazole-4-carboxylique ;
Cyclopropylméthyl-{2-[(2,3-dihydro-benzo[1,4]dioxine-5-carbonyl)-amino]-éthyl}-amide de l'acide 2-amino-5-phényl-thiazole-4-carboxylique ;
Cyclopropylméthyl-{2-[(2,3-dihydro-benzo[1,4]dioxine-5-carbonyl)-amino]-éthyl}-amide de l'acide 2-bromo-5-(3-méthoxy-phényl)-thiazole-4-carboxylique ;
Cyclopropylméthyl-{2-[(2,3-dihydro-benzo[1,4]dioxine-5-carbonyl)-amino]-éthyl}-amide de l'acide 2-bromo-5-*p*-tolyl-thiazole-4-carboxylique ;
Cyclopropylméthyl-{2-[(2,3-dihydro-benzo[1,4]dioxine-5-carbonyl)-amino]-éthyl}-amide de l'acide 5-(2-fluoro-phényl)-2-méthyl-thiazole-4-carboxylique ;
Cyclopropylméthyl-{2-[(2,3-dihydro-benzo[1,4]dioxine-5-carbonyl)-amino]-éthyl}-amide de l'acide 2-méthyl-5-(3-trifluorométhyl-phényl)-thiazole-4-carboxylique ;
Cyclopropylméthyl-{2-[(2,3-dihydro-benzo[1,4]dioxine-5-carbonyl)-amino]-éthyl}-amide de l'acide 5-(3-fluoro-phényl)-2-méthyl-thiazole-4-carboxylique ;
Cyclopropylméthyl-{2-[(2,3-dihydro-benzo[1,4]dioxine-5-carbonyl)-amino]-éthyl}-amide de l'acide 5-(3-méthoxy-phényl)-2-méthyl-thiazole-4-carboxylique ;
Cyclopropylméthyl-{2-[(2,3-dihydro-benzo[1,4]dioxine-5-carbonyl)-amino]-éthyl}- amide de l'acide 5-(3-cyano-phényl)-2-méthyl-thiazole-4-carboxylique ;
Cyclopropylméthyl-{2-[(2,3-dihydro-benzo[1,4]dioxine-5-carbonyl)-amino]-éthyl}- amide de l'acide 5-(3,4-diméthyl-phényl)-2-méthyl-thiazole-4-carboxylique ;
Cyclopropylméthyl-{2-[(2,3-dihydro-benzo[1,4]dioxine-5-carbonyl)-amino]-éthyl}- amide de l'acide 2-méthyl-5-*p*-tolyl-thiazole-4-carboxylique ;
{2-[Cyclopropylméthyl-(3'-méthyl-biphényl-2-carbonyl)-amino]-éthyl}-amide de l'acide 2,3-dihydro-benzo[1,4]dioxine-5-carboxylique ;
{2-[Cyclopropylméthyl-(4'-méthyl-biphényl-2-carbonyl)-amino]-éthyl}-amide de l'acide 2,3-dihydro-benzo[1,4]dioxine-5-carboxylique ;
{2-[Cyclopropylméthyl-(4'-fluoro-biphényl-2-carbonyl)-amino]-éthyl}-amide de l'acide 2,3-dihydro-benzo[1,4]dioxine-5-carboxylique ;
{2-[(Benzofuran-4-carbonyl)-amino]-éthyl}-cyclopropylméthyl-amide de l'acide 2-cyclopropyl-5-phényl-thiazole-4-carboxylique ;
{2-[(Benzofuran-4-carbonyl)-amino]-éthyl}-cyclopropylméthyl-amide de l'acide 2-cyclopropyl-5-*m*-tolyl-thiazole-4-carboxylique ;
{2-[(Benzofuran-4-carbonyl)-amino]-éthyl}-cyclopropylméthyl-amide de l'acide 2-cyclopropyl-5-(3-méthoxy-phényl)-thiazole-4-carboxylique ;
{2-[(Benzofuran-4-carbonyl)-amino]-éthyl}-cyclopropylméthyl-amide de l'acide 2-amino-5-*m*-tolyl-thiazole-4-carboxylique ;
{2-[(Benzofuran-4-carbonyl)-amino]-éthyl}-cyclopropylméthyl-amide de l'acide 2-amino-5-(3-fluoro-phényl)-thiazole-4-carboxylique ;
{2-[(Benzofuran-4-carbonyl)-amino]-éthyl}-cyclopropylméthyl-amide de l'acide 2-amino-5-(3-méthoxy-phényl)-thiazole-4-carboxylique ;
{2-[(Benzofuran-4-carbonyl)-amino]-éthyl}-cyclopropylméthyl-amide de l'acide 2-amino-5-(4-fluoro-phényl)-thiazole-4-carboxylique ;
{2-[(Benzofuran-4-carbonyl)-amino]-éthyl}-cyclopropylméthyl-amide de l'acide 2-amino-5-(3-chloro-phényl)-thiazole-4-carboxylique ;
{2-[(Benzofuran-4-carbonyl)-amino]-éthyl}-cyclopropylméthyl-amide de l'acide 2-amino-5-phényl-thiazole-4-carboxylique ;
{2-[(Benzofuran-4-carbonyl)-amino]-éthyl}-cyclopropylméthyl-amide de l'acide 2-bromo-5-(3-méthoxy-phényl)-thiazole-4-carboxylique ;
{2-[(Benzofuran-4-carbonyl)-amino]-éthyl}-cyclopropylméthyl-amide de l'acide 2-bromo-5-*p*-tolyl-thiazole-4-carboxylique ;
{2-[(Benzofuran-4-carbonyl)-amino]-éthyl}-cyclopropylméthyl-amide de l'acide 5-(2-fluoro-phényl)-2-méthyl-thiazole-4-carboxylique ;
{2-[(Benzofuran-4-carbonyl)-amino]-éthyl}-cyclopropylméthyl-amide de l'acide 2-méthyl-5-(3-trifluorométhyl-phényl)-thiazole-4-carboxylique ;
{2-[(Benzofuran-4-carbonyl)-amino]-éthyl}-cyclopropylméthyl-amide de l'acide 5-(3-fluoro-phényl)-2-méthyl-thiazole-4-carboxylique ;
{2-[(Benzofuran-4-carbonyl)-amino]-éthyl}-cyclopropylméthyl-amide de l'acide 2-méthyl-5-(4-trifluorométhyl-phényl)-thiazole-4-carboxylique ;
{2-[(Benzofuran-4-carbonyl)-amino]-éthyl}-cyclopropylméthyl-amide de l'acide 5-(4-chloro-phényl)-2-méthyl-thiazole-4-carboxylique ;
{2-[(Benzofuran-4-carbonyl)-amino]-éthyl}-cyclopropylméthyl-amide de l'acide 5-(3-méthoxy-phényl)-2-méthyl-thiazole-4-carboxylique ;
{2-[(Benzofuran-4-carbonyl)-amino]-éthyl}-cyclopropylméthyl-amide de l'acide 5-(3-cyano-phényl)-2-méthyl-thiazole-4-carboxylique ;
{2-[(Benzofuran-4-carbonyl)-amino]-éthyl}-cyclopropylméthyl-amide de l'acide 5-(3,4-diméthyl-phényl)-2-méthyl-thiazole-4-carboxylique;
{2-[(Benzofuran-4-carbonyl)-amino]-éthyl}-cyclopropylméthyl-amide de l'acide 2-méthyl-5-*p*-tolyl-thiazole-4-carboxylique ;
{2-[Cyclopropylméthyl-(3'-méthyl-biphényl-2-carbonyl)-amino]-éthyl}-amide de l'acide benzofuran-4-carboxylique ;
{2-[Cyclopropylméthyl-(4'-méthyl-biphényl-2-carbonyl)-amino]-éthyl}-amide de l'acide benzofuran-4-carboxylique ; et
{2-[Cyclopropylméthyl-(4'-fluoro-biphényl-2-carbonyl)-amino]-éthyl}-amide de l'acide benzofuran-4-carboxylique ;
ou sel de celui-ci.

9. Composition pharmaceutique contenant, comme principe actif, un composé de formule (I) selon l'une quelconque des revendications 1 à 8 ou un sel pharmaceutiquement acceptable de celui-ci et au moins un excipient inerte sur le plan thérapeutique.

10. Composé de l'une quelconque des revendications 1 à 8 ou sel pharmaceutiquement acceptable de celui-ci, en vue d'une utilisation comme médicament.

11. Composé selon l'une quelconque des revendications 1 à 8 ou sel pharmaceutiquement acceptable de celui-ci pour la prévention ou le traitement de maladies sélectionnées dans le groupe consistant en tous les types de troubles du sommeil, de syndromes liés à un stress, d'usage ou d'abus de substances psychoactives, de dysfonctionnements cognitifs dans la population saine et chez des patients atteints de maladies psychiatriques et neurologiques et de troubles du comportement alimentaire ou perturbations des apports de liquides.

12. Utilisation d'un composé selon l'une quelconque des revendications 1 à 8 ou d'un sel pharmaceutiquement acceptable de celui-ci dans la préparation d'un médicament pour la prévention ou le traitement de maladies sélectionnées dans le groupe consistant en tous les types de troubles du sommeil, de syndromes liés à un stress, d'usage ou d'abus de substances psychoactives, de dysfonctionnements cognitifs dans la population saine et chez des patients atteints de maladies psychiatriques et neurologiques et de troubles du comportement alimentaire ou perturbations des apports de liquides.
